Europäisches Patentamt

European Patent Office

Office européen des brevets

(19)

(11) Publication number: **0 015 755**
**B1**

(12) **EUROPEAN PATENT SPECIFICATION**

(45) Date of publication of patent specification: **08.08.84**

(21) Application number: **80300692.3**

(22) Date of filing: **06.03.80**

(51) Int. Cl.³: **C 07 G 7/00, C 12 P 21/00, G 01 N 33/54, A 61 K 49/00 //A61K39/00, C12N5/00**

(54) **Recognins, their chemoreciprocals, target attaching globulins and processes for producing these products; methods of detecting cancer tumors.**

(30) Priority: **09.03.79 US 19078**

(43) Date of publication of application:
**17.09.80 Bulletin 80/19**

(45) Publication of the grant of the patent:
**08.08.84 Bulletin 84/32**

(84) Designated Contracting States:
**AT BE CH DE FR GB IT LU NL SE**

(56) References cited:
**EP - A - 0 007 214**
**DE - A - 2 546 670**
**FR - A - 2 397 449**
**GB - A - 1 524 221**
**GB - A - 1 532 803**
**GB - A - 1 533 464**
**US - A - 4 160 019**

**CHEMICAL ABSTRACTS, vol. 91, no. 19,
November 5, 1979, page 477, abstract 155789s.
Columbus, Ohio, USA BOGOCH, S, et al.
"Production of two recognins related to
malignin: recogin M from mammary MCF-7
carcinoma cells and recognin L from lymphoma
P3G cells". & Neurochem. Res. 1979, 4(4), 465-
72**

(73) Proprietor: **Bogoch, Samuel, Dr.**
**46 East 91st Street**
**New York, New York 10028 (US)**

(72) Inventor: **Bogoch, Samuel, Dr.**
**46 East 91st Street**
**New York, New York 10028 (US)**

(74) Representative: **Laredo, Jack Joseph et al,
Elkington and Fife High Holborn House 52/54
High Holborn
London, WC1V 6SH (GB)**

Courier Press, Leamington Spa, England.

**0 015 755**

## Description

This invention is directed to a group of compounds, herein termed Recognins. Recognins are made by treating normal or tumour cells or artificial cancer cells, and separating the desired products. The Recognins may be used to prepare their Chemoreciprocals, i.e., by contacting with body fluids unsupported Recognins or the Recognins on a support. These Chemoreciprocals are useful for diagnostic and therapeutic purposes, i.e., for diagnosing and treating cancers. The Chemoreciprocals are substances which react with immunochemical-like specificity with a Recognin *in vivo* or *in vitro*, e.g., in a quantative precipitin test, in Ouchterlony double diffusion or in immunofluorescence.

Recognins and their Chemoreciprocals as novel groups of compounds were first referred to in my U.S. Application Serial No. 941,940, filed September 13, 1978; which in turn was a continuation of my U.S. Application Serial No. 852,200, filed November 17, 1977; which in turn was a continuation of my U.S. Application Serial No. 621,112, filed October 9, 1975, which in turn was a Continuation-in-Part of each of my U.S. Applications Serial Number 553,075, filed February 25, 1975; Serial Number 550,432, filed February 18th, 1975; Serial Number 450,404, filed March 12, 1974; and Serial Number 385,451 filed August 3, 1973; and also a Continuation-in-Part of my U.S. Application Serial No. 922,799, filed July 7, 1978. Reference should also be made to my U.K. Patents Nos. 1,524,221, and 1,532,803 and also to my U.K. Patent No. 1,533,464 (which claimed priority from the above-noted U.S. Applications Serial Nos. 550,432; 553,075; and 621,112). Furthermore, reference should be made to my copending European Application No. 79 301315.2 filed on July 6, 1979 (claiming priority from the above-noted U.S. Applications Serial Nos. 922,799 and 941,940).

One of the Recognins is Astrocytin. Astrocytin has been produced from brain tumour tissue, preferably brain glioma tumour tissue (see publications referred to just above). Protein fractions containing the Astrocytin precursor are first extracted from the tissue. A preferred method of accomplishing the extraction is to treat the tissue with a neutral buffer under conditions of homogenization or other techniques to disrupt the cells and tissues in order to solubilize protein fractions which contain the Astrocytin precursor.

At this point, the Astrocytin precursor is still bound to many large molecular weight substances including proteins, glycoproteins, lipoproteins, nucleic acids, nucleoproteins, and so on. The solubilized proteins are separated from the resulting tissue extract. The extract solution from the tissue is clarified to remove insoluble particles. The low molecular weight contaminants are removed from the resulting solution by a perevaporation concentration technique. The solution which is obtained is then treated to cleave Astrocytin precursor from other contaminants in order to obtain the protein fraction having a pK range within the range of from 1 to 4, inclusive. Thus, for example, the solution is placed on a chromatographic column and eluted with increasingly acidic solvents. All of the fractions which are eluted in the neutral or acid range down to pK 4 are discarded, and those fractions with the pK range 1—4 are collected. The eluate is then treated to obtain a product having a molecular weight of 8,000 (TLGC). TLGC=thin layer gel chromatograph=method for determining molecular weight unless otherwise specified. The treatment is accomplished, for example, by first filtering the material to remove low molecular weight substances, i.e., those of molecular weight below 1,000, and filtering again to remove those of molecular weight above 25,000. The fraction having a molecular weight between 1,000 and 25,000 is then further treated, i.e., by thin layer gel (TLG) chromatography, to yield Astrocytin.

This, Astrocytin may be produced by extracting brain glioma tumour tissue with a neutral buffer, by repeated homogenization and high speed centrifuging, separating from the resulting extract the fraction having a pK value within the range of from 1 to 4, separating from said fraction the substances having a high molecular weight, i.e., up to 230,000, and isolating therefrom the product Astrocytin having a molecular weight of 8,000.

The product Astrocytin prepared in accordance with this process is characterized by forming a single line precipitate with its specific antibody in quantitative precipitin tests and Ouchterlony gel diffusion tests, being soluble in water and aqueous solutions having an acid or neutral pH, and insoluble at an alkaline pH, having a spectrophotometric adsorption peak wave-length of 280 m$\mu$ and having a molecular weight of 8,000 (as determined by thin layer gel chromatography).

Astrocytin is also characterised as a polypeptide having a very high percentage of residues of glutamic acid and aspartic acid and a very high ratio of these acids to histidine. A further analysis of Astrocytin is provided below.

In a manner similar to that described above, another Recognin, called Malignin, has been produced from artificial cancer cells, i.e. cancer cells grown by *in vitro* fermentation. Malignin has a molecular weight of 10,000 and is also a polypeptide with similar but distinct aminoacid residue composition to Astrocytin, i.e., high ratios of glutamic acid and aspartic acid and high ratios of these acids to histidine. A further analysis of Malignin is provided below.

Thus, Malignin can be produced by extracting artificial brain glioma cancer cells grown in a fermentation culture with a neutral buffer by repeated homogenization and high speed centrifuging, separating from the resulting extract the fraction having a pK range of 1 to 4, separating from said

2

fraction the substances having a high molecular weight, i.e. up to 230,000, and isolating therefrom the product having a molecular weight of 10,000.

The amount of Malignin produced in artificial cell fermentation and the percentage of total protein produced in artificial cell fermentation which is Malignin can be increased by the growth of an artificial cancer cell culture in large size growth containers.

Malignin prepared in accordance with this process is characterized by forming a single line precipitate with its specific antibody in quantitative precipitin tests and Ouchterlony gel diffusion tests, being soluble in water and aqueous solutions having an acid or neutral pH, and insoluble at an alkaline pH, having a spectrophotometric adsorption peak wave-length of 280 m$\mu$ and having a molecular weight of 10,000.

In a manner similar to that described above, I have now produced another Recognin, called Recognin M, from artificial mammary cancer cells, as distinct from brain cells, i.e., mammary cancer cells grown by *in vitro* fermentation. Recognin M has a molecular weight of 8,000 and is a polypeptide with a similar but distinct aminoacid residue composition to Astrocytin and Malignin, i.e., high ratios of glutamic acid and aspartic acid and high ratios of these acids to histidine. The principal differences in composition between Malignin and Recognin M are that the latter has lower amounts of aspartic acid, glutamic acid, leucine and tryosine and larger amounts of proline, glycine and alanine. A further analysis of Recognin M is provided below.

Recognin M can be produced by extracting artificial mammary cancer cells grown in fermentation culture with a neutral buffer by repeated homogenization and high speed centrifuging, separating from the resulting extract the fraction having pK range of 1 to 4, separating from said fraction the substances having a high molecular weight, i.e., up to 230,000, and isolating therefrom the product having a molecular weight of 8,000.

Recognin M prepared in accordance with this process is characterized by forming a single line precipitate with its specific antibody in quantitative precipitin tests and Ouchterlony gel diffusion tests, being soluble in water and aqueous solutions having an acid or neutral pH, and insoluble at an alkaline pH, having a spectrophotometric adsorption peak wave-length of 280 m$\mu$ and having a molecular weight of 8,000.

In a manner similar to that described above, a yet further Recognin, called Recognin L, has been produced this time from artificial lymphoma cancer cells, i.e., lymphoma cancer cells grown by *in vitro* fermentation. Recognin L has a molecular weight of 8,000 and is a polypeptide with a similar but distinct aminoacid residue composition to Astrocytin, Malignin and Recognin M, i.e., high ratios of glutamic acid and aspartic acid and high ratios of these acids to histidine. As compared with Malignin, Recognin L has lower amounts of aspartic acid, glutamic acid, leucine and tryosine and also lower amounts of proline, glycine and alanine. A further analysis of Recognin L is provided below.

Thus, Recognin L can be produced by extracting artificial lymphoma cancer cells grown in fermentation culture with a neutral buffer by repeated homogenization and high speed centrifuging, separating from the resulting extract the fraction having a pK range of 1 to 4, separating from said fraction the substances having a high molecular weight, i.e., up to 230,000, and isolating therefrom the product having a molecular weight of 8,000.

Recognin L prepared in accordance with this process is characterised by forming a single line precipitate with its specific antibody in quantitative precipitin tests and Ouchterlony gel diffusion tests, being soluble in water and aqueous solutions having an acid or neutral pH, and insoluble at an alkaline pH, having a spectrophotometric adsorption peak wave length of 280 m$\mu$ and having a molecular weight of 8,000.

Recognins are further characterized by being capable of complexing with bromoacetylcellulose to form bromoacetylcellulose-Recognin and producing the specific Anti-Recognin antibodies upon injection into mammals, the Anti-Recognin attaching specifically to the Recognin precursor *in situ*. For example, one Anti-Recognin, Anti-Malignin is toxic to brain tumour cells *in vitro*.

The Recognins of which Astrocytin, Malignin, Recognin M and Recognin L have been identified, are useful as products which may be introduced into a biological system to reduce foreign reactions, such as by coating a material with a Recognin. A further example may be to introduce a Recognin into the biological system in order to produce its Chemoreciprocal in the system. The Recognins may also be used nutritionally to encourage the growth of a particular biological system of which they are a part. A further utility of Recognins is the production of so-called Target reagents (described in detail below), which comprise the complexes of the Recognins with carriers to facilitate their applicability in biological systems. Thus, for example, the complex conveys the physiochemical characteristics of the Recognin itself. The carrier should be selected from those which form a complex with the Recognin and which are substantially biologically inert.

Any substance known in the art which will form a stable complex with polypeptides or proteins may be useful for complexing with the Recognins. An example is a cellulose-based material, such as bromoacetylcellulose. In addition to being inert to the biological system, the carrier should be one which does not alter the specific physiochemical properties of the Recognin which are useful for the purposes set forth herein.

The complex of a Recognin and its carrier is useful for producing, separating and identifying its chemoreciprocal in any biological system with which it is brought into contact. The Recognin-carrier complex is also useful for stimulating the production of its chemoreciprocal precursor in any biological system into which it is introduced.

One class of Chemoreciprocals are the Anti-Recognins, of which Anti-Astrocytin, Anti-Malignin, Anti-Recognin M and Anti-Recognin L have been identified. These may be made by injecting the recognin into a biological system. An immunologically effective dose of Recognin is brought into contact with body tissues or fluids in a manner which induces an antibody response in accordance with techniques known in the art for producing antibodies. The Anti-Recognins may be used for the delivery of materials such as diagnostic, nutritional and therapeutic agents to specific cells or sites in a biological system; this comprises introducing said agent in complexed form with the Anti-Recognin into the biological system. The Anti-Recognins are also useful for diagnosing the presence of tumour cells in an histology section, by applying the Anti-Recognin conjugated with a labelling substance such as a dye or radioactive substance, to said section, whereby staining or radioactive labelling occurs only with the tumour cells. Yet another use for Anti-Recognins is to increase the yield of other useful Chemoreciprocal products (such as TAG products described below) from a mammal; this comprises injecting an immunologically effective dose of a Recognin into the mammal, or other biological system.

Prior to the present invention, glycoprotein complexes were prepared from brain tissue and caused to yield antibodies. Thus, separated materials known as 10B glycoproteins produced from Tay-Sachs's disease brain tissue, were injected into rabbits and antibodies produced. These Tay-Sachs antibodies were used in the immunofluorescent study of brain-containing tumours. However, only reactive, normal, non-tumour glia, not tumour glia, were stained by these antibodies.

In contrast, when Astrocytin was produced from tumour tissue, and antibodies to Astrocytin (Anti-Astrocytin) were produced and employed in immunofluorescent studies of the brain, only tumour glia, not normal (non-tumour) glia, were stained by Anti-Astrocytin. Thus, the tissue of origin and the nature of the antibodies, as well as the specific type of cells stained show that anti-Tay-Sachs antibodies and Anti-Astrocytin are clearly different products.

Another class of Chemoreciprocals is Target reagents complexed with their chemoreciprocals. For example, Astrocytin Target (the product of Astrocytin complexed with a carrier such as bromoacetyl-cellulose) may be brought into contact with Anti-Astrocytin. The type of complex which results may be coupled with, and used for, the delivery of diagnostic, nutritional and therapeutic agents to specific cells or sites in a biological system. The complexes may also be used for purification procedures. For example, Anti-Astrocytin may be made by the decomplexing of Bromoacetylcellulose-Astrocytin-Anti-Astrocytin by hydrolytic treatment with an acid or proteinase enzyme. Target reagents are also useful in increasing the amounts of TAG products (described below) in biological systems, e.g. by bringing an immunologically effective dose of a Target reagent into contact with a body tissue or fluid.

Additional Chemoreciprocals are Tag reagents (i.e., Target-Attaching Globulins). A TAG product may be produced by bringing a Target reagent into contact with a body fluid for varying periods of time to form a complex and cleaving the TAG product therefrom. Two useful embodiments are S-TAG and F-TAG.

A process for producing an S-TAG (Slow-Target-Attaching-Globulin) product comprises reacting a blood serum or other body fluid with a Target reagent (e.g., Bromoacetylcellulose-Malignin, Bromoacetylcellulose-Recognin M and Bromoacetylcellulose-Recognin L) for approximately two hours or more at a low temperature, e.g., at 4°C, and cleaving the desired S-TAG product from the resulting material, e.g., with dilute acid for approximately two hours at a temperature of 37°C. The S-TAG product prepared in accordance with this process is characterized by being soluble in aqueous buffered solutions, forming a single line precipitate with its corresponding Recognin in Ouchterlony gel diffusion tests, being non-dialysable in cellophane membranes, being retained by millipore filters which retain molecules of over 25,000 molecular weight, having molecular weights in different states of aggregation (as determined by thin layer gel chromatography) of approximately 50,000 and multiples thereof into the macroglobulin range, and having a spectrophotometric adsorption peak wave-length of 280 m$\mu$.

A process for producing F-TAG (Fast-Target-Attaching-Globulin) products comprises reacting a blood serum or other body fluid with a Target (e.g., Bromoacetylcellulose-Malignin, Bromoacetyl-cellulose-Recognin L or Bromoacetylcellulose-Recognin M) reagent for approximately 10 minutes at a low temperature, e.g. at 4°C, and cleaving the desired F-TAG product from the resulting material, e.g. with a dilute acid for approximately two hours at a temperature of 37°C. The F-TAG product prepared in accordance with this process is characterized by being soluble in aqueous buffered solutions, forming a single line precipitate with its corresponding Recognin in Ouchterlony gel diffusion tests, being non-dialysable in cellophane membranes, being retained by millipole filters which retain molecules of over 25,000 molecular weight, having molecular weights in different state of aggregation (as determined by thin layer gel chromatography) of approximately 50,000 and multiples thereof into the macroglobulin range, and having a spectrophotometric absorption peak wave-length of 280 m$\mu$.

TAG products are useful for detecting cancer tumours in living mammals by determining the concentrations of S-TAG and F-TAG, respectively, produced by a known volume of the mammal's blood serum or other body fluid, and correlating this concentration with amounts determined to be indicative of cancer. TAG products are also useful for diagnosing the presence of tumour cells in an histology section; this comprises applying a TAG product conjugated with a labelling substance such as a dye or radioactive substance to said section, whereby staining or radioactive labelling occurs only with the tumour cells. TAG products have additionally been found to be cytotoxic to tumour cells. TAG products are also useful for directing the delivery of diagnostic, nutritional and therapeutic agents to specific cells or sites by introducing said agents in complexed form with the TAG products.

Normal cell division in plants or animals is restricted or inhibited when the cells come to occupy fully a particular space. The mechanisms (a) by which normal cells "recognize" that they have filled the space available to them, and (b) by which the operation of this recognition mechanism in turn inhibits cell division have both been unknown. A group of compounds have now been produced whose precursors are increased in concentration when normal recognition and learning occur, and which relate to recognition and learning in particles and cells, and to the connection of cells to each other. These compounds are termed RECOGNINS. Attempts to produce these compounds from ordinary cancer cells have shown that they are absent as such, and that changes in their molecular structure have occurred at the same time as the cancer cells have lost their ability (a) to recognize that they have filled their normal volume, and/or (b) to stop dividing when they have filled their normal volume.

RECOGNINS are compounds which have physiochemical characteristics which mimic those configurations characteristic of cancer cells in terms of their failure to recognize and to stop cell division. The use of RECOGNINS goes beyond insight into the cancer mechanism, for immediate products and methods are thereby provided which are useful in the diagnosis and treatment of cancer, and for its prevention.

Methods by which artificially cultured cells can be used to produce Recognins have also been devised. One advantage of the methods disclosed herein is that Recognins and the products derived from them can be manufactured efficiently in virtually limitless quantities. The Recognins and products derived from them are applicable to biological systems in which it is desired to influence both growth and metabolism. Thus, by the manufacture of the particular compound or compounds of appropriate cell type in artificial culture, and the further manufacture of products derived from these substances, specific influence may be brought to bear on tissues, cells, cell organelles, sub-organelle molecules or molecular aggregates in various living system. In particular, specific nutritional influences at critical times in development, specific diagnostic, preventative and treatment methods, and in the construction of an artificial bioelectrical system (as in tissue or organ transplants) can all be exerted for the first time. Specifically, these artificial bio-electrical systems can be made to bear the characteristics of the specific RECOGNIN, in particular, RECOGNIN L or RECOGNIN M, or their CHEMORECIPROCALS of the normal tissue or component which they will neighbour and thus be "recognized" as "foreign", thereby avoiding the usual reactions to alien substances, including rejection.

Another aspect of this invention is the production of a valuable specific antibody-like product, namely, Anti-Recognin L or Anti-Recognin M, relative to a specific lymph or mammary product, namely, Recognin L or Recognin M, respectively, permitting the use of this antibody-like product specifically to complex with, and, as a specific delivery vehicle to, specific points in the nervous systems of various species.

Still another aspect of this invention is the production from biological fluids of the new products, TARGET-ATTACHING GLOBULINS (TAG) derived from Recognin L and Recognin M, respectively. These TAG products are so named because they are produced by two reactions. The first is the reaction of biological fluids with a synthetic complex containing physiochemical configurations which mimic those of RECOGNIN L and RECOGNIN M, respectively, and called TARGET. The second is the cleavage of the specific TAG from the complex, and by the measure of the TAG so produced, obtaining a quantitative indication from the biological fluids of living organisms as to whether there is present a tumour in that organism; hence, a diagnostic test for tumours. Because the ANTI-RECOGNINS L and M and the latter TAG products are physiochemically complementary to Recognins L and M, respectively, they are termed CHEMORECIPROCALS of Recognins L and M, respectively.

I have further discovered that two quantitatively and qualitatively distinct TAG products can be produced from the latter Recognins, depending upon the time permitted for the reaction of serum with the specific TARGET reagent used, and depending upon the time permitted for the cleavage of the product which has been complexed.

After examining the amounts of these products which could be produced from a number of different individuals with various lymph and mammary tumours and other medical disorders, as well as in those with no apparent disease process, it became apparent that the amounts of those two new products which could be produced in a given individual were indicative of whether that individual had a tumour, and hence, a novel serum diagnostic test for tumours has been devised.

The utility of these new products, in addition to their use in the diagnosis from serum and other biological fluids of the presence of the latter tumours, is illustrated by the demonstration that the TAG and Anti-RECOGNIN Compounds derived from Recognins L and M attach to glyial tumour cells preferentially in histological section of the tumour and surrounding tissue removed upon surgery of the tumour. This preferential labelling by the TAG products and Anti-RECOGNINS of tumour cells is demonstrated through standard immunofluorescent techniques. Thus, a new method is also available

5

# O O15 755

for determining through histological examination with a new degree of certainty whether tumour cells have penetrated to the very edges of the tissue removed indicating the likelihood that the tumour still remains in the organ, or that tumour cells are absent from the periphery of the tissue removed, indicating the possibility that all of the tumour has been removed from the brain or other organ. In addition, the TAGs and Anti-RECOGNINS produced as described have been found to be cytotoxic in relation to glioma tumour cells grown in tissue culture *in vitro.* This high affinity for tumour cells in another medium, here grown in tissue culture, is further evidence of the specific-coupling potential of the new TAG products, and explains the adoption of the name TARGET-ATTACHING-GLOBULINS (TAG) as do the properties of the TAG product in relation to the synthetic product TARGET, and to tumour cells in histological section. Further, the cytotoxicity of the TAGs and Anti-RECOGNINS in relation to tumour cells provides an additional new diagnostic test for the serum of patients who are suspected of suffering from a tumour. Thus, for example, the serum or other body fluid of these patients is reacted with TARGET to produce TAG, and the product TAG is tested in tissue culture growths of tumour cells for cytotoxicity. Both the concentration of TAG and the degree of cytotoxicity manifested by the TAG which can be produced from a given individual's serum may be not only diagnostic but also of value in tracing the course of the disorder pre-operatively and post-operatively in a given patient. The coupling of radioactive and dye tracers to TAG produces new TAG products which are useful *in vivo* in the diagnosis of tumours and in their exact localization. Thus, e.g., the injection of suitably labelled TAG either intra-arterially or intravenously, for example, permits the demonstration by radioactive means, or by visualization of the coupled dye, of the presence of a tumour, for it is only to the tumour cells that the TAG specifically attaches. Further, this method permits the precise visualization of the location of the tumour. This can be seen to be an improvement of the *in vivo* diagnostic method using Anti-ASTROCYTIN produced in rabbit blood to label a brain tumour, because the use of TAG produced from human serum avoids the possibility of foreign protein reactions. Since the TAGs and Anti-RECOGNINS have a chemical specificity which permits preferential attachment to the Recognin precursor containing tumour cells both *in vitro* and *in vivo*, these products may also be used therapeutically, as well as diagnostically, when coupled, e.g., with radioactive, proton capture agents, or other toxic physical or chemical agents, so that these toxic substances may be localized preferentially through these compounds' specificity of attachment in the tumour cells as compared with their neighbouring normal cells. This selectivity is universally recognized as the crucial, or at least one crucial factor, for achieving effective chemical or physical therapy of tumours. Thus, the TAG products demonstrate efficacy in attaching preferentially to tumour cells, and should have promise as novel therapeutic products for these reasons.

In the serum of patients with malignant tumours, as will be seen in the examples below, one type of TAG, SLOW-TAG (S-TAG) as distinguished from FAST-TAG (F-TAG), can be produced in relatively greater amounts from a given volume of serum than in patients without such tumours. This suggests that either one of a TAG's naturally occurring precursors (P-TAG) is increased in concentration, or that other factors exist which favour the relative *in vitro production* of S-TAG over F-TAG.

The possible relationship of the function of the actual synthetic products TARGET and TAG to their Recognin precursors, and in turn of the functions of postulated but not demonstrated cell "antigens" and circulating "antibodies" which may exist *in vivo*, has yet to be elucidated. Thus, for example, in antibody-like fashion, F-TAG and S-TAG produce single discrete lines of reaction with Recognins L and M in Ouchterlony gel diffusion, and the injection of TARGET products into rabbits induces an increase in the yield of TAG products from rabbit serum after reaction with the TARGET products. The finding that there may be a normal level of a precursor resembling antibody to a cell antigen which is hidden in the non-dividing cell raises a question as to the possible function of the pair. It is here proposed that TAG *precursor* (P-TAG) and TARGET-like substances exist *in vivo* and function in the control of cell proliferation and cell death. Thus, for example, the exposure of a cell constituent which normally is not directly exposed to serum proteins may occur during cell division. The exposure of this cell constituent could result in that constituent becoming converted to a TARGET-like substance to which the attachment of a P-TAG-like molecule from serum may then occur, which would stimulate cell division or inhibit it. Alternatively, a non-dividing cell which is injured or malfunctioning may expose a TARGET-like substance to which the attachment of P-TAG-like molecules may be reparative. However, under certain cell conditions, the attachment of P-TAG-like molecules may induce the destruction of the cell (e.g., ANTI-GLIOMA-TAG synthetically produced as here described is markedly cytotoxic to glioma tumour cells growing in tissue culture). This could thus represent a mirror of a normal mechanism for the control of cell division, and for either the repair or the removal of individual cells in the body throughout the life of the organism. If the exposure of cell constituents is abnormally increased so that abnormally large amounts of cell TARGET-like substances are formed, as may occur in rapidly dividing cancer cells such as in grain gliomas, an increase in the concentration of one type of serum P-TAG relative to another may be induced.

Whatever the actual function of the precursors, the increase in the relative amount of predominately one type of TAG, SLOW-TAG (S-TAG), which can be produced *in vitro* by the method here described from the serum of patients with malignant tumours, is the basis of the serum diagnostic test described in the examples which follows.

6

The invention is illustrated by the following examples. For the sake of completeness the Recognins previously discovered, namely, Astrocytin and Malignin and products derived therefrom are described and illustrated at the same time as the novel Recognins L and M of the present invention and the products derived therefrom. As regards the latter, the products obtained from Recognins L and M are derived in precisely similar manner. Examples 5A and 5B refer specifically to Recognins L and M of the present invention but Example 5B contains also a full discussion of the class of Recognins which also comprises the Astrocytin and Malignin already referred to.

As background and by way of comparison, Examples 1 and 2 refer to Astrocytin precursor and Astrocytin, respectively, Example 2A refers to a fourth member of the class of Recognins, namely, so-called "Reeler" Recognin, Examples 3 to 5 refer to Malignin precursor and Malignin, respectively, and Example 5 refers to improved production of Malignin. Example 6 relates to Target reagents obtained from Astrocytin and Malignin in addition to Recognins L and M. Example 7 refers to the antisera of Astrocytin, Malignin and Target reagents generally, Example 8 to detection by TAG products, Example 9 tumour diagnosis by immunofluorescence.

Example 10 to the cytotoxicity demonstrated by two specific Recognins, i.e., Anti-Astrocytin and Anti-Malignin and by S-TAG, Example 11 to the hydrolytic cleavage of Recognins, Example 12 to the production of artificial tissue or organ with Recognins, Example 13 to a serum diagnostic test illustrated by Malignin; Example 14 to the detection of non-brain malignant cells using a fluorescent signal from a TAG product and Example 15 to the detection of cancer cells with a radioisotope signal from a TAG product.

Example 1
Production of crude ASTROCYTIN-precursor-containing fraction

Human brain glioma tumour tissue, removed at surgery, is detected so as to be as free as possible of surface blood vessels and normal brain tissue. For a typical amount of dissected tumour tissue of 11 grams, the tissue is weighed into six 1.5 g and two 1.0 g aliquots. Each aliquot is then treated as follows.

Each aliquot is homogenized in neutral buffer solution by sonification or other mechanical means. For example, each aliquot is homogenized in 100 cc per g of tissue of 0.005 M phosphate buffer solution, pH 7, in a Waring blender. Homogenization should be done in the cold to prevent denaturing of proteins. For example, the blender should be precooled in a cold room at 0—5°C and operated for only three minutes.

The homogenate is then centrifuged for clarification, for example, at 80,000 times gravity for 30 minutes in a refrigerated ultracentrifuge. The soluble supernatant material is decanted and kept in the cold. The insoluble residue is rehomogenized with a further 100 cc of neutral buffer and centrifuged as before, and the second soluble extract is combined with the first. The best yields are obtained when this procedure of homogenization and centrifuging is repeated until less than 50 micrograms of protein per ml of solution are obtained in the supernatant. With most tissue, this is accomplished by the fifth extraction.

The solutions thus obtained are combined and concentrated by pre-evaporation with subsequent dialysis, as by dialysis against 0.006 M phosphate buffer in the cold, to produce a volume of 15 ml. The volume of this solution is noted, an aliquot is taken for total protein analysis, and the remainder is fractionated to obtain the protein fraction having a pK range between 1 and 4. The preferred method of fractionation is chromatography as follows.

The solution is fractionated in the cold room (4°C) on a DEAE cellulose (Cellex-D) (trade mark) column 2.5×11.0 cm, which has been equilibrated with 0.005 M sodium phosphate buffer. Stepwise eluting solvent changes are made with the following solvents (solutions): Solution (1): 4.04 g $NaH_2PO_4$ and 6.50 g $Na_2HPO_4$ are dissolved in 15 litres of distilled $H_2O$ (0.005 molar, pH 7); Solution (2): 8.57 g $NaH_2PO_4$ are dissolved in 2480 ml of distilled $H_2O$; Solution (3): 17.1 g of $NaH_2PO_4$ are dissolved in 2480 ml of distilled $H_2O$ (0.05 molar, pH 4.7); Solution (4): 59.65 g of $NaH_2PO_4$ are dissolved in 2470 ml distilled $H_2O$ (0.175 molar); Solution (5): 101.6 g of $NaH_2PO_4$ are dissolved in 2455 ml distilled $H_2O$ (0.3 molar, pH 4.3); Solution (6): 340.1 g of $NaH_2PO_4$ are dissolved in 2465 ml of distilled $H_2O$ (1.0 molar, pH 4.1); and Solution (7): 283.64 g of 80% phosphoric acid ($H_3PO_4$) are made up in 2460 ml of distilled $H_2O$ (1.0 molar, pH 1.0).

Add nervous tissue extract, 6 to 10 ml volume. Let it pass into the column. Then, overlay with Solution (1) and attach a reservoir of 300 ml of Solution (1) to drip by gravity onto the column. Three ml aliquots of effluent are collected by means of an automatic fraction collector. The subsequent eluting solutions are exchanged stepwise at the following elution tube numbers: Solution (2): at tube 88, bring solution on column to top of resin, then overlay and attach reservoir of 50 ml of Solution (2); Solution (3); at tube 98, bring solution on column to top of resin, then overlay and attach reservoir of 75 ml of Solution (3); Solution (4): at tube 114, bring solution on column to top of resin, then overlay and attach reservoir of 150 ml of Solution (4); Solution (5): at tube 155, bring solution on column to top of resin, then overlay and attach reservoir of 125 ml of solution (5); Solution (6): at tube 187, bring solution on column to top of resin, then overlay and attach reservoir of 175 ml of Solution (7); continue eluting until, at tube 260, elution is complete. Use freshly prepared resin for every new volume of tissue extract. Each effluent tube is quantitatively analysed for protein. The elutes in tube numbers 212 to 230 are combined, and contain the crude products from which ASTROCYTIN will be produced.

While data have previously been published on this crude material, called fraction 10B [Protein Metabolism of the Nervous System, pp 555—69 (Plenum Press, 1970); Journal of Neurosurgery, Vol. 33, pp. 281—286 (September, 1970)], the cleavage from fraction 10B of the specific product herein called ASTROCYTIN has now been accomplished (see also my earlier work referred to above, e.g., U.K. Patents Nos. 1,524,221, 1,532,803 and 1,533,464). Crude fraction 10B can be prepared as a product in amounts between 0.1 and 10 mg per gm of original fresh nervous system tissue from which it was obtained. In addition to an ASTROCYTIN-precursor it contains varying amounts of covalently bound carbohydrate residues including a number of hexoses, namely, glucose, galactose and mannose; hexosamines, including glucosamine, glactosamine and mannosamine; and occasionally other sugars, such as fructose, ribose and perhaps rhamnose. It also contains large molecular weight protein products and several lipids and nucleic acids.

Example 2

Production of purified ASTROCYTIN from crude ASTROCYTIN-precursor-containing fraction

The ASTROCYTIN-Precursor-Containing Fraction is further isolated from contaminants. In the preferred embodiments, the material from Example 1 is chromatographed on Sephadex G-50 (trade mark) resin with a typical column of 40 cm length, 2.5 cm diameter, and 196 ml volume. The pressure used is 0.04 bar (40 mm Hg), the flow rate is 35 ml per hour, and the buffer is 0.05 molar phosphate buffer solution, pH 7.2. The first (flow-through) peak contains ASTROCYTIN-Precursor together with impurities, whereas subsequent peaks contain only impurities.

In the preferred embodiment, the products in the above first flow-through peak are concentrated on Sephadex G-15 (trade mark), and then passed onto a column of Cellex-D (trade mark), with the same solutions (1) to (7) as in Example 1 and the same elution steps as are performed in Example 1. The product ASTROCYTIN is presented as a sharp peak in the same tubes (numbers 212—230 as before, thus maintaining its behaviour on Cellex-D (trade mark) chromatography without the presence of a large number of contaminants.

Low molecular weight contaminants may then be removed by techniques known to the art, such as millipore disc filtration. In the preferred method, the product ASTROCYTIN is freed of salt and other small molecular weight contaminants by filtration through Millipore Pellicon Disc No. 1000, 13 mm, which retains substances of molecular weights greater than 1000 and allows those of molecular weight below 1000 to pass through. The product ASTROCYTIN remains on the Pellicon Disc, and is recovered therefrom by washing with Solution (1) of Example 1.

ASTROCYTIN is then obtained by isolating the compound having a molecular weight of 8000 from the above solution. A preferred method uses thin layer gel (TLG) chromatography as follows:

The apparatus used is of the type commerically available from Boehringer Mannheim GmbH; Pharmacia Fine Chemicals and CAMAG (Switzerland). The resin, 2.5 g of Sephadex G-200 (trade mark) superfine, is prepared in 85 ml of 0.5 M NaCl in 0.02 M $Na_2HPO_4KH_2PO_4$ Phosphate Buffer, pH 6.8 (6.6—7.0); allow to swell for two or three days at room temperature with occasional gentle mixing (magnetic and other stirrers should not be used). The swollen gel is stabilized for three weeks at refrigerator temperature; However, bacterial and fungal growth may interfere with the swollen gel so that if the gel is to be kept for longer periods of time, a small amount of a bacteriostatic agent should be added (e.g., sodium azide: 0.02%). 2.5 g of dry gel are used to make two 20×20 cm glass plates, 0.5 mm thick. The plates are allowed to dry at room temperature for 10 minutes and transferred to a moist chamber where they can be stored for two weeks, or they are used immediately after appropriate pre-equilibration (usually, during the night, for a minimum of 12 hours). The main function of equilibration is to normalize the ratio between the stationary and mobile phase volumes. With the pre-equilibrated plates in an horizontal position, the substances to be determined are applied with micropipettes as spots or as a streak at the start line. 10 ml to 20 ml of 0.2—2% protein solution are placed on the edge of a microscopic cover slide (18 mm by 18 mm) and are held against the gel surface. In a few seconds, the solution will soak into the gel. All samples are first prepared on the cover slides and then quickly applied. If not enough material is used, it is difficult to locate individual spots after separation. If too much material is applied, no defined separation occurs. The samples are diluted with buffer for easier handling and the separation of samples is carried out in a decending technique with the plate at an angle of 22°. A flow rate of 1—2 cm/hour is most suitable. Marker substances (such as cytochrome C, haemoglobin, myoglobin or Bromophenol Blue labelled albumin) are applied at different positions across the plate and also to serve as reference proteins for the calculation of the relative distance (mobility) of unknowns. After the appliation of the samples, the plates are replaced in the apparatus and the paper wick is pushed slightly downwards to ensure good contact with the gel layer. The paper wick must not drip. Excess moisture is wiped off. The liquid solvent in the reservoir is kept constant at 1 cm from the upper end of the vessel. The runs are usually completed in from 4 to 7 hours depending on the progress of the separation. With coloured substances, separation follows directly. The separated spots of protein are easily made visible by transferring them to a paper sheet replica of TLG plate after the chromatographic separation has been completed, and by staining them on the prewashed

methanol+$H_2O$+acetic acid—90:5:5, for 48 hours. The paper sheet is 3 mm filter paper. A sheet of paper 20×18 cm is placed over the gel layer and pressed (rolled) just enough to ensure contact with the gel. Care is taken not to trap air under the paper (replica) and not to disturb the gel layer. The liquid phase is soaked off from the gel layer by the paper and removed after one minute, immediately dried in an oven at 60° temperature for 15 minutes, and stained in the normal way with any of the routine staining procedures. Staining is performed by spraying the replica-paper with 0.03% diazotized sulphanilic acid in 10% sodium carbonate (Pauley's Reagent). Staining can also be accomplished with a saturated solution of Amido Black in methanol-acetic acid (90:10 v/v is used); the staining time is 5—10 minutes; for destaining, rinse with two volumes of the 90:10 methanol and acetic acid solution mixed with one volume of $H_2O$. It is difficult to obtain low background staining without very extensive washing. The plates themselves may also be dried at 60°C (in an oven with air circulation) but only if the ASTROCYTIN is to be stained. For isolation purposes, the plate should only be air dried at room temperature. Over-heating can lead to cracking, but this can usually be avoided by the use of a temperature of 50°C—60°C which dries a Sephadex G-200 (trade mark) plate in 15—30 minutes. The dry plates are allowed to swell for 10 minutes in a mixture of methanol+$H_2O$+acetic acid (75:20:5), stained in a saturated Amido Black in the same solvent system for five hours, and subsequently washed by bathing for two hours in the same solvent before they are dried. For molecular weight determinations, the distance from the starting line to the middle of each zone is measured with an accuracy of 0.05 mm either directly on the print (replica) or on the densitogram. The result is expressed by the $R_m$ value defined as the ratio of the migration distance of the tested protein ($d_p$) to that of cytochrome C or myoglobin ($d_m$) which is used as the reference protein.

The formula:

$$(-R_m = \frac{d_p}{d_m})$$

relates to the migration distance of the tested substance relative to standard.

A straight calibration line is obtained by plotting the logarithm of the molecular weight of the standards used against the $R_m$ value. From this line, the molecular weight of the unknown protein can be obtained; for the most precise results, mix equal parts of the protein sample solution with the standard, in this case, Cytochrome C, before applying to the plate. By the above TLG procedure, the product ASTROCYTIN is observed as a discrete spot at a distance of approximately 0.83±0.02, with reference to the standard Cytochrome C, yielding an approximate molecular weight of 8000 for ASTROCYTIN. Several discrete products are separated by this procedure from ASTROCYTIN on the basis of slight differences in molecular weight. Thus, three products carried as contamination to this point with molecular weights of approximately 64,000, 148,000 and 230,000 respectively, and one occasionally of molecular weight 32,000, have been detected and removed by the TLG methods described above. The product ASTROCYTIN is aspirated with the gel in which it is contained, in dry form, dissolved in Solution (1) and freed of resin, e.g. by centrifuging.

The product ASTROCYTIN which has been produced at this stage is soluble in distilled water, soluble at neutral and acid pH values, insolube at an alkaline pH and has a spectrophotometric absorption peak wave-length of 280 m$\mu$. It is a polypeptide with a molecular weight, as stated above, of approximately 8000. Its covalently linked aminoacids are shown by hydrolysis with 6N HCl, followed by quantitative automatic determination, to have the following average composition of aminoacids:

# 0 015 755

|  | Approximate number of residues |
|---|---|
| Aspartic acid | 9 |
| Threonine | 5 |
| Serine | 6 |
| Glutamic acid | 13 |
| Proline | 4 |
| Glycine | 6 |
| Alanine | 9 |
| Valine | 4 |
| 1/2 Cysteine | 2 |
| Methionine | 1 |
| Isoleucine | 2 |
| Leucine | 8 |
| Tyrosine | 2 |
| Phenylalanine | 3 |
| Lysine | .8 |
| Histidine | .2 |
| Arginine | 4 |
| Approximate total | 88 |

Cysteic acid, hydroxyproline, norleucine, ammonia, isodesmosine, desmosine, hydroxyysine, lysinonorleucine and gamma-aminobutyric acid are all absent in detectable amounts, but a trace of glucosamine may be present.

From 11 grams of the starting brain tumour tissue of Example 1, approximately 3 mg of purified ASTROCYTIN are produced by the above methods.

Example 2A
Production of "Reeler" recognin

Reeler disease is a genetic disorder in which animals fail to achieve stable coordinated motor activity, producing a reeling state, due to the failure of certain nerve cells to migrate to a particular place in the brain (the cerebellum) at a particular developmental time. Particular glial cells have been shown, in electron microscopic studies, to provide the vertical pole-like axes along which the nerve cells migrate to their new positions in the normal state. The failure in Reeler disease of nerve cells to climb these glial fibers is thought to be due to some disturbance in the nerve cells of the glia, or both.

Following the methods of Examples 1 and 2, respectively, Recognin from mouse reeler brain was produced and compared with Recognin produced from normal mouse brain. The molecular weight of the Recognin produced from all areas of normal mouse brain was 8,000. The molecular weight of "Reeler" Recognin was 3,600 to 5,000. "Reeler" Recognin is abnormal, as shown by its much smaller molecular weight. Furthermore, the amount of Recognin which can be produced from the cerebellum of reeler mouse brain is much reduced when compared with the normal. This is shown in Table I, as follows:

TABLE I
Recognin concentration mouse brain mg/g

|  | Normal | Reeler |
|---|---|---|
| Cerebellum (16-day old mouse) | 2.50 | 0.71 |
| Cortex (16-day old mouse) | 0.60 | 0.96 |
| Cortex (4-day old mouse) | 0.29 | 0.53 |
| Brainstem (16-day old mouse) | 0.90 | 1.64 |
| Whole brain (17-day old mouse) | 1.27 | 1.53 |
| Whole brain (1-day old mouse) | 5.00 | 5.86 |

Table I shows that, while there is a marked decrease in the concentration of Recognin in the cerebellum of Reeler mouse, there are same or greater amounts of Recognins in other areas of the brain. The Recognin may not move from the other areas of the brain to the cerebellum, or the slight increase in other areas of the Reeler brain may reflect some compensatory action.

This pathological Recognin in Reeler brain is correlated with the inability for migrating brain cells to make the contacts they must in order to achieve proper placement, confirming the role of RECOGNINS in recognition and learning in cells.

By analogy, the ANTI-RECOGNINS to Recognin produced from mouse reeler brain may be produced and used in mice in a manner similar to the uses of ANTI-ASTROCYTIN and ANTI-MALIGNIN, as described herein.

10

Example 3
Production of MALIGNIN-precursor in artificial cancer cell culture fermentations.

Generally, a sterile technique is scrupulously maintained.

All solutions (e.g. Hank's Bala ced Salt (BSS), F-10 Nutrient medium, foetal calf serum and try-sin solution) are incubated at 35°C in a water bath for approximately 20 minutes or more before use.

Cells are removed from glioma tumour tissue and grown *in vitro* for many generations using a suitable medium, such as described below. Pre-rinse beakers are used with a sterilizing solution, for example 12-proponal plus amphyl or creolin solution.

In the preferred embodiment, the artificial cancer cells (i.e., cells grown *in vitro* for many generations) are grown in 250 ml flasks. The liquid medium in which the cells are growing is discharged into the pre-rinsed beakers. The cells are then washed gently with 5—10 ml of Hank's BSS for 30 seconds, agitation being avoided. All walls and surfaces are washed. The solution is clarified of cells by centrifuging in the cold for 10 to 20 minutes at 3,000 rpm. The medium is poured into a beaker as above. A small amount of buffered proteinase enzyme solution is added and the medium is rinsed quickly to avoid digestion of the cells. In the preferred method, 1—2 ml of trypsin solution (EDTA) are added and rinsing is for only 10 seconds. The trypsin solution is poured off.

A similar volume of fresh trypsin solution is added and the medium is incubated until the cells are seen through microscopic observation to be separated from the walls of the chamber. This usually requires 5—10 minutes. A suitable growth medium, such as 50 ml of a solution of a 7—10 percent solution of foetal calf serum in 100 ml of F-10 Nutrient medium is added.

Twenty-five ml of the fresh medium with cells are transferred to a new growth chamber for propagation. Both chambers are placed in an incubator at 35°C for approximately seven days. By the procedure of this Example to this point, an artificial cancer cell culture is divided into two fresh cultures approximately every seven days. This entire procedure may be repeated as often as desired, at approximately seven-day intervals, for each growth chamber. Thus, the number of cells growing *in vitro* may be doubled approximately every seven days.

The cells may be extracted for the production of MALIGNIN after approximately seven days' growth. For example, cells growing in each 250 ml growth chamber, as described above, may be recovered as follows.

The medium is transferred to a centrifuge tube and centrifuged at 3,000 rpm in the cold for 10 minutes. The medium is discarded. The cells remaining in the growth chamber are scraped from the chamber walls and washed into the centrifuge tubes with a neutral buffer solution. The cells are washed twice with neutral buffer solution and centrifuged again at 3,000 rpm in the cold, and the medium is discarded. The washed cells are suspended in 10 ml of neutral phosphate buffer until ready for extraction of crude MALIGNIN-Precursor-Containing fraction.

Example 4
Production of crude MALIGNIN-precursor-containing fraction

Washed cells suspended in neutral buffer from Example 3 are mechanically disrupted under conditions which avoid the denaturing of most proteins. In the preferred method, the washed cells are treated in the cold with a sonifier for 20 seconds.

After sonification, the cell residues are centrifuged at 30,000 rpm for 30 minutes and the supernatant material is decanted off. Ten ml aliquots of buffer solution are used to wash remaining cell residues. These are sonified and centrifuged as above and the supernatants are combined. The process is then repeated once more.

The combined supernatant is pre-evaporated to reduce the approximate 30 ml volume to 6—7 ml. An aliquot is taken for total protein analysis and the remainder is fractionated according to the methods of Example 1 for ASTROCYTIN Precursor.

Example 5
Production of purified MALIGNIN product from crude MALIGNIN-containing fraction

The product MALIGNIN is further isolated from contaminants by the methods of Example 2 for ASTROCYTIN.

In the TLG step of the preferred embodiment, the product MALIGNIN is observed as a discrete spot at a distance of approximately $0.91 \pm 0.02$ with reference to the standard cytochrome C, yielding an approximate molecular weight of 10,000 for MALIGNIN.

The product MALIGNIN which has been produced at this stage is soluble in distilled water, soluble at neutral or acid pH values, and insoluble at an alkaline pH, and has a spectrophotometric absorption peak of 280 m$\mu$. It is a polypeptide with a molecular weight of approximately 10,000.

The molecular weights of MALIGNIN produced in fermentation cultures stabilized in successive generations of the cultures (as shown by the thin layer gel chromatography determinations) are set forth in Table II (below). The reproducibility of the molecular weight determinations is remarkable in view of the inherent limitations of TLG chromatography.

# 0 015 755

TABLE II
Reproducibility of molecular weight of Malignin produced

| Run No. | Mol wt | Run No. | Mol wt | Run No. | Mol wt |
|---|---|---|---|---|---|
| 1 | 9,500 | 9 | 10,100 | 17 | 10,180 |
| 2 | 8,900 | 10 | 10,180 | 18 | 10,190 |
| 3 | 10,000 | 11 | 10,180 | 19 | 10,190 |
| 4 | 10,050 | 12 | 10,180 | 20 | 10,180 |
| 5 | 10,100 | 13 | 10,180 | 21 | 10,000 |
| 6 | 10,000 | 14 | 10,050 | 22 | 9,500 |
| 7 | 10,150 | 15 | 10,180 | 23 | 10,180 |
| 8 | 12,500 | 16 | 10,190 | | |

MALIGNIN's covalently linked aminoacids are shown by hydrolysis with 6N HCl followed by quantitative determination to have the following average composition of aminoacids:

| | Approximate number of residues |
|---|---|
| Aspartic acid | 9 |
| Threonine | 5 |
| Serine | 5 |
| Glutamic acid | 13 |
| Proline | 4 |
| Glycine | 6 |
| Alanine | 7 |
| Valine | 6 |
| 1/2 Cysteine | 1 |
| Methionine | 2 |
| Isoleucine | 4 |
| Leucine | 8 |
| Tyrosine | 3 |
| Phenylalanine | 3 |
| Lysine | 6 |
| Histidine | 2 |
| Arginine | 5 |
| Approximate Total | 89 |

The aminoacids cysteic acid, hydroxyproline, norleucine, ammonia, isodesmosine, desmosine, hydroxylysine, lysinonorleucine and gamma-aminobutyric acid are absent in detectable amounts.

A typical yield of pure MALIGNIN from twelve 250 ml reaction chambers of Example 3 together is approximately 1 mg of MALIGNIN.

Example 5A
Production of RECOGNIN L

Malignant cells grown in tissue culture, a lymphocytic lymphoma line of cells designated $P_3J$, were obtained from the Mason Research Institute, Rockville, Maryland, U.S.A.

Approximately 1 gm of packed cells of each of these were not further propagaged upon receipt but extracted immediately, and the Recognin was produced according to the identical protocol used for producing Malignin from glioma cells (Examples 3, 4 and 5). Thus, the entire medium plus cells was transferred to centrifuge tubes with cold 0.005 H phosphate buffer, at pH 7, and centrifuged at 3,000 rpm in the cold for 10 minutes, the medium was discarded, the cells were washed twice with cold buffer and centrifuged again twice as before, and the washings were discarded. The washed cells were suspended in the same buffer and disrupted by sonification for 20 seconds. The cell residues were centrifuged at 30,000 rpm for 30 minutes, the solubilized protein the supernatant material was decanted and collected, and the cell residues were sonified twice more, until no further appreciable protein was solubilized. The solubilized protein was concentrated and the recognin was cloven and purified by Cellex   D (trade mark) (BioRad) and Sephadex 200 (trade mark) (Pharmacia) gel chromatography. The yield, molecular weight, aminoacid composition, behaviour on thin layer gel chromatography and immunological properties of this polypeptide were similar to those of Astrocytin and Malignin. The yield in the case of $P_3J$ cells was approximately 1 mg/g wet weight of cells.

The covalently linked aminoacids of (Recognin L are shown by hydrolysis *(in vacuo)* with 6N HCL at 108°C for 12 hours, followed by quantitative aminoacid determination, to have the following

12

average composition of aminoacids (the nearest integer for the mole number of each amino acid is the average of two separate determinations):

| | Number of residues |
|---|---|
| Threonine | 5 |
| Serine | 5 |
| 1/2 Cysteine | 1 |
| Methionine | 1 |
| Valine | 6 |
| Isoleucine | 4 |
| Phenylalanine | 3 |
| Lysine | 6 |
| Histidine | 2 |
| Arginine | 5 |
| Aspartic acid | 8 |
| Glutamic acid | 10 |
| Leucine | 7 |
| Tyrosine | 1 |
| Proline | 5 |
| Glycine | 13 |
| Alanine | 10 |
| Approximate total | 92 |

Example 5B

Production of RECOGNIN M

Malignant cells grown in tissue culture, a mammary carcinoma cell line designated MCF-7, were obtained from the Mason Research Institute, Rockville, Maryland, U.S.A.

Approximately 1 gm of packed cells of MCF-7 was not further propagated upon receipt but extracted immediately, and the Recognin was produced according to the protocol used for producing Malignin from glioma cells (Examples 3, 4 and 5). Thus, the entire medium plus cells was transferred to centrifuge tubes with cold 0.005 M phosphate buffer, pH 7, and centrifuged at 3,000 rpm in the cold for 10 minutes, the medium discarded, the cells washed twice with cold buffer, centrifuged again twice as before, and the washings discarded. The washed cells were suspended in the same buffer and disrupted by sonification for 20 seconds. The cell residues were centrifuged at 30,000 rpm for 30 minutes, the solubilized protein in the supernatant material was decanted and collected, and the cell residues were sonified twice more, until no further appreciable protein was solubilized. The solubilized protein was concentrated and the recoginin was cloven and purified by Cellex D (trade mark) (BioRad) and Sephadex 200 (trade mark) (Pharmacia) gel chromatography. The yield, molecular weight, aminoacid composition, behaviour on thin layer gel chromatography and immunological properties of this polypeptide are similar to those of Astrocytin and Malignin, the previous two cancer recognins described. The yield in the case of MCF-7 cells was approximately 1 mg/g wet weight of cells.

The covalently linked aminoacids of Recognin M are shown, by hydrolysis *(in vacuo)* with 6N HCL at 108°C for 12 hours followed by quantitative automatic determination, to have the following average composition of aminoacids (the nearest integer for the mole number of each aminoacid is the average of two separate determinations):

|  | Number of residues |
|---|---|
| Threonine | 5 |
| Serine | 5 |
| 1/2 Cysteine | 1 |
| Methionine | 1 |
| Valine | 6 |
| Isoleucine | 4 |
| Phenylalanine | 3 |
| Lysine | 6 |
| Histidine | 2 |
| Arginine | 5 |
| Aspartic acid | 9 |
| Glutamic acid | 11 |
| Leucine | 8 |
| Tyrosine | 2 |
| Proline | 4 |
| Glycine | 9 |
| Alanine | 9 |
| Approximate total | 90 |

The unique structures of MALIGNIN, ASTROCYTIN, RECOGNIN M and RECOGNIN L were confirmed by an exhaustive computerized search which compared their compositions with virtually all known protein substances.

The aminoacid composition of MALIGNIN and ASTROCYTIN in terms of the absolute and relative amounts of each aminoacid component based on the total number of aminoacid residues per mole, and the absolute and relative amounts of each aminoacid component in terms of the molecular weight of the molecule, were submitted to matrix computer analysis against the largest known library of protein structures in the world, i.e., that of the National Biomedical Research Foundation, Washington, D.C., U.S.A. No structure identical to, or even very close to, that of ASTROCYTIN, MALIGNIN, RECOGNIN M or RECOGNIN L was found in the matrix analysis comparison with several hundred thousand proteins and protein fragments.

The only proteins that were structurally related in any way are shown in Table III (below) with their individual amino compositions and molecular weights for comparison. The computer is programmed to identify, from the several hundred thousand possibilities, any degree of similarity in structure. Thus, for example, proteins of molecular weight much larger or smaller will not match, nor those with fewer than 85 or more than 95 residues, nor those with fewer than 10 or more than 15 glutamic acid residues, nor those with fewer than 6 or more than 11 aspartic acid residues, and so on for each of the twenty aminoacids involved.

14

## TABLE III

### Comparison by computer search of the structures of Recognins with nearest structures

| | Astrocytin | Malignin | Recognin M | Recognin L | Cytochrome $b_5$ |
|---|---|---|---|---|---|
| Aspartic acid | 9 | 9 | 9 | 8 | 9 |
| Threonine | 5 | 5 | 5 | 5 | 6 |
| Serine | 6 | 5 | 5 | 5 | 5 |
| Glutamic acid | 13 | 13 | 11 | 10 | 14 |
| Proline | 4 | 4 | 4 | 5 | 3 |
| Glycine | 6 | 6 | 9 | 13 | 6 |
| Alanine | 9 | 7 | 9 | 10 | 4 |
| Valine | 4 | 6 | 6 | 6 | 4 |
| 1/2 Cysteine | 2 | 1 | 1 | 1 | 0 |
| Methionine | 1 | 2 | 1 | 1 | 1 |
| Isoleucine | 2 | 4 | 4 | 4 | 4 |
| Leucine | 8 | 8 | 8 | 7 | 7 |
| Tyrosine | 2 | 3 | 2 | 1 | 3 |
| Phenylalanine | 3 | 3 | 3 | 3 | 3 |
| Lysine | 8 | 6 | 6 | 6 | 7 |
| Histidine | 2 | 2 | 2 | 2 | 7 |
| Arginine | 4 | 5 | 5 | 5 | 3 |
| Asparagine | 0 | 0 | 0 | 0 | 0 |
| Tryptophane | 0 | 0 | 0 | 0 | 1 |
| Glutamine | 0 | 0 | 0 | 0 | 0 |
| Total no. residues | 88 | 89 | 90 | 92 | 87 |
| Molecular weight | *8,000 (9,690) | *10,000 (10,067) | *8,000 (9,870) (by calculation) | *8,000 (9,606) | 10,035 |

*By thin layer gel chromatography.

### TABLE III (Continued)

| | Ferredoxin Luc. G1 | Ferredoxin Alf. | Acyl-carrier E. Coli. | Neurophysin bovine | Neurophysin pig | Gonadotropin Releas. |
|---|---|---|---|---|---|---|
| Aspartic acid | 10 | 8 | 7 | 2 | 3 | 0 |
| Threonine | 4 | 6 | 6 | 2 | 2 | 0 |
| Serine | 7 | 8 | 3 | 6 | 7 | 1 |
| Glutamic acid | 13 | 13 | 14 | 9 | 9 | 0 |
| Proline | 5 | 3 | 1 | 8 | 7 | 1 |
| Glycine | 7 | 7 | 4 | 16 | 14 | 2 |
| Alanine | 6 | 9 | 7 | 6 | 7 | 0 |
| Valine | 6 | 9 | 7 | 4 | 2 | 0 |
| 1/2 Cysteine | 5 | 5 | 0 | 14 | 14 | 0 |
| Methionine | 0 | 0 | 1 | 1 | 1 | 0 |
| Isoleucine | 4 | 4 | 7 | 2 | 2 | 0 |
| Leucine | 10 | 6 | 5 | 6 | 7 | 1 |
| Tyrosine | 3 | 4 | 1 | 1 | 1 | 1 |
| Phenylalanine | 3 | 2 | 2 | 3 | 3 | 0 |
| Lysine | 5 | 5 | 4 | 2 | 2 | 0 |
| Histidine | 1 | 2 | 1 | 0 | 0 | 1 |
| Arginine | 2 | 1 | 1 | 7 | 5 | 1 |
| Asparagine | 0 | 1 | 2 | 3 | 2 | 0 |
| Tryptophane | 1 | 1 | 0 | 0 | 0 | 1 |
| Glutamine | 4 | 3 | 4 | 5 | 4 | 0 |
| Total No. residues | 96 | 97 | 77 | 97 | 92 | 10 |
| Molecular weight | 10,493 | 8,509 | | | | |

This 'fingerprint' thus has some 22 individual variables to match. Some substances will match on one, on four or on five variables but none matches on all 22. In fact, none matches in better than 14 variables leaving differences in 8 variables.

Thus, the closest fit is cytochrome $b_5$ (human). However, as seen from Table III, the alanine, arginine, asparagine, aspartic acid, cysteine, glutamine, histidine, methionine and tyrosine and tryptophane residue numbers all differ from appreciably to markedly from the Recognins. Because cytochrome $b_5$ contains 7 histidines while Astrocytin, Malignin, Recognin L and Recognin M contains only 2, they could not possibly have the same chemical structure.

The most unusual thing about the composition of Astrocytin, Malignin, Recognin L and Recognin M is the high concentration of glutamic acid. One would normally expect to find only 5 or 6 glutamic acid residues in a total of 88 to 92 amins-acid residues instead of the 10 to 13 actually identified.

Other next-closest fits are the ferrodoxins of *leucaene glauca* and of alfalfa, respectively, but these also differ markedly in four and six aminoacids, respectively, and appreciably in two others, and in having 96 and 97 residues, respectively. The next-closest fit is in the acylcarrier protein of *E. Coli* 26, but this also differs markedly in eleven aminoacids from Malignin, Astrocytin, Recognin L and Recognin M and has only 77 residues.

Some other brain proteins (neurophysin, bovine and neurophysin, pig, and gonadotropin-releasing hormone) are listed in Table III to illustrate how much worse the match is for the remaining several hundred thousand protein fragments in the computer memory bank referred to above.

Respiratory proteins may contain metals and/or haeme components in their *in situ* state, but the isolated protein fragment, e.g., for apocytochrome $b_5$ contains neither. Additional microanalysis of Astrocytin and Malignin has shown them to be free of iron, sulphur, phosphorus and magnesium (all below 0.01%), and the spectral characteristics show typical absorption at 280 m$\mu$. Upon recombination of haeme with apoprotein, the typical absorption spectra between 400 m$\mu$ and 450 m$\mu$ are restored.

Despite the structural uniqueness of Astrocytin, Malignin, Recognin L and Recognin M as compared with all other proteins and protein fragments, it is noteworthy and perhaps of great importance that the closest structures are those of respiratory proteins. It is well known in the art that many important relationships can be drawn from both a developmental genetic point of view and from a functional point of view in the type of structure represented. If the Recognins are new protein products whose *in situ* structural equivalents represent respiratory functions, and these proteins are related, as is now shown, to malignancy, then an opening has been found to solve one of the great puzzles of cancer that is: how are the energetics satisfied for these voracious, rapidly reproducing malignant cells?

Aside from the theoretical importance of this discovery, the data above on the increase in the percentage of Malignin in more malignant cells, and the data provided below indicating that Anti-Malignin now only attaches to a Malignin-like chemical grouping of the cancer cell, but having so attached is cytotoxic to the cells, come together meaningfully. If the malignin-like *in situ* compounds in cancer cells are respiratory proteins, since the Anti-Malignin products of this invention attach preferentially to these *in situ* compounds, then, if functional respiratory groups are involved in this attachment. It is easy to understand how this results in the death of the cancer cells.

The therapeutic possibilities for the Anti-Malignins and other similar Chemoreciprocals are greatly strengthened by this structural information on Malignin and Astrocytin, as well as information on the demonstrated relationship of the amount of Malignin to the degree of Malignancy.

The molecular weights of the four Recognins described with particularity herein, i.e. Astrocytin, Malignin, Recognin L and Recognin M, calculated from their respective aminoacid compositions are close to each other (see Table III above). Thin layer gel chromatography with Sephadex G200 (trade mark) (Pharmacia) gave a molecular weight for both Recognin L and Recognin M of approximately 8,000, the same as that obtained with Astrocytin. Since the calculated molecular weight of Astrocytin, based upon quantitative aminoacid analysis, is 9,690, the lower apparent molecular weight of 8,000 obtained by thin layer chromatography may be, as in the case of MCF-7 and $P_3J$, a function of the slightly lower not acidic charge of these three polypeptides as compared with that of Malignin. The molar compositions of the constituent aminoacids of the four Recognins are also in close agreement with each other and (as shown by the computer comparison), different from those of all other known polypeptides. The fact that the only four polypeptides whose compositions are remotely related are phylogenetially ancient anaerobic oxidation-reduction enzymes has led to the suggestion that this may be an important clue to the basis of the anaerobic advantage of malignant cells recognized for many years but whose basis has not previously been defined. Whether the minor differences between the four cancer Recognins represent significant structural differences is as yet unknown. The composition of nine of the seventeen aminoacids detected is seen to be identical for each of the three products from those cells which were grown in tissue culture. The principal differences between the compositions of Recognin L and Recognin M when compared with that of Malignin, are a decrease in aspartic acid, glutamic acid, leucine and tyrosine, and an increase in proline, glycine and alanine.

Despite these chemical differences, the four Recognins are, up to the present, indistinguishable immunologically in each mode tested: (1) each reacts equally well on Ouchterlony double diffusion with anti-maligin antibody; and (2) separate immobilized (bound to solid support) antigen preparations of each showed them to be approximately equal in their ability to bind antibody from human serum regardless of the type of cancer from which the patient suffered. Thus, immobilized Recognin M was observed to bind antibody from the serum of brain glioma patients as well as did immobilized Malignin.

Example 5C

Demonstration of increased yield, degree of malignancy, and proportion of Malignin, by the provision of greater volume and surface area during fermentation

Examples 3 to 5 were repeated using 1000 cc flasks instead of 250 cc flasks. All quantities of reagents were increased threefold.

The yield of the product MALIGNIN after 7 days' growth in inoculum was increased almost twofold by increasing the space available for malignant cell growth from 250 cc to 1000 cc. Table IV (below) shows the yield of total protein in mg and the MALIGNIN produced as a percentage of said total protein for successive generations of fermentation cultures in each size flask. Using 250 cc flasks, the mean total protein produced was 17.5 mg. Using 1000 cc flasks, the mean total protein produced was 40.4 mg.

Surprisingly, as the amount of malignant cell growth (degree of malignancy) was increased per seven-day growth period by providing a greater space and surface for cell growth, the amount of Malignin produced, as a percentage of total protein, increased from a mean of 10.7 percent using 250 cc flasks to a mean of 28.3 percent using 1000 cc flasks for a constant growth period of seven days.

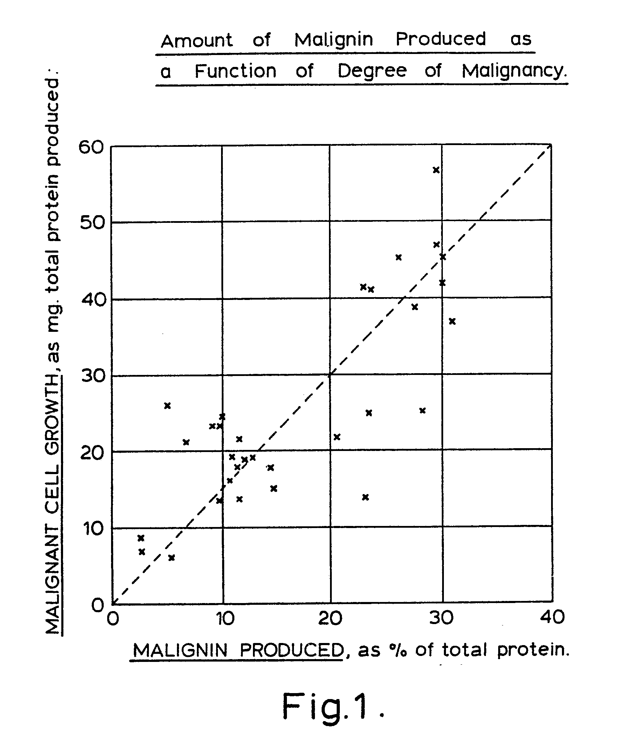

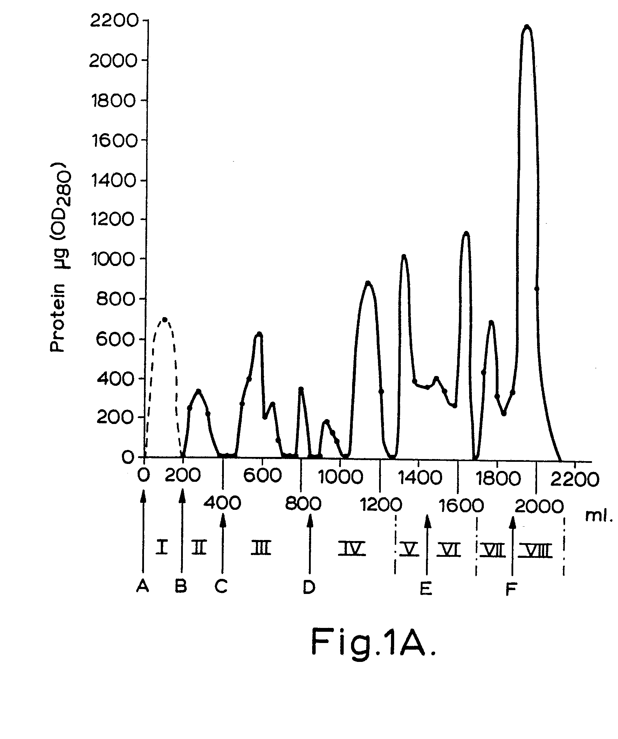

The relationship between the proportion of MALIGNIN produced, and the degree of malignancy (i.e., as a function of the amount of malignant cell growth *in vitro* in seven days, measured as total protein produced) is shown in Fig. 1 of the accompanying drawings.

TABLE IV

Improved yields in successive generations of fermentation culture production of Malignin

| Flask size | Malignin, mg | Total protein, mg | % Malignin |
|---|---|---|---|
| 250 cc | .33 | 6.4 | 5.1 |
| " | .16 | 6.7 | 2.4 |
| " | .21 | 8.9 | 2.4 |
| " | 1.3 | 26.3 | 4.8 |
| " | 1.4 | 21.6 | 6.4 |
| " | 2.6 | 17.9 | 14.4 |
| " | 1.8 | 16.4 | 10.7 |
| " | 1.3 | 13.4 | 9.8 |
| " | 2.0 | 17.8 | 11.3 |
| " | 2.3 | 18.9 | 12.0 |
| " | 2.1 | 19.4 | 10.8 |
| " | 1.6 | 13.8 | 11.6 |
| " | 2.2 | 15.1 | 14.6 |
| " | 4.4 | 21.6 | 20.4 |
| " | 3.3 | 14.0 | 23.2 |
| " | 2.2 | 23.0 | 9.7 |
| " | 2.1 | 23.2 | 9.0 |
| " | 2.8 | 22.3 | 12.5 |
| " | 2.4 | 18.9 | 12.7 |
| " | 2.4 | 24.5 | 9.8 |
| | Mean | 17.5 mg | 10.7% |

TABLE IV (contd.)

| Flask size | Malignin, mg | Total protein, mg | % Malignin |
|---|---|---|---|
| 1000 cc | 9.8 | 41.3 | 23.6 |
| " | 7.2 | 25.4 | 28.4 |
| " | 5.9 | 24.9 | 23.6 |
| " | 11.7 | 37.5 | 31.2 |
| " | 13.3 | 44.8 | 29.8 |
| " | 16.5 | 56.5 | 29.4 |
| " | 9.5 | 41.3 | 22.9 |
| " | 10.7 | 38.8 | 27.5 |
| " | 12.5 | 41.6 | 29.9 |
| " | 13.3 | 46.7 | 29.4 |
| " | 11.6 | 45.2 | 25.7 |
| | Mean | 40.4 mg | 28.3% |

Fig. 1 shows that the increased flask size results in approximately trebeled proportions of MALIGNIN product.

Fig. 1 also demonstrates a relationship between MALIGNIN and malignancy. The interrupted line represents an ideal linear relationship. This demonstrated relationship is clearly not trivial since the proportion of MALIGNIN present increases as the cells become more unrestrained (Pathological) in their growth. The normal *in situ* Recognin function relates, as previously stated, to the contact inhibition of growth of cells. The more pathological the growth of the malignant cells, the less contact inhibition operates, and the more MALIGNIN becomes the predominant protein.

Example 5C demonstrates that the growth of artificial cancer cell culture in large size growth containers unexpectedly results in an increased proportion of MALIGNIN produced, that is, in an increase in the percentage of total protein produced which is MALIGNIN. As used in this application, a large size growth container means one in which the ratio of the container volume to the volume of total medium which cells utilize in accordance with the methods of Example 3 is greater than 7:1 for example, from 7:1 to 10:1. Example 5C illustrates a ratio of 8:1.

Example 6
Production of TARGET reagents from RECOGNINS

ASTROCYTIN, prepared as in Example 2 above, or MALIGNIN, prepared as in Example 5 above, or Recognin L or Recognin M, is complexed with a carrier to produce TARGET reagent.

In the preferred embodiment, ASTROCYTIN or MALIGNIN or Recognin L or Recognin M, i.e., the RECOGNIN (the procedure works in the same way for each), is dissolved in 0.15 M $NaH_2PO_4$-citrate buffer, pH 4.0. A bromoacetyl-resin, for example bromoacetylcellulose (BAC) having 1.0 to 1.5 milli-equivalents of Br per gram of cellulose, stored in the cold, is prepared in 0.15 M $NaH_2PO_4$ buffer, pH 7.2. The buffer is converted to pH 4 by pouring off the pH 7.2 buffer solution and adding 0.15 M $NaH_2PO_3$-citrate buffer, pH 4.0. The RECOGNIN solution and the BAC solution are stirred together (10:1 BAC to RECOGNIN ratio) for 30 hours at room temperature, and then centrifuged.

It is preferred that all sites on the BAC which are available to bind to RECOGNIN be bound. This may be accomplished as follows. The supernantant material from the immediately preceding step is lyophilized and the protein content is determined to indicate the amount of RECOGNIN not yet complexed to BAC. The complexed BAC-ASTROCYTIN (or BAC-MALIGNIN) is resuspended in 0.1 M bicarbonate buffer at pH 8.9, and stirred for 24 hours at 4° to permit the formation of chemical bonds between the BAC and the RECOGNIN. After 24 hours, the suspension is centrifuged and supernatant material is analysed for protein. The complexed BAC-RECOGNIN is now resuspended in 0.05 M aminoethanol-0.1 M bicarbonate buffer, pH 8.9, in order to block any unreacted bromine. The suspension is centrifuged, and the supernatant material is kept but not analysed because of the presence of aminoethanol. The removal of all unbound RECOGNIN is then accomplished by centrifuging and resuspension for three washings in 0.15 M NCl until no absorbence is measured on the spectrophotometer at 266 m$\mu$. The BAC-RECOGNIN complex is now stirred in 8 M urea for 2 hours at 38°C, centrifuged, and washed (three times usually suffices) with 8 M urea until no absorbence is shown in the washings at 266 m$\mu$. The complex is then stirred at 37°C in 0.25 M acetic acid for 2 hours to demonstrate its stability. The material is centrifuged and the absorbence of the supernatant material is read at 266 m$\mu$; no absorbence should be present. This chemically complexed BAC-RECOGNIN is therefore stable and can now be used as a (TARGET) reagent in the methods described below; in this stable reagent form, it is referred to as a synthetically produced complex whose physical and chemical properties mimic the stable cell-bound precursor of the RECOGNIN when it is in a potentially reactive state with serum components. For storage, the TARGET reagent is centrifuged and washed until neutralized with 0.05 M $NaH_2PO_4$ buffer, pH 7.2.

TARGET reagents may be prepared from bromacetyl liganded carriers other than cellulose, such as bromoacetylated resins or even filter paper.

Example 7
Production of Antisera to Astrocytin, Malignin, and TARGET

Antisera to Astrocytin, Malignin or TARGET reagents may be produced by inducing an antibody response in mammal. The following procedure have been found to be satisfactory.

One mg of the RECOGNIN is injected into the toe pads of white male rabbits with standard Freund's adjuvant, and the same injection is then made intraperitoneally one week later, again intraperitoneally ten days later and, if necessary, three weeks later. Specific antibodies may be detected in the blood serum of these rabbits as early as one week to ten days after the first injection. The same procedure is followed for TARGET antigen by injecting that amount of target which contains 1 mg of Astrocytin or Malignin as determined by the Folin-Lowry determination of protein.

The specific antibody to Astrocytin is named anti-Astrocytin. The specific antibody to Malignin is named Anti-Malignin. Similarly, the specific antibody to TARGET reagent is named Anti-Target. Similarly, the specific antibodies to Recognin L and M, respectively, are named Anti-Recognin L and Anti-Recognin M, respectively.

# 0 015 755

These antibodies show clearly on standard Ouchterlony gel diffusion tests for antigen-antibody reactions with specific single sharp reaction lines produced with their specific antigen.

The presence of specific antibodies in serum can also be tested by the standard quantitative precipitin test for antigen-antibody reactions. Good quantitative precipitin curves are obtained and the micrograms of specific antibody can be calculated therefrom.

Further evidence of the presence of specific antibodies in serum can be obtained for example by absorption of the specific antibody Anti-Astrocytin onto Bromoacetyl-cellulose-Astrocytin (BAC-Astrocytin) prepared above. Thus, the antiserum containing specific Anti-Astrocytin can be reacted with BAC-Astrocytin. When the serum is passed over BAC-Astrocytin, only the specific antibodies to Astrocytin bind to their specific antigen Astrocytin. Since Astrocytin is covalently bound to Bromoacetyl-cellulose the specific antibody, Anti-Astrocytin, is now bound to BAC-Astrocytin to produce BAC-Astrocytin-Anti-Astrocytin (BACA-Anti-Astrocytin). This is proved by testing the remainder of the serum which is washed free from BAC-Astrocytin. On standard Ouchterlony diffusion, no antibodies now remain in the serum which will react with Astrocytin. It is therefore concluded that all specific antibodies (Anti-Astrocytin) previously shown to be present in the serum have been absorbed to BAC-Astrocytin. Furthermore, when Anti-Astrocytin is released from its binding to BAC-Astrocytin, it is thereby isolated so as to be free of all contaminating antibodies. This release of Anti-Astrocytin may be accomplished by washing the BACA-Anti-Astrocytin coupled with 0.25 M acetic acid (4°C, 2 hours) which has been shown above not to break the BAC-Astrocytin bond.

The antibodies to TARGET reagents show clearly on standard Ouchterlony gel diffusion tests for antigen-antibody reactions with specific single reaction lines produced with TARGET reagents which show a line of identity with the line of reaction to ANTI-ASTROCYTIN or ANTI-MALIGNIN antisera (i.e. that produced in response to the injection of ASTROCYTIN or MALIGNIN itself). Some rabbits, it has been noted, have levels of ANTI-TARGET in their blood prior to being injected with TARGET. These ANTI-TARGET substances, when reacted specifically with TARGET reagent as to be described in tests of human sera, lead to the production of approximately equivalent amounts of the two types of TAG, S-TAG and F-TAG (see later Examples).

Example 8

Detection of malignant tumours by quantitative production *in vitro* of TARGET-ATTACHING-GLOBULINS (TAG products) from biological fluids

TARGET reagent prepared in accordance with Example 6 is washed to remove any of the unbound RECOGNIN which may be present due to deterioration. The following procedure is satisfactory. TARGET reagent is stirred for two hours at 37°C with acetic and then centrifuged, the supernatant material is decanted, and the optical density of the supernatant material is read at 266 m$\mu$. If there is any absorbence, this wash is repeated until no further material is solubilized. The TARGET reagent is then resuspended in phosphate buffered saline, pH 7.2 (Standard S-TAG and F-TAG purified from the previous reactions of human serum by the procedure described below can be used, if available, as reference standards to test the TARGET reagent, as can whole rabbit serum which has been determined to contain S-TAG and F-TAG by other TARGET preparations).

The slow-Binding (S-TAG) determination is performed as follows: Frozen serum stored for more than a few days should not be used. Serum is carefully prepared from freshly obtained whole blood or other body fluid by one of various standard procedures known in the art. The following procedure has been found to be satisfactory. Blood is allowed to clot by standing for 2 hours at room temperature in a glass test tube. The clots are separated from the walls with a glass stirring rod, and the blood is allowed to stand at 4°C for a minimum of 2 hours (or overnight). The clots are separated from the serum by centrifuging at 20,000 rpm and at 4°C for 45 minutes. The serum is decanted into a centrifuge tube and centrifuged again but this time at 2,000 rpm at 4°C for 45 minutes. The serum is decanted and a 1% Solution of Methiolate (1 g in 95 ml water and 5 ml of 0.2 M bicarbonate buffer, pH 10) is added to the extent of 1% of the volume and serum.

In duplicate determination, serum samples of 0.2 ml, each prepared by the above or similar procedures, are added to each of 0.25 ml aliquots of TARGET suspension reagent containing 100—200 micrograms of the RECOGNIN per 0.25 ml TARGET reagent. Each resulting suspension is mixed at 4°C in such manner as to avoid pellet formation. For example, a small rubber cap rapidly shaken may be used for 1—2 seconds and, then, with the tubes slightly slanted, they may be shaken in a Thomas shaker for 2 hours or more. The TARGET reagent and the protein bound thereto are separated from the serum. One procedure which has been found to be satisfactory is as follows. The tubes are centrifuged at 2,000 rpm for 20 minutes at 4°C, the supernatant material is decanted, the pellet which is formed by centrifuging is washed 3 times by remixing and shaking at room temperature with 0.2—0.3 ml of 0.15 M Saline, and centrifuged, and the supernatants are discarded.

The protein which remains attached to the TARGET reagent is cleaved therefrom and quantitatively determined. For example, 0.2 ml of 0.25 M acetic acid is needed, and the suspension is shaken for 1 to 2 hours in a 37°C incubator. The tubes are centrifuged at 2,000 rpm and at 4°C for 30 minutes. The supernatant material is carefully decanted to avoid transferring particles and the optical density of the supernatant is read at 280 m$\mu$. The value of the optical density is divided by a factor of 1.46 for results

19

in micrograms per ml of serum protein (S-TAG). Duplicate determinations should not vary by more than 5%. Any other procedure effective for determining protein content may be used, such as a Folin-Lowry determination, but standards must be specified to determine the range of control and tumour values of S-TAG minus F-TAG concentration.

The Fast-Binding (F-TAG) determination is performed as follows: Frozen serum stored for more than a few days should not be used. Serum is carefully prepared from freshly obtained whole blood or other body fluid by standard procedures in the art. The procedure given above in this Example for serum preparation is satisfactory.

Serum samples, prepared by the above or like procedures, are allowed to stand at 4°C for 10 minutes less than the total time the S-TAG serum determinations were allowed to be in contact with the TARGET reagent above [e.g., 1 hour, 50 minutes if a "two hour" S-TAG determination was made]. This procedure equilibrates the temperature histories of S-TAG and F-TAG determinations.

0.2 ml samples of the temperature-equilibrated serum were added to each of 0.25 ml aliquots of TARGET suspension reagent containing 100—200 micrograms of the RECOGNIN per 0.25 ml of TARGET reagent, in duplicate determination. The suspension is then mixed at 4°C for approximately 10 minutes in such manner as to avoid pellet formation. For example, a small rubber cap rapidly shaken may be used for 1—2 seconds, and, then, with the tubes slightly slanted, they may be shaken in a Thomas shaker for approximately 10 minutes. The TARGET reagent and protein bound thereto are separated from the serum. One procedure which has been found to be satisfactory is as follows. The tubes are centrifuged at 2,000 rpm for 20 minutes at 4°C, the supernatant is decanted, and the pellet which is formed by centrifuging is washed 3 times by remixing and shaking at room temperature with 0.2—0.3 ml of 0.15 M Saline, further centrifuging is carried out, and the supernatants are discarded.

The protein which remains attached to the TARGET reagent is cleaved therefrom and quantitatively determined. The procedure described above in this Example for determining S-TAG concentration is satisfactory. Various other procedures effective to determine protein content may be used, e.g. a Folin-Lowry determination, but standards must be specified to determine the range of control and tumour values of S-TAG minus F-TAG concentration.

The final results are expressed as TAG micrograms per ml of Serum, and equal S-TAG minus F-TAG. TAG values in non-brain-tumour patients and other controls currently range from zero (or a negative number) to 135 or 140 micrograms per ml of serum. In any result over 100 $\mu$g/ml, a repeat determination is indicated. Some other tumours may yield high TAG values, especially if secondary (metastic) tumours are present in the brain. TAG values in brain tumour patients currently range from 136 to 500 or more micrograms per ml of serum. In the first "blind" study of 50 blood samples conducted according to the procedures of this Example utilizing TARGET reagent prepared from Astrocytin and bromoacetylcellulose, 11 out of 11 brain tumours and 28 out of 32 normals were correctly identified. One of the 4 supposed normals (i.e. non-brain-tumour controls) turned out to have a cancer of the thyroid gland which had apparently been successfully treated some years before. The three remaining normals were individuals aged 60—70 who were in poor health, possibly having non-diagnosed cancer. Of the remaining 7 samples, three out of three cases of Hodgkin's Disease were correctly identified; one sample in the tumour range (136—500 $\mu$g. TAG/ml) corresponded to a patient having a severe gliosis, and three samples in the non-tumour range (0—135 $\mu$g. TAG/ml) corresponded to patients having respectively an intercranial mass diagnosis uncertain but non-tumour, an osteosarcoma (non-brain tumour) and a melanotic sarcoma (nonbrain tumour).

A subsequent study was conducted according to the procedure of this example utilizing TARGET reagent prepared from Malignin and malignant brain tumours and all normals.

A still further study conducted according to the procedure of this example extended the total number of human serum specimens tested from 50 to 114. The utility of these products and procedures is demonstrated in Table V which records below the results of these tests (see Example 13 below for results on 290 specimens determined blind).

TABLE V

| Normals* | | | | Malignant brain tumours, primary |
|---|---|---|---|---|
| Serum TAG $\mu$g/ml | Serum TAG $\mu$g/ml | Serum TAG $\mu$g/ml | Serum TAG $\mu$g/ml | Serum TAG $\mu$g/ml |
| 124 | 19 | 54 | 65 | 459 |
| 113 | 55 | 27 | 113 | 397 |
| 105 | 51 | 41 | 130 | 236 |
| 130 | 82 | 21 | 79 | 137 |
| 127 | 44 | 27 | 61 | 298 |
| 38 | 127 | 21 | 123 | 397 |
| 100 | 31 | 0 | 14 | 241 |
| 125 | 0 | 14 | 20 | 241 |
| 30 | 125 | 62 | 41 | 217 |
| 250[1] | 118 | 38 | 34 | 147 |
| 39 | 89 | 93 | 93 | 127 |
| 363[1] | 99[6] | 21 | 48 | 185 |
| 4 | 13[2] | 0 | 20 | 253 |
| 31 | 270[3] | 120 | 82 | 253 |
| 42 | 7 | 16 | 20 | 565 |
| 34 | 58 | 20 | 55 | 277 |
| 76 | 24 | 113 | 0 | 137 |
| 48 | 62 | 72 | | 78[13] |
| 85 | 89 | | | 138 |
| | 89 | | | 650 |
| | | | | 160 |

| Malignant other tumours, brain secondaries | Malignant other tumours, no brain secondaries | Uncertain cerebral diagnosis |
|---|---|---|
| Serum TAG $\mu$g/ml | Serum TAG $\mu$g/ml | Serum TAG $\mu$g/ml |
| 270 | 34[4] | 165[9] |
| 257 | 31[5] | 144[9] |
| 188 | 442[14] | 13[9] |
| 205 | 288[14] | 209[10] |
| 157[7] | | 75[10] |
| | | 184[11] |
| | | 27[11] |
| | | 110[12] |
| | | 192[15] |

*Includes normals, non-tumour medical and surgical disorders. 1—very ill; undiagnosed, 2—Extra brain intracranial mass, undiagnosed, 3—Marked gliosis, 4—Malignant melanoma, 5—Osteosarcoma, 6—Brain cyst fluid, 7—Adenocarcinoma of colon, 8—Gastrectomy, 9—Headaches, 10—Emphysema, 11—Polymyalgia, 12—Colon cancer, 13—Convulsions, 14—Cancer of prostrate, secondaries to bone, 15—Clinically "normal", 18—Months earlier, when this abnormal serum TAG obtained: Now developed severe headaches, loss of smell and taste.

Example 9

Diagnosis of tumour cells by immunofluorescence

The compounds Anti-Astrocytin, Anti-Malignin and S-TAG, for example, have been shown to attach preferentially to tumour cells. The specificity permits the use of these compounds to diagnose tumour cells in histology sections by conjugating dyes or radioactive substances to Anti-Astrocytin, Anti-Malignin or S-TAG. Standard labelling techniques may then be used. A procedure using S-TAG is as follows.

One procedure which has been found satisfactory is a modified St. Marie Procedure. Human brain tumour specimens are frozen and 5 micron-thick sections are cut. They are stored in a moist container at minus 70°C for from 4 to 8 weeks before staining. The conjugate may be a standard antiserum such as a goat anti-rabbit conjugate. The conjugate is labelled by techniques known in the art with a fluorescent or other labelling substance. Fluorescein-labelled goat anti-rabbit conjugate as commercially available may be used. The fluorescent technique used was a standard one in which a 1:200 to 1:400 solution of TAG was incubated for 30 minutes or more on the tumour section, followed by washes to remove unattached TAG. Three washes with phosphate-buffered saline were found satisfactory. Conjugate incubation with fluorescein-labelled conjugate followed by washes was then performed, followed by microscopic inspection. Normal cells and their processes failed to stain both in tumour sections and in control sections of normal non-tumour brain. Fluorescence was brightly present in tumour glial cells and their processes.

Example 10

Demonstration that Anti-Astrocytin, Anti-Malignin and S-TAG are cytotoxic to tumour cells growing in tissue culture

Standard tests for determining cytoxicity may be used. Generally, the number of cells in a fixed counting chamber, usually arranged to contain about 100 live cells, are counted. These cells are then treated with the agent being tested and the number of cells which are still alive is determined.

In a standard test of cytotoxicity of S-TAG Solution obtained in accordance with the methods of Example 8 against cells in tissue culture derived from a patient with a glioblastoma Grade III—IV, well characterized as of glial origin, S-TAG produced the death of all cells in the counting chamber even when in high dilutions of 1:100 and 1:1,000, respectively representing as little as 0.2 and 0.02 ug, respectively of S-TAG per ml of solution. Similar results were obtained with high dilutions of Anti-Astrocytin and Anti-Malignin.

Both the specificity exhibited in Example 9 and the cytotoxicity demonstrated in Example 10 are highly relevant to the therapeutic possibilites of Anti-Astrocytin, Anti-Malignin and S-TAG for brain tumours in man. These therapeutic users are in addition to the practical diagnostic potential of both of these phenomena for tumour tissue removed at operation but requiring diagnosis by histology already demonstrated herein.

Example 11

Hydrolytic cleavage of RECOGNINS

A solution of RECOGNIN, in this case, either Astrocytin or Malignin, at a pH between 1 and 2 is allowed to stand in the cold. After 7 to 14 days, TLG chromatography shows the product to have been reduced in molecular weight by approximately 200. When the solution is allowed to stand longer, further units of approximately 200 molecular weight are cleaved every 7 to 10 days. Thus, with astrocytin, the molecular weight is reduced from 8,000, and, with MALIGNIN, the molecular weight is reduced from 10,000, in each case by units of approximately 200, sequentially.

The physiochemical specificities of Astrocytin are retained by each product down to approximately 4,000 molecular weight. The physiochemical specifities of Malignin are retained by each product shown to approximately 5,000 molecular weight. This is shown by Ouchterlony gel diffusion tests against Anti-Astrocytin and Anti-Malignin, respectively.

This cleavage can also be accomplished enzymatically, as with trypsin and other proteinases, with similar results.

The molecular weights of the compounds prepared by hydrolytic cleavage of RECOGNINS may be approximately defined by the following formulae:

For products having the physiochemical specificities of Astrocytin:

$$400+200X=Y$$

For products having the physiochemical specificities of Malignin:

$$5,000+200X=Y,$$

wherein Y is the molecular weight of the product, and X is 0 or an integer of from 1 to 19.

Example 12

Production of artificial tissue or organ with RECOGNINS

A rigid walled tube of plastics, metal, or other suitable rigid material, is dipped into, or impregnated with, a highly concentrated [i.e., 10 mg/ml], viscous solution of a RECOGNIN, in this case, either Astrocytin or Malignin, until all surfaces are fully coated with the RECOGNIN. Alternately, a RECOGNIN solution is passed through and around the tube under pressure until all surfaces are fully coated. The tube is then dried in air or in vacuo, or lyophilized. The process of coating is repeated several times in order to build up multiple molecular layers of the RECOGNIN coating.

**O O15 755**

The tube is now ready to be placed in a cavity or in a tissue which contains Astrocytin- or Malignin-like precursors in the neighbouring tissue or fluid of a living mammal. This artificial tissue or organ may be used to minimize or to eliminate reactions which foreign substances without such RECOGNIN coating would incite.

Artificial tissues or organs of other geometries may similarly be produced.

Example 13

Malignin is useful in a serum diagnostic test for the detection of a wide variety of tumours, including non-brain malignancies. The results of determinations on individual sera, and remarks related thereto, are reported in the following table (Table VI):

TABLE VI

Anti-Malignin antibody determination in non-brain cancers with Malignin-based Target reagent

| Non-brain[a] Malignancies | Number of serums studied | Number abnormally elevated | Number borderline | Number in normal range |
|---|---|---|---|---|
| Carcinoma of: | | | | |
| Lung | 6 | 5 | | 1 |
| Breast[g] | 10 | 10 | | |
| Colon[g] | 7 | 7 | | |
| Rectum[g] | 6 | 5 | | 1 |
| Prostate | 2 | 2 | | |
| Bladder | 1 | 1 | | |
| Ovary | 4 | 2 | 1 | 1 |
| Kidney | 1 | 1 | | |
| Thyroid | 1 | 1 | | |
| Undifferentiated | 2 | 2 | | |
| Lymphoma | 10 | 9 | | 1 |
| Hodgkins's disease | 6 | 5 | 1 | |
| Multiple Myeloma | 6 | 6 | | |
| Acute Myelogenous leukaemia | 2 | 2 | | |
| Chronic Myelogenous leukaemia[b] | 5 | 5 | | |
| Chronic Lymphocytic leukaemia[c] | 3 | 2 | 1 | |
| Osteogenic Sarcoma | 1 | | | 1 |
| Melanotic Sarcoma | 4 | 3 | | 1 |
| Total non-brain malignancies | 77 | 68 | 3 | 6 |
| Brain cancer[d, g] | 85 | 80 | 1 | 4 |
| Total malignancies | 162 | 148 | 4 | 10 |
| Non-malignant medical and surgical disorders | 51 | 3 | —[e] | 48 |
| Normal | 77 | 3 | —[f] | 74 |

## O 015 755

TABLE VI (Continued)

| Non-brain[a] malignancies | % [c]Elevated | % Elevated+ borderline | Antibody bound, as protein complexes in micrograms/ml serum | |
|---|---|---|---|---|
| | | | S-TAG mean ($\pm$S.D.) | Net TAG mean ($\pm$S.D.) |
| Carcinoma of: | | | | |
| Lung | 83.3 | 83.3 | | |
| Breast[g] | 100.0 | 100.0 | | |
| Colon[g] | 100.0 | 100.0 | | |
| Rectum[g] | 83.3 | 83.3 | | |
| Prostate | 100.0 | 100.0 | | |
| Bladder | 100.0 | 100.0 | | |
| Ovary | 50.0 | 75.0 | | |
| Kidney | 100.0 | 100.0 | | |
| Thyroid | 100.0 | 100.0 | | |
| Undifferentiated | 100.0 | 100.0 | | |
| Lymphoma | 90.0 | 90.0 | | |
| Hodgkins's disease | 83.3 | 100.0 | | |
| Multiple Myeloma | 100.0 | 100.0 | | |
| Acute Myelogenous leukaemia | 100.0 | 100.0 | | |
| Chronic Myelogenous leukaemia[b] | 100.0 | 100.0 | | |
| Chronic Lymphocytic leukaemia[c] | 66.6 | 100.0 | | |
| Osteogenic Sarcoma | 0 | 0 | | |
| Melanotic Sarcoma | 75.0 | 75.0 | | |
| Total non-brain malignancies | 88.3 | 92.2 | 482.5 ($\pm$202.8) | 168.1 ($\pm$145.9) |
| Brain cancer[d, g] | 94.1 | 95.3 | 456.3 ($\pm$146.9) | 183.4 ($\pm$116.3) |
| Total malignancies | 91.4 | 93.8 | | |
| Non-malignant medical and surgical disorders | 5.9 | — | 259.8 ($\pm$115.8) | 59.5 ($\pm$66.0) |
| Normal | 3.9 | — | 280.3 ($\pm$79.5) | 60.2 ($\pm$46.9) |

Total number 290; % correct=91.4; 94.5% including border line values.

Legend: S-TAG is the amount in micrograms per ml of serum of antibody bound in 2 hours' incubation; F-TAG is that bound in 10 minutes' incubation; Net TAG=(S-TAG)—(F-TAG). Net TAG normal values: 0 to 134 micrograms per ml serum. Elevated values: Net TAG 135 micrograms per ml serum or above, or S-TAG greater than 400 micrograms per ml even if a high F-TAG value results in Net TAG below 135 micrograms per ml. Borderline values: 100 to 134 micrograms per ml. The values for S-TAG were significantly different for Gps. I and II as compared with Gps III and IV at a level of $p$ below 0.000001 and for Net TAG at a level of $p$ below 0.000001). All of these determinations were performed blind. Only those cases in which the clinical source of the serum was known, the clinical-pathological diagnosis established and the serum received in good condition frozen in dry ice not longer than 48 hours from the time it was drawn are included in the study; 270 specimens had to be excluded on these bases.

[a]Seven of these determinations were performed with Astrocytin-based TARGET reagent: 1 cancer of colon, 1 cancer of thyroid, 3 Hodgkins's Disease, 1 osteogenic sarcoma, 1 melanotic sarcoma.

[b]Polycythemia vera.

[c]Terminal cases: 1 leukaemia excluded, some additional may also be excluded.

[d]Eleven of these determinations performed with Astrocytin-based TARGET reagent.

[e]Six cases originally borderline, then normal when repeated; one sickle cell anaemia in crisis with renal insufficiency, 1 case of obesity, 1 of gout.

[f]Eleven cases originally borderline, then normal when repeated.

[g]Two cases of breast cancer, 1 of rectum and 1 of brain demonstrated normal values of serum TAG and 2 of colon cancer demonstrated borderline values, on serial determination during course of cancer treatment.

24

Example 14
Detection of non-brain malignant cells with fluorescent signal from TAG

The uses of TAG products coupled with a signal emitter such as a dye or a radioactive label to detect cancer cells has been described at various occurrences above. In this Example, the detection of non-brain malignant cells is described.

As described in Example 8 utilizing human serum, in the determination of the TAG product, after the anti-malignin antibody was bound to the immobilized antigen and non-bound serum proteins had been washed away, the antibody was cloven from the binding with 0.25 M acetic acid at 37°C for 2 hours and the TARGET reagent was separated therefrom by centrifuging. The TAG antibody solution was quantified by means of its absorption at 280 m$\mu$. The TAG solutions were stored at −20°C, then thawed and combined, brought to pH 7 by titration with 6N NaOH, dialysed against phosphate-buffered saline of pH 7, filtered and concentrated on Millipore Pellicon 1000 (Trade Mark) membranes, centrifuged to clear insoluble protein and the immune globulin complexes, concentrated and freed of immunologically non-active compounds by Cellex D (trade mark) and Blue Sepharose CL6B (trade mark) (Pharmacia) chromatography. This human anti-malignin antibody reacts with anti-human gamma globulin in Ouchterlony double diffusion. When the TAG product is used with fluorescein conjugated to anti-human gamma globulin in standard double layer Coons immunofluorescence, it stains malignant glia, breast carcinoma, overian carcinoma, adenocarcinoma of the colon, and other types of cancer cells in postoperative and biopsy tissue sections, as well as in human sputum, bronchial washings, pleural effusion fluid, gastric aspirate and bladder urine. The concentration of protein in the TAG product which yields clear fluorescence when the controls are negative is from 1 to 10 $\mu$g per section.

The production of a "purified" TAG was undertaken by reacting the sera from patients with a variety of cancers with Bromoacetylcellulose-MALIGNIN by methods described above (Example 8). The antibody bound in this reaction was cleaved with 0.25 M acetic acid, quantified by measurement at O.D. 280 using a conversion factor of 1.46 for gamma globulin, frozen and stored at −20°C. This antibody was found to contain immunoglobulin as determined by anti-human gamma-globulin antiserum specific for gamma chains (BioRad Laboratories, Inc.) and with anti-FAB and anti-Fc fragments (Miles Laboratories). It also reacts with rabbit anti-human albumin (BioRad Laboratories).

It was found that, whereas 10 to 50 micrograms of protein TAG are required to produce specific immunofluorescent staining of cells which contain Malignin, only 1 to 10 micrograms of purified protein TAG are required for this specific staining in all sections, and, in a few, less than one microgram has been found to suffice.

It was found that the most active preparation of purified TAG is that which is eluted with the highest ionic strength elution, i.e., from 0.15 M to 1.5 M. Any method of production which uses this fact is useful; three preferred methods are given below.

Method I—Fractionation of the TAG product by chromatography with DEAE cellulose (Cellex D, trade mark, BioRad Laboratories) was first employed with step-wise elution with increasing ionic strength and decreasing pH, and the same sequence of eluants as that given in Example 1 for the production of Crude Astrocytin-Precursor-Containing Fraction. Good separation was obtained of the bulk of the protein into three fractions, Peak I being obtained with solution 1 (see Example 1) and Peak II being obtained with solution 6 and solution 7. Ouchterlony double diffusion showed the TAG in Peak I still to contain appreciable protein with albumin mobility, and, while Peak II contained most of the albumin, appreciable IgG could be detected. Rechromatography of Peak I gave a progressively pure IgG until, after the seventh chromatography, essentially no albumin (less than 3%) could be detected by Ouchterlony gel diffusion in which 5 to 10 micrograms of human albumin were detectable with rabbit anti-human albumin. The IgG so obtained was prone to denaturing and to loss of immunological reactivity after a few days standing at 0—5°C.

Method II—A second fractionation of the TAG product was effected by chromatography on Sepharose CL-6B (trade mark) (Pharmacia Inc.), starting with a low molarity buffer (0.0005 M phosphate) and proceeding in two steps of 0.15 M and 1.5 M to elute the balance of the protein. As with the Cellex D (trade mark), one passage was found to be inadequate to separate, and recycling slowly improved the product. Once again, the most active fraction vis-a-vis Anti-Malignin antibody was the 1,5 M fraction.

Method III—Chromatography with Sepharose CL-6B (trade mark) next to the glass fitted disc and Cellex D (trade mark) layered above the Sepharose proved to be the most satisfactory method.

The graphical representation in Fig. 1A of the drawings shows the fractions obtained by chromatography of the TAG product utilizing Method III. After the first eluate of 200 mls, 50 ml or smaller sub-fractions were collected. The protein content of each eluate was determined by the optical density at 280 m$\mu$ with a uniform factor of 1.46 based on gamma globulin used to convert to micrograms for calculating recoveries. The absolute amount of protein requires correction in those fractions in which there is appreciable albumin. The points at which the stepwise solvent changes were made are indicated by arrows. The sub-fractions are designated by Roman Numerals I to VIII, inclusive.

The solvents corresponding to letters A—F at the arrows were as follows:

A—0.01 M TRIS (pH 7.2)
B—0.05 M TRIS with 0.1M NaCl (pH 7.2)
C—PBS, 0.11 M NaCl (pH 7.2)
D—PBS, 0.165 M NaCl (pH 7.2)
E—PBS, 0.33 M NaCl (pH 7.2)
F—0.05 M TRIS, 1.5 M NaCl (pH 7.2)

The following Table VII shows the recoveries from each fraction, a semi-quantitative determination of the gamma-globulin and albumin in each fraction, as well as the activity of each fraction in the immunofluorescent staining of cancer cells (the plus sign indicates reaction, zero no reaction and plus/minus reaction in some cases).

TABLE VII

| Fraction | I | II | III | IV | V | VI | VII | VIII |
|---|---|---|---|---|---|---|---|---|
| Recovered $\mu$g | 2,877 | 1,140 | 2,351 | 2,942 | 1,808 | 2,230 | 2,125 | 7,477 |
| % | 12.5 | 5.0 | 10.2 | 12.8 | 7.9 | 9.7 | 9.3 | 32.6 |
| Immunodiffusion against: Anti-human IgG. specific for gamma chains | +++ | ++ | ++ | + | 0 | + | ++ | +++ |
| Anti-human albumin | + | + | + | ++ | +++ | +++ | +++ | +++ |
| Anti-Fab | + | ++ | 0 | ++ | + | 0 | ++ | ++ |
| Anti-Fc | ++ | ++ | + | ++ | + | 0 | + | 0 |
| Immunofluorescence | ± | ± | ± | ± | ± | ± | ± | +++ |

Example 15
Detection of cancer cells with radioisotope signal from TAG
In this Example, the feasibility of attaching a radioactive label to a TAG product is demonstrated. Second, the injection into animals of this radio-labelled TAG has been accomplished and shown to be safe and effective. Third, the radio-labelled TAG localized preferentially in the cancer tissue when compared with normal tissue, thus indicating that the specificity previously demonstrated *in vitro* of the preference for cancer cells which is conveyed by the use of specific anti-Malignin TAG products is confirmed *in vivo*.

The labelling of TAG with 99m Technetium ($^{99m}$Tc)
Procedure for labelling
1. Two preparations of TAG were used, here designated TAG-1 and TAG-2, respectively TAG-1 and TAG-2 (concentration of each: 0.4 mg/0.5 ml) were added to separate sterile, evacuated vials.
2. To each vial was added 0.1 ml of a stannous chloride solution (10 mg $SnCl_2 . 2H_2O$ in 100 ml of 0.01 N HCl). The vials were mixed for 3—4 minutes.
3. 0.1 ml (6mCi) of $^{99m}$Tc-pertechnetate (sodium salt) was added and mixed for 2—3 minutes.

Procedure for determining labelling efficiency
Sample of the $^{99m}$Tc-TAG-1 and $^{99m}$Tc-TAG-2 were tested for labelling efficiency by descending paper chromatography using Watman No. 1 paper with 85% methanol as the solvent. A similar study was done with sodium pertechnetate-$^{99m}$Tc which acted as a control.
After 2 hours, the papers were removed from the chromatography tank and divided into two sections: (1) 1 cm about the origin; (2) the remaining paper up to the solvent front. Each section was then counted in a gamma well scintillation counter and its content of radioactivity was determined (CPM).
Approximately 50 lamda were plated on each paper strip.

26

Procedures for antigen-antibody reactions

A portion of the labelled solution was also plated on an Ouchterlony gel plate to determine its ability to react with Malignin in the antigen-antibody reaction. After a 3 hour period, the resulting sharp reactive lines were removed from the gel and their content of radioactivity was measured. An equal portion of the gel not involved in the reaction was also removed and its content of radioactivity was also measured as background.

Results
Labelling efficiency

TABLE 1
Labelling efficiency of $^{99m}$Tc-TAG-1 and $^{99m}$Tc-TAG$^{-2}$

| Compound | Site on paper | CPM |
|---|---|---|
| NaTcO$_4$-99mTc | origin | $4.94 \times 10^5$ |
| NaTcO$_4$-99mTc | solvent front | $6.25 \times 10^6$ |
| TAG-1 | origin | $4.35 \times 10^6$ |
| TAG-1 | solvent front | $6.76 \times 10^4$ |
| TAG-2 | origin | $1.96 \times 10^6$ |
| TAG-2 | solvent front | $3.98 \times 10^4$ |

TABLE 1 (Continued)

| Compound | % | Chemical species |
|---|---|---|
| NaTcO$_4$-99mTc | 7.33% | Reduced TcO$_4$- |
| NaTcO$_4$-99mTc | 92.67% | TcO$^{4-}$ |
| TAG-1 | 98.47% | TAG-99mTc |
| TAG-1 | 1.53% | TcO$_4$- |
| TAG-2 | 98.01% | TAG-99mTc |
| TAG-2 | 1.99% | TcO$_4$- |

TABLE 2
Antigen-antibody resection

| Gel area | Counts per min | % |
|---|---|---|
| TAG-2 line | $1.99 \times 10^6$ | 92.04% |
| Background gel | $1.72 \times 10^5$ | 7.96% |

Conclusions

The following conclusions were reached relative to the quality control tests employed:

1. $^{99m}$Tc-pertechnetate was reduced by stannous chloride to a more reactive oxidation state (+4+5).
2. The reduced pertechnetate labelled both the TAG-1 and TAG-2 preparations.
3. The 99mTc-TAG-2 was tested for its ability to retain its activity and was found to retain its ability to react immunologically.

The use of radio-labelled TAG in vivo to detect cancer cells

Wistar rats were injected intracerebrally with Cisgliome tumour cells which had had previous passages in rats and in tissue culture. The rats were observed for the first signs of growing tumour, such as weakness, tremors or unsteadiness. These symptoms first appeared seven to 10 days from injection, and, with fast growing tumours resulted in death within three to four days in many animals, and one week in all. As soon as the symptoms appeared, the animals were injected with labelled TAG intravenously in the tail vein, and then, the animal was anaesthetized at varying times, the brain was removed, the tumour dissected free of normal brain, and the radioactivity in each dissected specimen was compared.

Preliminary $^{99m}$Tc-TAG experiment

| Animal | Sacrifice (hr. post injection) | Tumour wt., mg | Counts/gm/min | |
|---|---|---|---|---|
| | | | Tumour | Normal brain |
| A | 1.25 | 1.9 | 149,100 | 13,400 |
| B | 5.30 | 6.0 | 16,200 | 6,600 |
| C | 7.21 | 23.0 | 53,000 | 5,800 |
| D | 24.10 | 29.0 | 66,700 | 7,500 |

Tumour and normal brain specimens were counted overnight in the gamma-well counter. All samples and standards were decay corrected for convenience to the mid-count of the first sample in the sequence.

Conclusion

The preferential localization of radioactivity in tumour as compared with normal tissue is demonstrated above.

**Claims for the Contracting States: BE, CH, DE, FR, GB, IT, LU, NL, SE**

1. A process for the production of the polypeptide products Recognin L and Recognin M, respectively, characterised in that:

(i) artificial lymphoma tumour cells in the case of Recognin L and mammary tumour cells in the case of Recognin M are grown in culture with a neutral buffer and extracted by repeated disruption of the tissue to solubilize protein fractions;

(ii) the fraction having a pK range of from 1 to 4 is separated from the resulting extract of solubilized proteins; and

(iii) the desired Recognin L or Recognin M product is respectively isolated from said fraction (ii), said Recognin L and Recognin M products being each characterised by:

(a) forming a precipitate with its specific antibody in quantitative precipitin tests and Ouchterlony gel diffusion tests;

(b) being soluble in water and aqueous solutions having an acid or neutral pH, and insoluble at an alkaline pH;

(c) having a spectrophotometric adsorption wave-length of 280 m$\mu$; and

(d) having a molecular weight of 8000 (as determined by thin-layer gel chromatography), said Recognin L product being further characterised by an aminoacid composition substantially as follows:

| | Number of aminoacid residues |
|---|---|
| Aspartic acid | 8 |
| Threonine | 5 |
| Serine | 5 |
| Glutamic acid | 10 |
| Proline | 5 |
| Glycine | 13 |
| Alanine | 10 |
| Valine | 6 |
| 1/2 Cysteine | 1 |
| Methionine | 1 |
| Isoleucine | 4 |
| Leucine | 7 |
| Tyrosine | 1 |
| Phenylalanine | 3 |
| Lysine | 6 |
| Histidine | 2 |
| Arginine | 5 |
| Approximate Total | 92 |

and by the fact that the aminoacids cysteic acid, hydroxyproline, norleucine, ammonia, isodesmosine, desmosine, hydroxylysine, lysinonorleucine and gamma-aminobutyric acid are absent in detectable amounts, and said Recognin M product being further characterised by an aminoacid composition substantially as follows:

# 0 015 755

| | Number of aminoacid residues |
|---|---|
| Aspartic acid | 9 |
| Threonine | 5 |
| Serine | 5 |
| Glutamic acid | 11 |
| Proline | 4 |
| Glycine | 9 |
| Alanine | 9 |
| Valine | 6 |
| 1/2 Cysteine | 1 |
| Methionine | 1 |
| Isoleucine | 4 |
| Leucine | 8 |
| Tyrosine | 2 |
| Phenylalanine | 3 |
| Lysine | 6 |
| Histidine | 2 |
| Arginine | 5 |
| Approximate total | 90 |

and by the fact that the aminoacids cysteic acid, hydroxyproline, norleucine, ammonia, isodesmosine, desmosine, hydroxylysine, lysinonorleucine and gamma-aminobutyric acid are absent in detectable amounts.

2. A process according to claim 1, further characterised in that said Recognin L or Recognin M product is capable of complexing with bromoacetylcellulose to form bromoacetylcellulose-Recognin L or bromoacetylcellulose-Recognin M, respectively, and in that said Recognin L and Recognin M produce the specific antibodies Anti-Recognin L and Anti-Recognin M, respectively, upon injection into mammals, said Anti-Recognin L and Anti-Recognin M being respectively toxic to tumour cells *in vitro* and producing fluorescence of glioma cells when coupled with fluorescein.

3. A process according to claim 1 or claim 2, further characterised in that said separation step (ii) is effected by adding said extract of solubilized proteins to a chromatographic column and eluting with increasingly acidic solvents.

4. A process according to claim 3, further characterised in that said isolation step (iii) of claim 1 is carried out by filtering the eluate of step (ii) to obtain a fraction containing Recognin L or Recognin M, respectively, and separating Recognin L or Recognin M therefrom by thin layer gel chromatography.

5. A polypeptide product called Recognin L, characterised by forming a single line precipitate with its specific antibody in quantitative precipitin tests and Ouchterlony gel diffusion tests; being soluble in water and aqueous solutions having an acid or neutral pH, and insoluble at an alkaline pH; having a spectrophotometric absorption peak wave-length of 280 m$\mu$; having a molecular weight of 8,000 (as determined by thin layer gel chromatography), and having an aminoacid composition substantially as follows:

| | Number of amino acid residues |
|---|---|
| Aspartic acid | 8 |
| Threonine | 5 |
| Serine | 5 |
| Glutamic acid | 10 |
| Proline | 5 |
| Glycine | 13 |
| Alanine | 10 |
| Valine | 6 |
| 1/2 Cysteine | 1 |
| Methionine | 1 |
| Isoleucine | 4 |
| Leucine | 7 |
| Tyrosine | 1 |
| Phenylalanine | 3 |
| Lysine | 6 |
| Histidine | 2 |
| Arginine | 5 |
| Approximate total | 92 |

and by the fact that the amino acids cysteic acid, hydroxyproline, norleucine, ammonia, isodesmosine, desmosine, hydroxylysine, lysinonorleucine and gamma-aminobutyric acid are absent in detectable amounts.

6. A polypeptide product called Recognin M characterised by forming a single line precipitate with its specific antibody in quantitative precipitin tests and Ouchterlony gel diffusion tests; being soluble in water and aqueous solutions having an acid or neutral pH, and insoluble at an alkaline pH; having a spectrophotometric absorption peak wave-length of 280 m$\mu$; having a molecular weight of 8,000 (as determined by thin layer gel chromatography), and having an aminoacid composition substantially as follows:

| | Number of aminoacid residues |
|---|---|
| Aspartic acid | 9 |
| Threonine | 5 |
| Serine | 5 |
| Glutamic acid | 11 |
| Proline | 4 |
| Glycine | 9 |
| Alanine | 9 |
| Valine | 6 |
| 1/2 Cysteine | 1 |
| Methionine | 1 |
| Isoleucine | 4 |
| Leucine | 8 |
| Tyrosine | 2 |
| Phenylalanine | 3 |
| Lysine | 6 |
| Histidine | 2 |
| Arginine | 5 |
| Approximate total | 90 |

and by the fact that the aminoacids cysteic acid, hydroxyproline, norleucine, ammonia, isodesmosine, desmosine, hydroxylysine, lysinonorleucine and gamma-aminobutyric acid are absent in detectable amounts.

7. A process for producing the product Anti-Recognin L or Anti-Recognin M, characterised by injection into a mammal of said Recognin L or Recognin M as claimed in claim 5 or 6, respectively, or produced by a process as claimed in any of claims 1 to 4, thereby producing the specific antibody Anti-Recognin L or Anti-Recognin M, respectively, said Anti-Recognin L or Anti-Recognin M being toxic to tumour cells *in vitro* and producing fluoresence of glioma cells when coupled with fluorescein, and by the recovery of said Anti-Recognin L or Anti-Recognin M, respectively.

8. The products Anti-Recognin L and Anti-Recognin M consisting of the antibody product produced by injection into a mammal of Recognin L or Recognin M as claimed in claim 5 or 6, respectively, or produced as claimed in any of claims 1 to 4, said specific antibodies Anti-Recognin L and Anti-Recognin M being each toxic to tumour cells *in vitro* and producing fluorescence of glioma cells when coupled with fluorescein.

9. A process for producing the complex Bromoacetylcellulose-Recognin L or Bromoacetyl-cellulose-Recognin M characterised in that Recognin L or Recognin M as claimed in claim 5 or 6, respectively, or produced as claimed in any of claims 1 to 4, is mixed with Bromoacetylcellulose in a manner adapted to form said complex Bromoacetylcellulose-Recognin L or Bromoacetylcellulose-Recognin M, respectively.

10. The complexes Bromoacetylcellulose-Recognin L and Bromoacetylcellulose-Recognin M characterised as complexes of Bromoacetylcellulose with Recognin L or Recognin M as claimed in claim 5 or 6, respectively, or produced by a process as claimed in any of claims 1 to 4.

11. A process for producing Bromoacetylcellulose-Recognin L-Anti-Recognin L or Bromoacetyl-cellulose-Recognin M-Anti-Recognin M, characterised by the reaction of Anti-Recognin L or Anti-Recognin M, respectively, as claimed in claim 8 or produced by a process as claimed in claim 7, with Bromoacetylcellulose-Recognin L or Bromoacetylcellulose-Recognin M, respectively, as claimed in Claim 10, or produced by a process as claimed in claim 9.

12. The complexes Bromoacetylcellulose-Recognin L-Anti-Recognin L and Bromoacetylcellulose-Recognin M-Anti-Recognin M, characterised as complexes of Anti-Recognin L or Anti-Recognin M, respectively, as claimed in claim 8, or produced by a process as claimed in claim 7, with Bromoacetyl-cellulose-Recognin L or Bromoacetylcellulose-Recognin M, respectively, as claimed in claim 10, or produced by a process as claimed in claim 9.

13. A process for producing Anti-Recognin L or Anti-Recognin M, respectively, as claimed in Claim 8, characterised by decomplexing Bromoacetylcellulose-Recognin L-Anti-Recognin L or Bromoacetylcellulose-Recognin M-Anti-Recognin M, respectively, as claimed in claim 12, or produced by a process as claimed in claim 11, by hydrolytic treatment with acid or with a proteinase enzyme.

14. A process for producing a Slow-Target Attaching Globulin (S-TAG), characterised by the reaction of blood serum or other body fluid with Bromoacetylcellulose-Recognin L or Bromoacetyl-cellulose-Recognin M, respectively, as claimed in claim 10, or produced by a process as claimed in claim 9, for at least two hours at a low temperature to prevent degradative reactions; and treating the resulting material with dilute acid to cleave said Slow-Target Attaching Globulin (S-TAG) therefrom.

15. A product S-TAG prepared in accordance with the process of claim 14, said product being characterised by being soluble in aqueous buffered solutions, forming a single line precipitate with Recognin L or Recognin M in Ouchterlony gel diffusion tests, being non-dialysable in cellophane membranes, being retained by millipore filters which retain molecules over 25,000 molecular weight, having molecular weights in different states of aggregation as determined by thin layer gel chromatography of approximately 50,000, and multiples thereof into the macroglobulin range, and having a spectrophotometric adsorption peak wave-length of 280 m$\mu$.

16. A process for producing Fast-Target Attaching Globulin (F-TAG), characterised by reacting blood serum or other body fluid with Bromoacetylcellulose-Recognin L or Bromoacetylcellulose-Recognin M, respectively, as claimed in claim 10, or produced by a process as claimed in claim 9, for approximately 10 minutes at a low temperature to prevent degradative reactions; and treating the resulting material with dilute acid to cleave said Fast-Target-Attaching Globulin (F-TAG) therefrom.

17. A product F-TAG prepared in accordance with the process of claim 16, said product being characterised by being soluble in aqueous buffered solutions, forming a single line precipitate with Recognin L or Recognin M in Ouchterlony gel diffusion tests, being non-dialysable in cellophane membranes, being retained by millipore filters which retain molecules over 25,000 molecular weight having molecular weights in different states of aggregation as determined by thin layer gel chromatography of approximately 50,000, and multiples thereof into the macroglobulin range, and having a spectrophotometric adsorption peak wave-length of 280 m$\mu$.

18. A method of detecting cancer tumours in living animals, characterised by determining *in vitro* the concentrations of S-TAG and F-TAG, respectively, as claimed in claims 15 and 17, respectively, produced by a known volume of the mammal's blood serum or other body fluid.

19. A method of diagnosing *in vitro* the presence of tumour cells in histology section, characterised by applying a product consisting of fluorescein conjugated with a TAG product as claimed in claim 15 or 17, respectively, or with Anti-Recognin L or Anti-Recognin M, respectively, as claimed in claim 10, or produced by a process as claimed in claim 9, to said histology section, and determining whether or not fluorescence occurs, said fluorescence occurring only in said tumour cells.

20. The polypeptide products Recognin L and Recognin M, as claimed in Claim 5 or 6, respectively, or as produced in any of claims 1 to 4, for use in cancer diagnosis.

21. The antibody products Anti-Recognin L and Anti-Recognin M, respectively, as claimed in claim 8 or produced by a process as claimed in claim 7, for use in cancer diagnosis.

22. The complexes Bromoacetylcellulose-Recognin L and Bromoacetylcellulose-Recognin M as claimed in claim 10 or produced by a process as claimed in claim 9, for use in cancer diagnosis.

23. Bromoacetylcellulose-Recognin L-Anti-Recognin L or Bromoacetylcellulose-Recognin M-Anti-Recognin M, respectively, as claimed in claim 12, or produced by a process as claimed in claim 11, for use in cancer diagnosis.

24. S-TAG and F-TAG products as claimed in claim 15 or 17, respectively, or produced by a process as claimed in claim 14 or 16, respectively, for use in cancer diagnosis.

25. The polypeptide products Recognin L and Recognin M, as claimed in claim 5 or 6, respectively, or as produced in any of claims 1 to 4, for use in cancer therapy.

26. The antibody products Anti-Recognin L and Anti-Recognin M, respectively, as claimed in claim 8, or produced by a process as claimed in claim 7, for use in cancer therapy.

27. The complexes Bromoacetylcellulose-Recognin L and Bromoacetylcellulose-Recognin M as claimed in claim 10 or produced by a process as claimed in claim 9, for use in cancer therapy.

28. Bromoacetylcellulose-Recognin L-Anti-Recognin L or Bromoacetylcellulose-Recognin M-Anti-Recognin M, respectively, as claimed in claim 12, or produced by a process as claimed in claim 11, for use in cancer therapy.

29. S-TAG and F-TAG products as claimed in claim 15 or 17, respectively, or produced by a process as claimed in claim 14 or 16, respectively, for use in cancer therapy.

**Claims for the Contracting State: AT**

1. A process for the production of the polypeptide products Recognin L and Recognin M, respectively, characterised in that:

(i) artificial lymphoma tumour cells in the case of Recognin L and mammary tumour cells in the case of Recognin M are grown in culture with a neutral buffer and extracted by repeated disruption of the tissue to solubilize protein fractions;
(ii) the fraction having a pK range of from 1 to 4 is separated from the resulting extract of solubilized proteins; and
(iii) the desired Recognin L or Recognin M product is respectively isolated from said fraction (ii), said Recognin L and Recognin M products being each characterised by:

(a) forming a precipitate with its specific antibody in quantitative precipitin tests and Ouchterlony gel diffusion tests;
(b) being soluble in water and aqueous solutions having an acid or neutral pH, and insoluble at an alkaline pH;
(c) having a spectrophotometric adsorption wave-length of 280 m$\mu$; and
(d) having a molecular weight of 8,000 (as determined by thin-layer gel chromatography), said Recognin L product being further characterised by an amino acid composition substantially as follows:

|  | Number of aminoacid residues |
|---|---|
| Aspartic acid | 8 |
| Threonine | 5 |
| Serine | 5 |
| Glutamic acid | 10 |
| Proline | 5 |
| Glycine | 13 |
| Alanine | 10 |
| Valine | 6 |
| 1/2 Cysteine | 1 |
| Methionine | 1 |
| Isoleucine | 4 |
| Leucine | 7 |
| Tyrosine | 1 |
| Phenylalanine | 3 |
| Lysine | 6 |
| Histidine | 2 |
| Arginine | 5 |
| Approximate total | 92 |

32

and by the fact that the aminoacids cysteic acid, hydroxyproline, norleucine, ammonia, isodesmosine, desmosine, hydroxylysine, lysinonorleucine and gamma-aminobutyric acid are absent in detectable amounts, and said Recognin M product being further characterised by an aminoacid composition substantially as follows:

|  | Number of amino acid residues |
| --- | --- |
| Aspartic acid | 9 |
| Threonine | 5 |
| Serine | 5 |
| Glutamic acid | 11 |
| Proline | 4 |
| Glycine | 9 |
| Alanine | 9 |
| Valine | 6 |
| 1/2 Cysteine | 1 |
| Methionine | 1 |
| Isoleucine | 4 |
| Leucine | 8 |
| Tyrosine | 2 |
| Phenylalanine | 3 |
| Lysine | 6 |
| Histidine | 2 |
| Arginine | 5 |
| Approximate total | 90 |

and by the fact that the aminoacids cysteic acid, hydroxyproline, norleucine, ammonia, isodesmosine, desmosine, hydroxylysine, lysinonorleucine and gamma-aminobutyric acid are absent in detectable amounts.

2. A process according to claim 1, further characterised in that said Recognin L or Recognin M product is capable of complexing with bromoacetylcellulose to form bromoacetylcellulose-Recognin L or bromoacetylcellulose-Recognin M, respectively, and in that said Recognin L and Recognin M produce the specific antibodies Anti-Recognin L and Anti-Recognin M, respectively, upon injection into mammals, said anti-Recognin L and Anti-Recognin M being respectively toxic to tumour cells *in vitro* and producing fluorescence of glioma cells when coupled with fluorescein.

3. A process according to claim 1 or claim 2, further characterised in that said separation step (II) is effected by adding said extract of solubilized proteins to a chromatographic column and eluting with increasingly acidic solvents.

4. A process according to claim 3, further characterised in that said isolation step (iii) of claim 1 is carried out by filtering the eluate of step (ii) to obtain a fraction containing Recognin L or Recognin M, respectively, and separating Recognin L or Recognin M therefrom by thin layer gel chromatography.

5. A polypeptide product called Recognin L, characterised by forming a single line precipitate with its specific antibody in quantitative precipitin tests and Ouchterlony gel diffusion tests; being soluble in water and aqueous solutions having an acid or neutral pH, and insoluble at an alkaline pH; having a spectrophotometric absorption peak wave-length of 280 m$\mu$; having a molecular weight of 8,000 (as determined by thin layer gel chromatography), and having an aminoacid composition substantially as follows:

## 0 015 755

| | Number of aminoacid residues |
|---|---|
| Aspartic acid | 8 |
| Threonine | 5 |
| Serine | 5 |
| Glutamic acid | 10 |
| Proline | 5 |
| Glycine | 13 |
| Alanine | 10 |
| Valine | 6 |
| 1/2 Cysteine | 1 |
| Methionine | 1 |
| Isoleucine | 4 |
| Leucine | 7 |
| Tyrosine | 1 |
| Phenylalanine | 3 |
| Lysine | 6 |
| Histidine | 2 |
| Arginine | 5 |
| Approximate total | 92 |

and by the fact that the amino acids cysteic acid, hydroxyproline, norleucine, ammonia, isodesmosine, desmosine, hydroxylysine, lysinonorleucine and gamma-aminobutyric acid are absent in detectable amounts, for use in cancer diagnosis.

6. A polypeptide product called Recognin M characterised by forming a single line precipitate with its specific antibody in quantitative precipitin tests and Ouchterlony gel diffusion tests; being soluble in water and aqueous solutions having an acid or neutral pH, and insoluble at an alkaline pH; having a spectrophotometric absorption peak wave-length of 280 m$\mu$; having a molecular weight of 8,000 (as determined by thin layer gel chromatography), and having an aminoacid composition substantially as follows:

| | Number of aminoacid residues |
|---|---|
| Aspartic acid | 9 |
| Threonine | 5 |
| Serine | 5 |
| Glutamic acid | 11 |
| Proline | 4 |
| Glycine | 9 |
| Alanine | 9 |
| Valine | 6 |
| 1/2 Cysteine | 1 |
| Methionine | 1 |
| Isoleucine | 4 |
| Leucine | 8 |
| Tyrosine | 2 |
| Phenylalanine | 3 |
| Lysine | 6 |
| Histidine | 2 |
| Arginine | 5 |
| Approximate total | 90 |

and by the fact that the aminoacids cysteic acid, hydroxyproline, norleucine, ammonia, isodesmosine, desmosine, hydroxylysine, lysinonorleucine and gamma-aminobutyric acid are absent in detectable amounts, for use in cancer diagnosis.

7. A process for producing the product Anti-Recognin L or Anti-Recognin M, characterised by injection into a mammal of said Recognin L or Recognin M as claimed in claim 5 or 6, respectively, or produced by a process as claimed in any of claims 1 to 4, thereby producing the specific antibody Anti-Recognin L or Anti-Recognin M, respectively, said Anti-Recognin L or Anti-Recognin M being toxic to tumour cells *in vitro* and producing fluoresence of glioma cells when coupled with fluorescein, and by the recovery of said Anti-Recognin L or Anti-Recognin M, respectively.

8. The products Anti-Recognin L and Anti-Recognin M consisting of the antibody product produced by injection into a mammal of Recognin L or Recognin M as claimed in claim 5 or 6, respectively, or produced as claimed in any of claims 1 to 4, said specific antibodies Anti-Recognin L and Anti-Recognin M being each toxic to tumour cells *in vitro* and producing fluorescence of glioma cells when coupled with fluorescein, for use in cancer diagnosis.

9. A process for producing the complex Bromoacetylcellulose-Recognin L or Bromoacetylcellulose-Recognin M characterised in that Recognin L or Recognin M as claimed in claim 5 or 6, respectively, or produced as claimed in any of claims 1 to 4, is mixed with Bromoacetylcellose in a manner adapted to form said complex Bromoacetylcellulose-Recognin L or Bromoacetylcellulose-Recognin M, respectively.

10. The complexes Bromoacetylcellulose-Recognin L and Bromoacetylcellulose-Recognin M characterised as complexes of Bromoacetylcellulose with Recognin L or Recognin M as claimed in claim 5 or 6, respectively, or produced by a process as claimed in any of claims 1 to 4, for use in cancer diagnosis.

11. A process for producing Bromoacetylcellulose-Recognin L-Anti-Recognin L or Bromoacetylcellulose-Recognin M-Anti-Recognin M, characterised by the reaction of Anti-Recognin L or Anti-Recognin M, respectively, as claimed in Claim 8 or produced by a process as claimed in claim 7, with Bromoacetylcellulose-Recognin L or Bromoacetylcellulose-Recognin M, respectively, as claimed in claim 10, or produced by a process as claimed in claim 9.

12. The complexes Bromoacetylcellulose-Recognin L-Anti-Recognin L and Bromoacetylcellulose-Recognin M-Anti-Recognin M, characterised as complexes of Anti-Recognin L or Anti-Recognin M, respectively, as claimed in claim 8, or produced by a process as claimed in claim 7, with Bromoacetylcellulose-Recognin L or Bromoacetylcellulose-Recognin M, respectively, as claimed in claim 10 or produced by a process as claimed in claim 9, for use in cancer diagnosis.

13. A process for producing Anti-Recognin L or Anti-Recognin M, respectively, as claimed in claim 8, characterised by decomplexing Bromoacetylcellulose-Recognin L-Anti-Recognin L or Bromoacetylcellulose-Recognin M-Anti-Recognin M, respectively, as claimed in claim 12, or produced by a process as claimed in claim 11, by hydrolytic treatment with acid or with a proteinase enzyme.

14. A process for producing a Slow-Target Attaching Globulin (S-TAG), characterised by the reaction of blood serum or other body fluid with Bromoacetylcellulose-Recognin L or Bromoacetylcellulose-Recognin M, respectively, as claimed in claim 10, or produced by a process as claimed in claim 9, for at least two hours at a low temperature to prevent degradative reactions; and treating the resulting material with dilute acid to cleave said Slow-Target Attaching Globulin (S-TAG) therefrom.

15. A product S-TAG prepared in accordance with the process of claim 14, said product being characterised by being soluble in aqueous buffered solutions, forming a single line precipitate with Recognin L or Recognin M in Ouchterlony gel diffusion tests, being non-dialysable in cellophane membranes, being retained by millipore filters which retain molecules over 25,000 molecular weight, having molecular weights in different states of aggregation as determined by thin layer gel chromatography of approximately 50,000, and multiples thereof into the macroglobulin range, and having a spectrophotometric adsorption peak wave-length of 280 m$\mu$, for use in cancer diagnosis.

16. A process for producing Fast-Target Attaching Globulin (F-TAG), characterised by reacting blood serum or other body fluid with Bromoacetylcellulose-Recognin L or Bromoacetylcellulose-Recognin M, respectively, as claimed in claim 10, or produced by a process as claimed in claim 9, for approximately 10 minutes at a low temperature to prevent degradative reactions; and treating the resulting material with dilute acid to cleave said Fast-Target-Attaching Globulin (F-TAG) therefrom.

17. A product F-TAG prepared in accordance with the process of claim 16, said product being characterised by being soluble in aqueous buffered solutions, forming a single line precipitate with Recognin L or Recognin M in Ouchterlony gel diffusion tests, being non-dialysable in cellophane membranes, being retained by millipore filters which retain molecules over 25,000 molecular weight having molecular weights in different states of aggregation as determined by thin layer gel chromatography of approximately 50,000, and multiples thereof into the macroglobulin range, and having a spectrophotometric adsorption peak wave-length of 280 m$\mu$, for use in cancer diagnosis.

18. A method of detecting cancer tumours in living animals, characterised by determining *in vitro* the concentrations of S-TAG and F-TAG, respectively, as claimed in claims 15 and 17, respectively, produced by a known volume of the mammal's blood serum or other body fluid.

19. A method of diagnosing *in vitro* the presence of tumour cells in histology section, characterised by applying a product consisting of fluorescein conjugated with a TAG product as claimed in claim 15 or 17, respectively, or with Anti-Recognin L or Anti-Recognin M, respectively, as claimed in claim 10, or produced by a process as claimed in claim 9, to said histology section, and determining whether or not fluorescence occurs, said fluorescence occurring only in said tumour cells.

20. A polypeptide product called Recognin L, characterised by forming a single line precipitate with its specific antibody in quantitative precipitin tests and Ouchterlony gel diffusion tests; being soluble in water and aqueous solutions having an acid or neutral pH, and insoluble at an alkaline pH;

**O 015 755**

having a spectrophotometric adsorption peak wave-length of 280 m$\mu$; having a molecular weight of 8,000 (as determined by thin layer gel chromatography), and having an aminoacid composition substantially as follows:

| | Number of aminoacid residues |
|---|---|
| Aspartic acid | 8 |
| Threonine | 5 |
| Serine | 5 |
| Glutamic acid | 10 |
| Proline | 5 |
| Glycine | 13 |
| Alanine | 10 |
| Valine | 6 |
| 1/2 Cysteine | 1 |
| Methionine | 1 |
| Isoleucine | 4 |
| Leucine | 7 |
| Tyrosine | 1 |
| Phenylalanine | 3 |
| Lysine | 6 |
| Histidine | 2 |
| Arginine | 5 |
| Approximate total | 92 |

and by the fact that the amino acids cysteic acid, hydroxyproline, norleucine, ammonia, isodesmosine, desmosine, hydroxylysine, lysinonorleucine and gamma-aminobutyric acid are absent in detectable amounts for use in cancer therapy.

21. A polypeptide product called Recognin M characterised by forming a single line precipitate with its specific antibody in quantitative precipitin tests and Ouchterlony gel diffusion tests; being soluble in water and aqueous solution having an acid or neutral pH, and insoluble at an alkaline pH; having a spectrophotometric adsorption peak wave-length of 280 m$\mu$; having a molecular weight of 8,000 (as determined by thin layer gel chromatography), and having an aminoacid composition substantially as follows:

| | Number of aminoacid residues |
|---|---|
| Aspartic acid | 9 |
| Threonine | 5 |
| Serine | 5 |
| Glutamic acid | 11 |
| Proline | 4 |
| Glycine | 9 |
| Alanine | 9 |
| Valine | 6 |
| 1/2 Cysteine | 1 |
| Methionine | 1 |
| Isoleucine | 4 |
| Leucine | 8 |
| Tyrosine | 2 |
| Phenylalanine | 3 |
| Lysine | 6 |
| Histidine | 2 |
| Arginine | 5 |
| Approximate total | 90 |

and by the fact that the aminoacids cysteic acid, hydroxyproline, norleucine, ammonia, isodesmosine, desmosine, hydroxylysine, lysinonorleucine and gamma-aminobutyric acid are absent in detectable amounts, for use in cancer therapy.

22. The products Anti-Recognin L and Anti-Recognin M consisting of the antibody product

36

**0 015 755**

produced by injection into a mammal of Recognin L or Recognin M as claimed in claim 5 or 6, respectively, or produced as claimed in any of claims 1 to 4, said specific antibodies Anti-Recognin L and Anti-Recognin M being each toxic to tumour cells *in vitro* and producing fluorescence of glioma cells when coupled with fluorescein, for use in cancer therapy.

23. The complexes Bromoacetylcellulose-Recognin L and Bromoacetylcellulose-Recognin M characterised as complexes of Bromoacetylcellulose with Recognin L or Recognin M as claimed in claim 5 or 6, respectively, or produced by a process as claimed in any of claims 1 to 4, for use in cancer therapy.

24. The complexes Bromoacetylcellulose-Recognin L-Anti-Recognin L and Bromoacetylcellulose-Recognin M-Anti-Recognin M, characterised as complexes of Anti-Recognin L or Anti-Recognin M, respectively, as claimed in claim 8, or produced by a process as claimed in claim 7, with Bromoacetyl-cellulose-Recognin L or Bromoacetylcellulose-Recognin M, respectively, as claimed in claim 10, or produced by a process as claimed in claim 9 for use in cancer therapy.

25. A product S-TAG prepared in accordance with the process of claim 14, said product being characterised by being soluble in aqueous buffered solutions, forming a single line precipitate with Recognin L or Recognin M in Ouchterlony gel diffusion tests, being non-dialysable in cellophane membranes, being retained by millipore filters which retain molecules over 25,000 molecular weight, having molecular weights in different states of aggregation as determined by thin layer gel chromatography of approximately 50,000, and multiples thereof into the macroglobulin range, and having a spectro-photometric adsorption peak-wave length of 280 m$\mu$, for use in cancer therapy.

26. A product F-TAG prepared in accordance with the process of claim 16, said product being characterised by being soluble in aqueous buffered solutions, forming a single line precipitate with Recognin L or Recognin M in Ouchterlony gel diffusion tests, being non-dialysable in cellophane membranes, being retained by millipore filters which retain molecules over 25,000 molecular weight having molecular weights in different states of aggregation as determined by thin layer gel chromatography of approximately 50,000, and multiples thereof into the macroglobulin range, and having a spectrophotometric adsorption peak wave-length of 280 m$\mu$, for use in cancer therapy.

**Revendications pour les Etats Contractants BE, CH, DE, FR, GB, IT, LU, NL, SE**

1. Procédé pour produire des produits à base de polypeptides la Recognine L et la Recognine M, respectivement, caractérisé en ce que:

(i) on cultive en milieu tampon neutre des cellules tumorales de lymphome artificielles dans le cas de la Recognine L et des cellules tumorales mammaires dans le cas de la Recognine M que l'on extrait par rupture répétée du tissu pour solubiliser des fractions protéiniques;

(ii) on sépare la fraction possédant un intervalle de pK de 1 à 4 de l'extrait résultant de protéines solubilisées; et

(iii) on isole de ladite fraction (ii) respectivement la Recognine L ou la Recognine M désirée, lesdits produits à base de Recognine L et de Recognine M étant caractérisés chacun en ce que:

(a) il forme un précipité avec son anticorps spécifique dans des tests quantitatifs de précipitine et dans des tests Ouchterlony de diffusion sur gel;

(b) il est soluble dans l'eau et dans les solutions aqueuses ayant un pH neutre ou acide, et insoluble à pH alcalin;

(c) il présente une longueur d'onde d'absorption spectrophotométrique de 280 m$\mu$; et

(d) il a un poids moléculaire de 8.000 tel que déterminé par chromatographie sur gel en couches minces, ledit produit à base de Recognine L étant caractérisé de plus par la composition approximative en aminoacides suivante:

# O O15 755

|  | Nombre de résidus aminoacide |
|---|---|
| Acide aspartique | 8 |
| Thréonine | 5 |
| Sérine | 5 |
| Acide glutamique | 10 |
| Proline | 5 |
| Glycine | 13 |
| Alanine | 10 |
| Valine | 6 |
| 1/2 Cystéine | 1 |
| Méthionine | 1 |
| Isoleucine | 4 |
| Leucine | 7 |
| Tyrosine | 1 |
| Phénylalanine | 3 |
| Lysine | 6 |
| Histidine | 2 |
| Arginine | 5 |
| Total approximatif | 92 |

et par le fait que les aminoacides acide cystéique, hydroxyproline, norleucine, ammoniaque, isodesmosine, desmosine, hydroxylysine, lysinonorleucine et acide gamma-aminobutyrique ne sont pas présents en quantité décelable, et ledit produit à base de Recognine étant de plus caractérisé par la composition approximative en aminoacides suivante:

|  | Nombre de résidus aminoacide |
|---|---|
| Acide aspartique | 9 |
| Thréonine | 5 |
| Sérine | 5 |
| Acide glutamique | 11 |
| Proline | 4 |
| Glycine | 9 |
| Alanine | 9 |
| Valine | 6 |
| 1/2 Cystéine | 1 |
| Méthionine | 1 |
| Isoleucine | 4 |
| Leucine | 8 |
| Tyrosine | 2 |
| Phénylalanine | 3 |
| Lysine | 6 |
| Histidine | 2 |
| Arginine | 5 |
| Total approximatif | 90 |

et par le fait que les aminoacides acide cystéique, hydroxyproline, norleucine, ammoniaque, isodesmosine, desmosine, hydroxylysine, lysinonorleucine et acide gamma-aminobutyrique ne sont pas présents en quantité décelable.

2. Procédé selon la revendication 1, caractérisé de plus en ce que ledit produit Recognine L ou Recognine M est capable de former un complexe avec la bromoacétylcellulose en donnant respectivement de la bromoacétylcellulose-Recognine L ou de la bromoacétylcellulose-Recognine M, et en ce que ladite Recognine L et ladite Recognine M produisent respectivement les anticorps spécifiques Anti-Recognine L et Anti-Recognine M, lorsqu'on les injecte chez des mammifères, lesdites Anti-Recognine L et Anti-Recognine M étant respectivement toxiques *in vitro* vis-à-vis des cellules tumorales et produisant la fluorescence des cellules du gliome lorsqu'elles sont associées à de la fluorescéine.

3. Procédé selon la revendication 1 ou la revendication 2, caractérisé de plus en ce qu'on effectue ladite étape de séparation (ii) en introduisant ledit extrait de protéines solubilisées dans une colonne chromatographique et en éluant avec des solvants d'acidité croissante.

4. Procédé selon la revendication 3, caractérisé de plus en ce qu'on réalise ladite étape d'isolation (iii) de la revendication 1 en filtrant l'éluat de l'étape (ii) de façon à obtenir respectivement une fraction contenant la Recognine L ou la Recognine M, et en séparant de celle-ci la Recognine L ou la Recognine M par chromatographie sur gel en couches minces.

5. Produit à base de polypeptide appelé Recognine L, caractérisé en ce qu'il forme un précipité en ligne unique avec son anticorps spécifique dans les tests quantitatifs de précipitine et dans les tests Ouchterlony de diffusion sur gel; il est soluble dans l'eau et dans les solutions aqueuses ayant un pH neutre ou acide, et insoluble à un pH alcalin; il posséde un pic d'absorption spectophotométrique à la longueur d'onde 280 m$\mu$; il a un poids moléculaire de 8.000 (tel qu'il est déterminé par chromatographie sur gel en couches minces), et il a la composition approximative en aminoacides suivante:

|  | Nombre de résidus aminoacide |
| --- | --- |
| Acide aspartique | 8 |
| Thréonine | 5 |
| Sérine | 5 |
| Acide glutamique | 10 |
| Proline | 5 |
| Glycine | 13 |
| Alanine | 10 |
| Valine | 6 |
| 1/2 Cystéine | 1 |
| Méthionine | 1 |
| Isoleucine | 4 |
| Leucine | 7 |
| Tyrosine | 1 |
| Phénylalanine | 3 |
| Lysine | 6 |
| Histidine | 2 |
| Arginine | 5 |
| Total approximatif | 92 |

et par le fait que les aminoacides acide cystéique, hydroxyproline, norleucine, ammoniaque, isodesmosine, desmosine, hydroxylysine, lysinonorleucine et acide gamma-aminobutyrique ne sont pas présents en quantité décelable.

6. Produit à base de polypeptide appelé Recognine M, caractérisé en ce qu'il forme un précipité en ligne unique aved son anticorps spécifique dans les tests quantitatifs de précipitine et les tests Ouchterlony de diffusion sur gel; il est soluble dans l'eau et dans les solutions aqueuses ayant un pH neutre ou acide, et insoluble à un pH alcalin; il possède un pic d'absorption spectrophotométrique à la longueur d'onde 280 m$\mu$; il a un poids moléculaire de 8.000 (tel qu'il est déterminé par chromatographie sur gel en couches minces), et il a la composition approximative en aminoacides suivante:

|  | Nombre de résidus aminoacide |
| --- | --- |
| Acide aspartique | 9 |
| Thréonine | 5 |
| Sérine | 5 |
| Acide glutamique | 11 |
| Proline | 4 |
| Glycine | 9 |
| Alanine | 9 |
| Valine | 6 |
| 1/2 Cystéine | 1 |
| Méthionine | 1 |
| Isoleucine | 4 |
| Leucine | 8 |
| Tyrosine | 2 |
| Phénylalanine | 3 |
| Lysine | 6 |
| Histidine | 2 |
| Arginine | 5 |
| Total approximatif | 90 |

et par le fait que les aminoacides acide cystéique, hydroxyproline, norleucine, ammoniaque, isodesmosine, desmosine, hydroxylysine, lysinonorleucine et acide gamma-aminobutyrique ne sont pas présents en quantité décelable.

7. Procédé pour produire le produit Anti-Recognine L ou Anti-Recognine M, caractérisé en ce qu'on l'injecte chez un mammifère ladite Recognine L ou ladite Recognine M comme revendiqué dans les revendications 5 ou 6, respectivement, ou produit par un procédé comme revendiqué dans l'une quelconque des revendications 1 à 4, produisant ainsi l'anticorps spécifique Anti-Recognine L ou Anti-Recognine M, respectivement, ladite Anti-Recognine L ou ladite Anti-Recognine M étant toxique *in vitro* vis-à-vis des cellules tumorales et produisant la fluorescence des cellules de gliome lorsqu'elles sont associées à de la fluorescéine, et en ce que l'on récupère ladite Anti-Recognine L ou ladite Anti-Recognine M, respectivement.

8. Produits Anti-Recognine L et Anti-Recognine M composés du produit anticorps fabriqué par injection chez un mammifère de Recognine L ou de Recognine M comme revendiqué dans les revendications 5 ou 6, respectivement, ou produit comme revendiqué dans l'une quelconque des revendications 1 à 4, lesdits anticorps spécifiques Anti-Recognine L et Anti-Recognine M étant chacun toxique vis-à-vis des cellules tumorales *in vitro* et produisant la fluorescence des cellules du gliome lorsqu'on les associe à de la fluorescéine.

9. Procédé pour fabriquer le complexe bromoacétylcellulose-Recognine L ou le complexe bromoacétylcellulose-Recognine M, caractérisé en ce que la Recognine L ou la Recognine M comme revendiqué dans les revendications 5 ou 6, respectivement, ou produite comme revendiqué dans l'une quelconque des revendications 1 à 4, est mélangée à de la bromoacétylcellulose d'une manière adaptée pour former ledit complexe bromoacétylcellulose-Recognine L ou ledit complexe bromoacétylcellulose-Recognine M, respectivement.

10. Complexes bromoacétylcellulose-Recognine L et bromoacétylcellulose-Recognine M, caractérisés comme complexes de la bromoacétylcellulose avec la Recognine L ou la Recognine M comme revendiqué dans les revendications 5 ou 6, respectivement, ou produits par un procédé tel que revendiqué dans l'une quelconque des revendications 1 à 4.

11. Procédé pour produire de la bromoacétylcellulose-Recognine L-Anti-Recognine L ou de la bromoacétylcellulose-Recognine M-Anti-Recognine M, caractérisé par la réaction de l'Anti-Recognine L ou de l'Anti-Recognine M, respectivement, comme revendiqué dans la revendication 8 ou produites par un procédé comme revendiqué dans la revendication 7, avec de la bromoacétylcellulose-Recognine L ou de la bromoacétylcellulose-Recognine M, respectivement, comme revendiqué dans la revendication 10, ou produites par un procédé comme revendiqué dans la revendication 9.

12. Complexes bromoacétylcellulose-Recognine L-Anti-Recognine L et bromoacétylcellulose-Recognine M-Anti-Recognine M, caractérisés comme complexes de l'Anti-Recognine L ou Anti-Recognine M, respectivement, comme revendiqué dans la revendication 8, ou produits par un procédé comme revendiqué dans la revendication 7, avec la bromoacétylcellulose-Recognine L ou la bromoacétylcellulose-Recognine M, respectivement, comme revendiqué dans la revendication 10, ou produits par un procédé comme revendiqué dans la revendication 9.

13. Procédé pour produire l'Anti-Recognine L ou l'Anti-Recognine M, respectivement, comme revendiqué dans la revendication 8, caractérisé en ce qu'on décomplexe la bromoacétylcellulose-Recognine L-Anti-Recognine L ou la Bromoacétylcellulose-Recognine M-Anti-Recognine M, respectivement, comme revendiqué dans la revendication 12, ou produites par un procédé comme revendiqué dans la revendication 11, par traitement hydrolytique avec de l'acide ou avec une enzyme protéinase.

14. Procédé pour produire une globuline se fixant sur une cible de façon lente (S-TAG), caractérisé en ce qu'on fait réagir du sérum sanguin ou un autre fluide corporel avec la bromoacétylcellulose-Recognine L ou la bromoacétylcellulose-Recognine M, respectivement, comme revendiqué dans la revendication 10, ou produite par un procédé comme revendiqué dans la revendication 9, pendant au moins deux heures à une température basse pour éviter les réactions de dégradation; et en ce qu'on traite le matériau résultant avec de l'acide dilué pour en séparer ladite globuline se fixant sur une cible de façon lente (S-TAG).

15. Produit S-TAG préparé selon le procédé de la revendication 14, ledit produit étant caractérisé en ce qu'il est soluble dans les solutions aqueuses tamponnées, qu'il forme un précipité en ligne unique avec la Recognine L ou la Recognine M dans les tests Ouchterlony de diffusion sur gel, est non-dialysable dans les membranes de cellophane, est retenu par les filtres millipore qui retiennent les molécules de poids moléculaire supérieur à 25.000, ont des poids moléculaires à différents états d'agrégation tels qu'on les détermine par chromatographie sur gel en couches minces d'environ 50.000, et de ses multiplex dans la gamme de macroglobulines, et présentent un pic d'absorption spectrophotométrique à la longueur d'onde de 280 m$\mu$.

16. Procédé pour fabriquer une globuline se fixant sur une cible de façon rapide, caractérisé en ce qu'on fait réagir le sérum sanguin ou un autre fluide corporel avec de la bromoacétylcellulose-Recognine L ou la bromoacétylcellulose-Recognine M, respectivement, comme revendiqué dans la revendication 10, on produit par un procédé comme revendiqué dans la revendication 9, pendant approximativement 10 minutes à une température basse pour éviter les réactions de dégradation; et en ce qu'on traite le matériau résultant avec de l'acide dilué pour en séparer ladite globuline se fixant sur une cible de façon rapide (F–TAG).

17. Produit F–TAG préparé selon le procédé de la revendication 16, ledit produit étant caractérisé par le fait qu'il est soluble dans les solutions aqueuses tamponnées, qu'il forme un précipité en ligne unique avec de la Recognine L ou de la Recognine M dans les tests Ouchterlony de diffusion sur gel, qu'il est non-dialysable dans les membranes de cellophane, qu'il est retenu par les filtres millipore qui retiennent les molécules de poids moléculaire supérieur à 25.000, qu'il a des poids moléculaires à différents états d'agrégation tels qu'ils sont déterminés par chromatographie sur gel en couches minces de 50.000 approximativement, et des multiples de celui-ci dans la gamme de macroglobulines; et qu'il a une pic d'absorption spectrophotométrique à la longueur d'onde 280 m$\mu$.

18. Méthode pour détecter les tumeurs cancéreuses dans les animaux vivants, caractérisée en ce qu'on détermine *in vitro* les concentrations en S–TAG et F–TAG, respectivement, produites par un volume connu du sérum sanguin ou d'un autre fluide corporel d'un mammifère.

19. Méthode pour diagnostiquer *in vitro* la présence de cellules tumorales dans une section histologique, caractérisée en ce qu'on applique un produit composé de fluorescéine conjuguée avec un produit TAG comme revendiqué dans la revendication 15 ou 17, respectivement, ou avec de l'Anti-Recognine L ou de l'Anti-Recognine M, respectivement, comme revendiqué dans la revendication 10, ou produite par un procédé comme revendiqué dans la revendication 9, à ladite section histologique, et en ce qu'on détermine si la fluorescence apparaît ou non, ladite fluorescence apparaissant seulement dans lesdites cellules tumorales.

20. Produits à base de polypeptides Recognine L et Recognine M, comme revendiqué dans la revendication 5 ou 6, respectivement, ou comme produits dans l'une quelconque des revendications 1 à 4, pour l'utilisation dans le diagnostic du cancer.

21. Produits anticorps Anti-Recognine L et Anti-Recognine M, respectivement, comme revendiqué dans la revendication 8 ou produits par un procédé comme revendiqué dans la revendication 7, pour l'utilisation dans le diagnostic du cancer.

22. Complexes bromoacétylcellulose-Recognine L et bromoacétylcellulose-Recognine M comme revendiqué dans la revendication 10 ou produits par un procédé comme revendiqué dans la revendication 9, pour l'utilisation dans le diagnostic du cancer.

23. Bromoacétylcellulose-Recognine L-Anti-Recognine L ou bromoacétylcellulose-Recognine M-Anti-Recognine M, respectivement, comme revendiqué dans la revendication 12, ou produites par un procédé comme revendiqué dans la revendication 11, pour l'utilisation dans le diagnostic du cancer.

24. Produits S–TAG et F–TAG comme revendiqué dans la revendication 15 ou 17, respectivement, ou produits par un procédé comme revendiqué dans la revendication 14 ou 16, respectivement, pour l'utilisation dans le diagnostic du cancer.

25. Produits à base de polypeptides Recognine L et Recognine M, comme revendiqué dans la revendication 5 ou 6, respectivement, ou comme produits dans l'une quelconque des revendications 1 à 4, pour l'utilisation dans la thérapie du cancer.

26. Produits anticorps Anti-Recognine L et Anti-Recognine M, respectivement, comme revendiqué dans la revendication 8, ou produits par un procédé comme revendiqué dans la revendication 7, pour l'utilisation dans la thérapie du cancer.

27. Complexes bromoacétylcellulose-Recognine L et bromoacétylcellulose-Recognine M, comme revendiqué dans la revendication 10 ou produits par un procédé comme revendiqué dans la revendication 9, pour l'utilisation dans la thérapie du cancer.

28. Bromoacétylcellulose-Recognine L-Anti-Recognine L ou bromoacétylcellulose-Recognine M-Anti-Recognine M, respectivement, comme revendiqué dans la revendication 11, pour l'utilisation dans la thérapie du cancer.

29. Produits S–TAG et F–TAG, comme revendiqué dans la revendication 15 ou 17, respectivement, ou produits par un procédé comme revendiqué dans la revendication 14 ou 16, respectivement, pour l'utilisation dans la thérapie du cancer.

**Revendications pour l'Etat Contractant: AT**

1. Procédé pour produire des produits à base de polypeptides la Recognine L et la Recognine M, respectivement, caractérisé en ce que:

(i) on cultive en milieu tampon neutre des cellules tumorales de lymphome artificielles dans le cas de la Recognine L et des cellules tumorales mammaires dans le cas de la Recognine M que l'on extrait par rupture répétée du tissu pour solubiliser des fractions protéiniques;

(ii) on sépare la fraction possédant un intervalle de pK de 1 à 4 de l'extrait résultant de protéines solubilisées; et

(iii) on isole de ladite fraction (ii) respectivement la Recognine L ou la Recognine M désirée, lesdits produits à base de Recognine L et de Recognine M étant caractérisés chacun en ce que:

(a) il forme un précipité avec son anticorps spécifique dans des tests quantitatifs de précipitine et dans des tests Ouchterlony de diffusion sur gel;

# 0 015 755

(b) il est soluble dans l'eau et dans les solutions aqueuses ayant un pH neutre ou acide, et insoluble à pH alcalin;

(c) il présente une longueur d'onde d'absorption spectrophotométrique de 280 m$\mu$; et

(d) il a un poids moléculaire de 8.000 tel que déterminé par chromatographie sur gel en couches minces, ledit produit à base de Recognine L étant caractérisé de plus par la composition approximative en aminoacides suivante:

|  | Nombre de résidus aminoacide |
| --- | --- |
| Acide aspartique | 8 |
| Thréonine | 5 |
| Sérine | 5 |
| Acide glutamique | 10 |
| Proline | 5 |
| Glycine | 13 |
| Alanine | 10 |
| Valine | 6 |
| 1/2 Cystéine | 1 |
| Méthionine | 1 |
| Isoleucine | 4 |
| Leucine | 7 |
| Tyrosine | 1 |
| Phénylalanine | 3 |
| Lysine | 6 |
| Histidine | 2 |
| Arginine | 5 |
| Total approximatif | 92 |

et par le fait que les aminoacides acide cystéique, hydroxyproline, norleucine, ammoniaque, isodesmosine, desmosine, hydroxylysine, lysinonorleucine et acide gamma-aminobutyrique ne sont pas présents en quantité décelable, et ledit produit à base de Recognine étant de plus caractérisé par la composition approximative en aminoacides suivante:

|  | Nombre de résidus aminoacide |
| --- | --- |
| Acide aspartique | 9 |
| Thréonine | 5 |
| Sérine | 5 |
| Acide glutamique | 11 |
| Proline | 4 |
| Glycine | 9 |
| Alanine | 9 |
| Valine | 6 |
| 1/2 Cystéine | 1 |
| Méthionine | 1 |
| Isoleucine | 4 |
| Leucine | 8 |
| Tyrosine | 2 |
| Phénylalanine | 3 |
| Lysine | 6 |
| Histidine | 2 |
| Arginine | 5 |
| Total approximatif | 90 |

et par le fait que les aminoacides acide cystéique, hydroxyproline, norleucine, ammoniaque, isodesmosine, desmosine, hydroxylysine, lysinonorleucine et acide gamma-aminobutyrique ne sont pas présents en quantité décelable.

2. Procédé selon la revendication 1, caractérisé de plus en ce que ledit produit Recognine L ou Recognine M est capable de former un complexe avec la bromoacétylcellulose en donnant respectivement de la bromoacétylcellullose-Recognine L ou de la bromoacétylcellulose-Recognine M, et en ce que ladite Recognine L et ladite Recognine M produisent respectivement les anticorps spécifiques Anti-Recognine L et Anti-Recognine M, lorsqu'on les injecte chez des mammifères, lesdites Anti-Recognine L et Anti-Recognine M étant respectivement toxiques *in vitro* vis-à-vis des cellules tumorales et produisant la fluorescence des cellules du gliome lorsqu'elles sont associées à de la fluorescéine.

3. Procédé selon la revendication 1 ou la revendication 2, caractérisé de plus en ce qu'on effectue ladite étape de séparation (ii) en introduisant ledit extrait de protéines solubilisées dans une colonne chromatographique et en éluant avec des solvants d'acidité croissante.

4. Procédé selon la revendication 3, caractérisé de plus en ce qu'on réalise ladite étape d'isolation (iii) de la revendication 1, en filtrant l'éluat de l'étape (ii) de façon à obtenir respectivement une fraction contenant la Recognine L ou la Recognine M, et en séparant de celle-ci la Recognine L ou la Recognine M par chromatographie sur gel en couches minces.

5. Produit à base de polypeptide appelé Recognine L, caractérisé en ce qu'il forme un précipité en ligne unique avec son anticorps spécifique dans les tests quantitatifs de précipitine et dans les tests Ouchterlony de diffusion sur gel; il est soluble dans l'eau et dans les solutions aqueuses ayant un pH neutre ou acide, et insoluble à un pH alcalin; il possède un pic d'absorption spectrophotométrique à la longueur d'onde 280 m$\mu$; il a un poids moléculaire de 8.000 (tel qu'il est déterminé par chromatographie sur gel en couches minces), et il a la composition approximative en aminoacides suivante:

|  | Nombre de résidus aminoacide |
|---|---|
| Acide aspartique | 8 |
| Thréonine | 5 |
| Sérine | 5 |
| Acide glutamique | 10 |
| Proline | 5 |
| Glycine | 13 |
| Alanine | 10 |
| Valine | 6 |
| 1/2 Cystéine | 1 |
| Méthionine | 1 |
| Isoleucine | 4 |
| Leucine | 7 |
| Tyrosine | 1 |
| Phénylalanine | 3 |
| Lysine | 6 |
| Histidine | 2 |
| Arginine | 5 |
| Total approximatif | 92 |

et par le fait que les aminoacides acide cystéique, hydroxyproline, norleucine, ammoniaque, isodesmosine, desmosine, hydroxylysine, lysinonorleucine et acide gamma-aminobutyrique ne sont pas présents en quantité décelable.

6. Produit à base de polypeptide appelé Recognine M, caractérisé en ce qu'il forme un précipité en ligne unique avec son anticorps spécifique dans les tests quantitatifs de précipitine et les tests Ouchterlony de diffusion sur gel; il est soluble dans l'eau et dans les solutions aqueuses ayant un pH neutre ou acide, et insoluble à un pH alcalin; il possède un pic d'absorption spectrophotométrique à la longueur d'onde 280 m$\mu$; il a un poids moléculaire de 8.000 (tel qu'il est déterminé par chromatographie sur gel en couches minces), et il a la composition approximative en aminoacides suivante:

| | Nombre de résidus aminoacide |
|---|---|
| Acide aspartique | 9 |
| Thréonine | 5 |
| Sérine | 5 |
| Acide glutamique | 11 |
| Proline | 4 |
| Glycine | 9 |
| Alanine | 9 |
| Valine | 6 |
| 1/2 Cystéine | 1 |
| Méthionine | 1 |
| Isoleucine | 4 |
| Leucine | 8 |
| Tyrosine | 2 |
| Phénylalanine | 3 |
| Lysine | 6 |
| Histidine | 2 |
| Arginine | 5 |
| Total approximatif | 90 |

et par le fait que les aminoacides acide cystéique, hydroxyproline, norleucine, ammoniaque, isodesmosine, desmosine, hydroxylysine, lysinonorleucine et acide gamma-aminobutyrique ne sont pas présents en quantité décelable, pour l'utilisation dans le diagnostic du cancer.

7. Procédé pour produire le produit Anti-Recognine L ou Anti-Recognine M, caractérisé en ce qu'on l'injecte chez un mammifère ladite Recognine L ou ladite Recognine M comme revendiqué dans les revendications 5 ou 6, respectivement, ou produit par un procédé comme revendiqué dans l'une quelconque des revendications 1 à 4, produisant ainsi l'anticorps spécifique Anti-Recognine L ou Anti-Recognine M, respectivement, ladite Anti-Recognine L ou ladite Anti-Recognine M étant toxique *in vitro* vis-à-vis des cellules tumorales et produisant la fluorescence des cellules de gliome lorsqu'elles sont associées à de la fluorescéine, et en ce que l'on récupère ladite Anti-Recognine L ou ladite Anti-Recognine M, respectivement.

8. Produits Anti-Recognine L et Anti-Recognine M composés du produit anticorps fabriqué par injection chez un mammifère de Recognine L ou de Recognine M comme revendiqué dans les revendications 5 ou 6, respectivement, ou product comme revendiqué dans l'une quelconque des revendications 1 à 4, lesdits anticorps spécifiques Anti-Recognine L et Anti-Recognine M étant chacun toxique vis-à-vis des cellules tumorales *in vitro* et produisant la fluorescence des cellules du gliome lorsqu'on les associe à de la fluorescéine, pour l'utilisation dans le diagnostic du cancer.

9. Procédé pour fabriquer le complexe bromoacétylcellulose-Recognine L ou le complexe bromoacétylcellullose-Recognine M, caractérisé en ce que la Recognine L ou la Recognine M comme revendiqué dans les revendications 5 ou 6, respectivement, ou produite comme revendiqué dans l'une quelconque des revendications 1 à 4, est mélangée à de la bromoacétylcellulose d'une manière adaptée pour former ledit complexe bromoacétylcellulose-Recognine L ou ledit complexe bromoacétylcellulose-Recognine M, respectivement.

10. Complexes bromoacétylcellulose-Recognine L et bromoacétylcellulose-Recognine M, caractérisés comme complexes de la bromoacétylcellulose avec la Recognine L ou la Recognine M comme revendiqué dans les revendications 5 ou 6, respectivement, ou produits par un procédé tel que revendiqué dans l'une quelconque des revendications 1 à 4, pour l'utilisation dans le diagnostic du cancer.

11. Procédé pour produire de la bromoacétylcellulose-Recognine L-Anti-Recognine L ou de la bromoacétylcellulose-Recognine M-Anti-Recognine M, caractérisé par la réaction de l'Anti-Recognine L ou de l'Anti-Recognine M, respectivement, comme revendiqué dans la revendication 8 ou produites par un procédé comme revendiqué dans la revendication 7, avec de la bromoacétylcellulose-Recognine L out de la bromoacétylcellulose-Recognine M, respectivement, comme revendiqué dans la revendication 10, ou produites par un procédé comme revendiqué dans la revendication 9.

12. Complexes bromoacétylcellulose-Recognine L-Anti-Recognine L et bromoacétylcellulose-Recognine M-Anti-Recognine M, caractérisés comme complexes de l'Anti-Recognine L ou Anti-Recognine M, respectivement, comme revendiqué dans la revendication 8, ou produits par un procédé comme revendiqué dans la revendication 7, avec la bromoacétylcellulose-Recognine L ou la bromoacétylcellulose-Recognine M, respectivement, comme revendiqué dans la revendication 10, ou produits par un procédé comme revendiqué dans la revendication 9, pour l'utilisation dans le diagnostic du cancer.

13. Procédé pour produire l'Anti-Recognine L ou l'Anti-Recognine M, respectivement, comme revendiqué dans la revendication 8, caractérisé en ce qu'on décomplexe la bromoacétylcellulose-Recognine L-Anti-Recognine L ou la bromoacétylcellulose-Recognine M-Anti-Recognine M, respectivement, comme revendiqué dans la revendication 12, ou produites par un proiédé comme revendiqué dans la revendication 11, par traitement hydrolytique avec de l'acide ou avec une enzyme protéinase.

14. Procédé pour produire une globuline se fixant sur une cible de façon lente (S-TAG), caractérisé en ce qu'on fait réagir du sérum sanguin ou un autre fluide corporel avec la bromoacétylcellulose-Recognine L ou la bromoacétylcellulose-Recognine M, respectivement, comme revendiqué dans la revendication 10, ou produite par un procédé comme revendiqué dans la revendication 9, pendant au moins deux heures à une température basse pour éviter les réactions de dégradation; et en ce qu'on traite le matériau résultant avec de l'acide dilué pour en séparer, ladite globuline se fixant sur une cible de façon lente (S-TAG).

15. Produit S-TAG préparé selon le procédé de la revendication 14, ledit produit étant caractérisé en ce qu'il est soluble dans les solutions aqueuses tamponnées, qu'il forme un précipité en ligne unique avec la Recognine L ou la Recognine M dans les tests Ouchterlony de diffusion sur gel, est non-dialysable dans les membranes de cellophane, est retenu par les filtres millipore qui retiennent les molécules de poids moléculaire supérieur à 25.000, ont des poids moléculaires à différents états d'agrégation tels qu'on les détermine par chromatographie sur gel en couches minces d'environ 50.000, et de ses multiples dans la gamme de macroglobulines, et présentent un pic d'absorption spectrophotométrique à la longueur d'onde de 280 m$\mu$, pour l'utilisation dans le diagnostic du cancer.

16. Procédé pour fabriquer u e globuline se fixant sur une cible de façon rapide, caractérisé en ce qu'on fait réagir le sérum sanguin ou un autre fluide corporel avec de la bromoacétylcellulose-Recognine L ou la bromoacétylcellulose-Recognine M, respectivement, comme revendiqué dans la revendication 10, on produit par un procédé comme revendiqué dans la revendication 9, pendant approximativement 10 minutes à une température basse pour éviter les réactions de dégradation; et en ce qu'on traite le matériau résultant avec de l'acide dilué pour en séparer ladite globuline se fixant sur une cible de façon rapide (F-TAG).

17. Produit F-TAG préparé selon le procédé de la revendication 16, ledit produit étant caractérisé par le fait qu'il est soluble dans les solutions aqueuses tamponnées, qu'il forme un précipité en ligne unique avec de la Recognine L ou de la Recognine M dans les tests Ouchterlony de diffusion sur gel, qu'il est non-dialysable dans les membranes de cellophane, qu'il est retenu par les filtres millipore qui retiennent les molécules de poids moléculaire supérieur à 25.000, qu'il a des poids moléculaires à différents états d'agrégation tels qu'ils sont déterminés par chromatographie sur gel en couches minces de 50.000 approximativement, et des multiplex de celui-ci dans la gamme de macroglobulines; et qu'il a un pic d'absorption spectrophotométrique à la longueur d'onde 280 m$\mu$, pour l'utilisation dans le diagnostic du cancer.

18. Méthode pour détecter les tumeurs cancéreuses dans les animaux vivants, caractérisée en ce qu'on détermine *in vitro* les concentrations en S-TAG et F-TAG, respectivement, produites par un volume connu du sérum sanguin ou d'un autre fluide corporel d'un mammifère.

19. Méthode pour diagnostiquer *in vitro* la présence de cellules tumorales dans une section histologique, caractérisée en ce qu'on applique un produit composé de fluorescéine conjuguée avec un produit TAG comme revendiqué dans la revendication 15 ou 17, respectivement, ou avec de l'Anti-Recognine L ou de l'Anti-Recognine M, respectivement, comme revendiqué dans la revendication 10, ou produite par un procédé comme revendiqué dans la revendication 9, à ladite section histologique, et en ce qu'on détermine si la fluorescence apparaît ou non, ladite fluorescence apparaissant seulement dans lesdites cellules tumorales.

20. Produit à base de polypeptide appelé Recognine L, caractérisé en ce qu'il forme un précipité en ligne unique avec son anticorps spécifique dans des tests quantitatifs de précipitine et dans des tests Ouchterlony de diffusion sur gel; en ce qu'il est soluble dans l'eau et dans les solutions aqueuses ayant un pH acide ou neutre, et insoluble à un pH alcalin; il a un pic d'absorption spectrophotométrique à la longueur d'onde 280 m$\mu$; il a un poids moléculaire de 8.000 (tel qu'il est déterminé par chromatographie sur gel en couches minces), et il a la composition approximative en aminoacides suivante:

| | Nombre de résidus aminoacide |
|---|---|
| Acide aspartique | 8 |
| Thréonine | 5 |
| Sérine | 5 |
| Acide glutamique | 10 |
| Proline | 5 |
| Glycine | 13 |
| Alanine | 10 |
| Valine | 6 |
| 1/2 Cystéine | 1 |
| Méthionine | 1 |
| Isoleucine | 4 |
| Leucine | 7 |
| Tyrosine | 1 |
| Phénylalanine | 3 |
| Lysine | 6 |
| Histidine | 2 |
| Arginine | 5 |
| Total approximatif | 92 |

et par le fait que les aminoacides acide cystéique, hydroxyproline, norleucine, ammoniaque, isodesmosine, desmosine, hydroxylysine, lysinonorleucine et acide gamma-aminobutyrique ne sont pas présents en quantitié décelable, pour l'utilisation dans la thérapie du cancer.

21. Produit à base de polypeptide appelé Recognine M, caractérisé en ce qu'il forme un précipité en ligne unique avec son anticorps spécifique dans les tests quantitatifs de précipitine et dans des tests Ouchterlony de diffusion sur gel; en ce qu'il est soluble dans l'eau et dans les solutions aqueuses ayant un pH acide ou neutre, et insoluble à un pH alcalin; il a un pic d'absorption spectrophotométrique à la longueur d'onde de 280 m$\mu$; il a un poids moléculaire de 8.000 (tel qu'il est déterminé par chromatographie sur gel en couches minces), et a la composition approximative en aminoacides suivante:

| | Nombre de résidus aminoacide |
|---|---|
| Acide aspartique | 9 |
| Thréonine | 5 |
| Sérine | 5 |
| Acide glutamique | 11 |
| Proline | 4 |
| Glycine | 9 |
| Alanine | 9 |
| Valine | 6 |
| 1/2 Cystéine | 1 |
| Méthionine | 1 |
| Isoleucine | 4 |
| Leucine | 8 |
| Tyrosine | 2 |
| Phénylalanine | 3 |
| Lysine | 6 |
| Histidine | 2 |
| Arginine | 5 |
| Total approximatif | 90 |

et par le fait que les aminoacides acide cystéique, hydroxyproline, norleucine, ammoniaque, isodesmosine, desmosine, hydroxylysine, lysinonorleucine et acide gamma-aminobutyrique ne sont pas présents en quantité décelable, pour l'utilisation dans la thérapie du cancer.

22. Produits Anti-Recognine L et Anti-Recognine M composés du produit anticorps fabriqué par injection chez un mammifère de Recognine L ou de Recognine M comme revendiqué dans la revendication 5 ou 6, respectivement, ou produit comme revendiqué dans l'une quelconque des revendications 1 à 4, lesdits anticorps Anti-Recognine L et Anti-Recognine M étant chacun toxique vis-à-vis des cellules tumorales *in vitro* et produisant la fluorescence des cellules du gliome lorsqu'on les associe à de la fluorescéine, pour l'utilisation dans la thérapie du cancer.

23. Complexes bromoacétylcellulose-Recognine L et bromoacétylcellulose-Recognine M, caractérisé comme complexes de la bromoacétylcellulose avec la Recognine L ou la Recognine M comme revendiqué dans la revendication 5 ou 6, respectivement, ou produits par un procédé tel que revendiqué dans l'une quelconque des revendications 1 à 4, pour l'utilisation dans la thérapie du cancer.

24. Complexes bromoacétylcellulose-Recognine L-Anti-Recognine L et bromoacétylcellulose-Recognine M-Anti-Recognine M, caractérisés comme complexes de l'Anti-Recognine L ou de l'Anti-Recognine M, respectivement, comme revendiqué dans la revendication 8, ou produits par un procédé comme revendiqué dans la revendication 7, avec la bromoacétylcellulose-Recognine L ou la bromoacétylcellulose-Recognine M, respectivement, comme revendiqué dans la revendication 10, ou produits par un procédé comme revendiqué dans la revendication 9, pour l'utilisation dans la thérapie du cancer.

25. Produit S-TAG préparé selon le procédé de la revendication 14, ledit produit étant caractérisé en ce qu'il est soluble dans les solutions aqueuses tamponnées, qu'il forme un précipité en ligne unique avec la Recognine L ou la Recognine M dans des tests Ouchterlony de diffusion sur gel, il est non-dialysable dans les membranes de cellophane, il est retenu par des filtres millipore qui retiennent les molécules de poids moléculaire supérieur à 25.000, ont des poids moléculaires à différents états d'agrégation tels qu'on les détermine par chromatographie sur gel en couches minces de 50.000 approximativement, et de ses multiples dans la gamme de macroglobulines et présentent un pic d'absorption spectrophotométrique à la longueur d'onde de 280 m$\mu$, pour l'utilisation dans la thérapie du cancer.

26. Produit F-TAG préparé selon le procédé de la revendication 16, ledit produit étant caractérisé en ce qu'il est soluble dans les solutions aqueuses tamponnées, forme un précipité en ligne unique avec de la Recognine L ou de la Recognine M dans les tests Ouchterlony de diffusion sur gel, il est non-dialysable dans les membranes de cellophane, il est retenu par des filtres millipore qui retiennent les molécules de poids moléculaire supérieur à 25.000 ayant des poids moléculaires sous différents états d'agrégation tels qu'ils sont déterminés par chromatographie sur gel en couches minces de 50.000 approximativement, et des multiples de ceux-ci dans la gamme de macroglobulines, et. a un pic d'absorption spectrophotométrique à la longueur d'onde 280 m$\mu$, pour l'utilisation dans la thérapie du cancer.

**Patentansprüche für die Vertragsstaaten: BE, CH, DE, FR, GB, IT, LU, NL, SE**

1. Verfahren zur Herstellung der Polypeptidprodukte Recognin L bzw. Recognin M, dadurch gekennzeichnet, daß man

(i) künstliche Lymphom-Tumorzellen im Falle von Recognin L und Mamma-Tumorzellen im Falle von Recognin M mit einem neutralen Puffer in Kultur züchtet und durch wiederholtes Aufbrechen des Gewebes zur Solubilisierung der Proteinfraktionen extrahiert,

(ii) die Fraktion mit einem pK-Bereich von 1 bis 4 von dem resultierenden Extrakt der solubilisierten Proteine abtrennt und

(iii) das gewünschte Recognin-L- bzw. Recognin-M-Produkt aus der genannten Fraktion (ii) isoliert, wobei die Recognin-L- und Recognin-M-Produkte jeweils durch folgendes charakterisiert sind:

(a) Bildung eines Niederschlags mit seinem spezifischen Antikörper bei quantitativen Präzipitintests und Ouchterlony-Geldiffusionstests,

(b) Löslichkeit in Wasser und wäßrigen Lösungen mit einem sauren oder neutralen pH-Wert und Unlöslichkeit bei einem alkalischen pH-Wert,

(c) eine spektrophotometrische Adsorptionswellenlänge von 280 m$\mu$ und

(d) ein Molekulargewicht von 8000 (bestimmt durch Dünnschicht Gelchromatographie), wobei das Recognin-L-Produkt weiterhin durch eine Aminosäurezusammensetzung, die im wesentlichen wie folgt ist:

|  | Anzahl der Amino-säurereste |
| --- | --- |
| Asparaginsäure | 8 |
| Threonin | 5 |
| Serin | 5 |
| Glutaminsäure | 10 |
| Prolin | 5 |
| Glycin | 13 |
| Alanin | 10 |
| Valin | 6 |
| 1/2-Cystein | 1 |
| Methionin | 1 |
| Isoleucin | 4 |
| Leucin | 7 |
| Tryosin | 1 |
| Phenylalanin | 3 |
| Lysin | 6 |
| Histidin | 2 |
| Arginin | 5 |
| ungefähre Gesamtsumme | 92 |

und durch die Tatsache, daß die Aminosäuren Cysteinsäure, Hydroxyprolin, Norleucin, Ammoniak, Isodesmosin, Desmosin, Hydroxylysin, Lysinonorleucin und gamma-Aminobuttersäure in erfaß baren Mengen nicht vorhanden, sind charakterisiert ist und wobei das Recognin-M-Produkt weiterhin durch eine Aminosäurenzusammensetzung, die im wesentlichen wie folgt ist:

|  | Anzahl der Amino-säurereste |
| --- | --- |
| Asparaginsäure | 9 |
| Threonin | 5 |
| Serin | 5 |
| Glutaminsäure | 11 |
| Prolin | 4 |
| Glycin | 9 |
| Alanin | 9 |
| Valin | 6 |
| 1/2 Cystein | 1 |
| Methionin | 1 |
| Isoleucin | 4 |
| Leucin | 8 |
| Tyrosin | 2 |
| Phenylalanin | 3 |
| Lysin | 6 |
| Histidin | 2 |
| Arginin | 5 |
| ungefähre Gesamtsumme | 90 |

und durch die Tatsache, daß die Aminosäuren Cysteinsäure, Hydroxyprolin, Norleucin, Ammoniak, Isodesmosin, Desmosin, Hydroxylysin, Lysinonorleucin und gamma-Aminobuttersäure in erfaßbaren Mengen nicht vorhanden sind, charakterisiert ist.

2. Verfahren nach Anspruch 1, dadurch gekennzeichnet, daß das genannte Recognin-L- oder Recognin-M-Produkt dazu imstande ist, mit Bromacetylcellulose unter Bildung von Bromacetylcellulose-Recognin L bzw. Bromacetylcellulose-Recognin M einen Komplex zu bilden, und daß das genannte Recognin L und Recognin M die spezifischen Antikörper Anti-Recognin L bzw. Anti-Recognin M nach Injektion in Säugetiere erzeugen, wobei das genannte Anti-Recognin L bzw. Anti-Recognin M gegenüber Tumorzellen in vitro toxisch ist und bei der Kupplung mit Fluoroescein eine Fluoreszenz von Glyomzellen erzeugen.

3. Verfahren nach Anspruch 1 oder 2, dadurch gekennzeichnet, daß man die Abtrennungsstufe (ii) in der Weise bewirkt, daß man den genannten Extrakt von solubilisierten Proteinen auf eine chromatographische Säule aufgibt und mit steigend sauren Lösungsmitteln eluiert.

4. Verfahren nach Anspruch 3, dadurch gekennzeichnet, daß man die genannte Isolierungsstufe

**0 015 755**

(iii) nach Anspruch 1 in der Weise durchführt, daß man das Eluat der Stufe (ii) filtriert, um eine Recognin L bzw. Recognin M enthaltende Fraktion zu erhalten, und daß man das Recognin L oder Recognin M daraus durch Dünnschicht-Gelchromatographie abtrennt.

5. Als Recognin L bezeichnetes Polypeptidprodukt, dadurch gekennzeichnet, daß es ein Einzellinien-Präzipitat mit seinem spezifischen Antikörper bei quantitativen Präzipitintests und Ouchterlony-Geldiffusionstests bildet, daß es in Wasser und wäßrigen Lösungen mit einem sauren oder neutralen pH-Wert löslich und bei einem alkalischen pH-Wert unlöslich ist, daß es einen spektrophotometrischen Absorptionspeak bei einer Wellenlänge von 280 $m\mu$ hat, daß es ein Moleculargewicht von 8000 (bestimmt durch Dünnschicht-Gelchromatographie) hat und daß es eine Aminosäurenzusammensetzung, die im wesentlichen wie folgt ist:

| | Anzahl der Amino-säurereste |
|---|---|
| Asparaginsäure | 8 |
| Threonin | 5 |
| Serin | 5 |
| Glutaminsäure | 10 |
| Prolin | 5 |
| Glycin | 13 |
| Alanin | 10 |
| Valin | 6 |
| 1/2-Cystein | 1 |
| Methionin | 1 |
| Isoleucin | 4 |
| Leucin | 7 |
| Tyrosin | 1 |
| Phenylalanin | 3 |
| Lysin | 6 |
| Histidin | 2 |
| Arginin | 5 |
| ungefähre Gesamtsumme | 92 |

aufweist und gekennzeichnet durch die Tatsache, daß die Aminosäuren Cysteinsäure, Hydroxyprolin, Norleucin, Ammoniak, Isodesmosin, Desmosin, Hydroxylysin, Lysinonorleucin und gamma-Aminobuttersäure in erfaßbaren Mengen nicht vorhanden sind.

6. Als Recognin M bezeichnetes Polypeptidprodukt, dadurch gekennzeichnet, daß es ein Einzellinien Präzipitat mit seinem spezifischen Antikörper bei quantitativen Präzipitintests und Ouchterlony-Geldiffusionstests bildet, daß es in Wasser und wäßrigen Lösungen mit einem sauren oder neutralen pH-Wert löslich und bei einem alkalischen pH-Wert unlöslich ist, daß es einen spektrophotometrischen Absorptionspeak bei einer Wellenlänge von 280 $m\mu$ hat, daß es ein Moleculargewicht von 8000 (bestimmt durch Dünnschicht-Gelchromatographie) hat und daß es eine Aminosäurenzusammensetzung, die im wesentlichen wie folgt ist:

| | Anzahl der Amino-säurereste |
|---|---|
| Asparaginsäure | 9 |
| Threonin | 5 |
| Serin | 5 |
| Glutaminsäure | 11 |
| Prolin | 4 |
| Glycin | 9 |
| Alanin | 9 |
| Valin | 6 |
| 1/2-Cystein | 1 |
| Methionin | 1 |
| Isoleucin | 4 |
| Leucin | 8 |
| Tyrosin | 2 |
| Phenylalanin | 3 |
| Lysin | 6 |
| Histidin | 2 |
| Arginin | 5 |
| ungefähre Gesamtsumme | 90 |

49

**0 015 755**

aufweist und gekennzeichnet durch die Tatsache, daß die Aminosäuren Cysteinsäure, Hydroxyprolin, Norleucin, Ammoniak, Isodesmosin, Desmosin, Hydroxylysin, Lysinonorleucin und gamma-Aminobuttersäure in erfaßbaren Mengen nicht vorhanden sind.

7. Verfahren zur Herstellung des Produkts Anti-Recognin L oder Anti-Recognin M, dadurch gekennzeichnet, daß man in ein Säugetier das genannte Recognin L oder Recognin M nach Anspruch 5 bzw. 6 oder hergestellt nach einem Verfahren nach einem der Ansprüche 1 bis 4 injiziert, wodurch man den spezifischen Antikörper Anti-Recognin L oder Anti-Recognin M erzeugt, wobei das Anti-Recognin L oder Anti-Recognin M gegenüber Tumorzellen in vitro toxisch ist, und bei der Kupplung mit Fluorescein eine Fluoreszenz von Glyomzellen erzeugt, und daß man das Anti-Recognin L bzw. Anti-Recognin M gewinnt.

8. Die Produkte Anti-Recognin L und Anti-Recognin M, bestehend aus dem Antikörperprodukt, hergestellt durch Injektion von Recognin L oder Recognin M nach Anspruch 5 bzw. 6 oder hergestellt nach einem der Ansprüche 1 bis 4 in ein Säugetier, wobei die spezifischen Antikörper Anti-Recognin L und Anti-Recognin M jeweils genenüber Tumor zellen in vitro toxisch sind und bei der Kupplung mit Fluorescein eine Fluoreszenz von Glyomzellen erzeugen.

9. Verfahren zur Herstellung des Komplexes Bromacetylcellulose-Recognin L oder Bromacetylcellulose-Recognin M, dadurch gekennzeichnet, daß man Recognin L oder Recognin M nach Anspruch 5 bzw. 6 oder hergestellt nach einem der Ansprüche 1 bis 4 mit Bromacetylcellulose in einer Weise vermischt, die dazu geeignet ist, den genannten Komplex Bromacetylcellulose-Recognin L bzw. Bromacetylcellulose-Recognin M zu bilden.

10. Die Komplexe Bromacetylcellulose-Recognin L und Bromacetylcellulose-Recognin M, gekennzeichnet als Komplexe von Bromacetylcellulose mit Recognin L oder Recognin M nach Anspruch 5 bzw. 6 oder heregestellt durch ein Verfahren nach einem der Ansprüche 1 bis 4.

11. Verfahren zur Herstellung von Bromacetylcellulose-Recognin-L-Anti-Recognin L oder Bromacetylcellulose-Recognin-M-Anti-Recognin-M, gekennzeichnet durch Reaktion von Anti-Recognin L bzw. Anti-Recognin M nach Anspruch 8 oder hergestellt nach einem Verfahren nach Anspruch 7, mit Bromacetylcellulose-Recognin L bzw. Bromacetylcellulose-Recognin M nach Anspruch 10 oder hergestellt nach einem Verfahren nach Anspruch 9.

12. Die Komplexe Bromacetylcellulose-Recognin-L-Anti-Recognin L und Bromacetylcellulose-Recognin-M-Anti-Recognin M, gekennzeichnet als Komplexe von Anti-Recognin L bzw. Anti-Recognin M nach Anspruch 8 oder hergestellt nach einem Verfahren nach Anspruch 7, mit Bromacetylcellulose-Recognin L bzw. Bromacetylcellulose-Recognin M nach Anspruch 10 oder hergestellt durch ein Verfahren nach Anspruch 9.

13. Verfahren zur Herstellung von Anti-Recognin L bzw. Anti-Recognin M nach Anspruch 8, dadurch gekennzeichnet, daß man Bromacetylcellulose-Recognin-L-Anti-Recognin L bzw. Bromacetylcellulose-Recognin-M-Anti-Recognin M nach Anspruch 12 oder hergestellt durch ein Verfahren nach Anspruch 11 durch hydrolytische Behandlung mit Säure oder mit einem Proteinaseenzym entkomplexiert.

14. Verfahren zur Herstellung eines sich langsam an das Ziel anheftenden Globulins (S-TAG), dadurch gekennzeichnet, daß man Blutserum oder eine andere Körperflüssigkeit mit Bromacetylcellulose-Recognin L bzw. Bromacetylcellulose-Recognin M nach Anspruch 10 oder hergestellt durch ein Verfahren nach Anspruch 9 mindestens 2 h bei niedriger Temperatur, daß Abbaureaktionen verhindert werden, umsetzt und daß man das resultierende Material mit verdünnter Säure behandelt, um davon das genannte sich langsam an das Ziel anheftende Globulin (S-TAG) abzuspalten.

15. Ein Produkt S-TAG, hergestellt nach dem Verfahren nach Anspruch 14, wobei das Produkt dadurch charakterisiert ist, daß es in wäßrigen gepufferten Lösungen löslich ist, ein Einzellinien-Präzipitat mit Recognin L oder Recognin M bei Ouchterlony-Geldiffusionstests bildet, in Cellophanmembranen nicht dialysierbar ist, von Milliporefiltern, die Moleküle mit einem Molekulargewicht von über 25000 zurückhalten, zurückgehalten wird, Molekulargewichte in verschiedenen Aggregationszuständen, bestimmt durch Dünnschicht-Gelchromatographie von ungefähr 50000 und vielfachem davon in den Makroglobulinbereich hinein aufweist, und daß es eine spektrophotometrische Adsorptionspeak-Wellenlänge von 280 m$\mu$ aufweist.

16. Verfahren zur Herstellung von sich schnell an das ziel anheftendem Globulin (F-TAG), dadurch gekennzeichnet, daß man Blutserum oder eine andere Körperflüssigkeit mit Bromacetylcellulose-Recognin L bzw. Bromacetylcellulose-Recognin M nach Anspruch 10 oder hergestellt nach einem Verfahren nach Anspruch 9 ungefähr 10 min bei einer niedrigen Temperatur, daß Abbaureaktionen verhindert werden, umsetzt und daß man das resultierende Material mit verdünnter Säure behandelt, um davon das sich schnell an das Ziel anheftende Globulin (F-TAG) abzuspalten.

17. Ein Produkt F-TAG, hergestellt gemäß dem Verfahren nach Anspruch 16, wobei das Produkt dadurch charakterisiert ist, daß es in wäßrigen gepufferten Lösungen löslich ist, ein Einzellinien-Präzipitat mit Recognin L oder Recognin M bei Ouchterlony-Geldiffusionstests bildet, in Cellophanmembranen nicht dialysierbar ist, von Milliporefiltern, die Moleküle mit einem Molekulargewicht von mehr als 25000 zurückhalten, zurückgehalten wird, daß es Molekulargewichte in verschiedenen Aggregationszuständen, bestimmt durch Dünnschicht-Gelchromatographie, von ungefähr 50000 und

50

Vielfache davon in den Makroglobulinbereich hinein aufweist und daß es eine spektrophotometrische Adsorptionspeak-Wellenlänge von 280 mμ hat.

18. Verfahren zur Erfassung von Krebstumoren in lebenden Tieren, dadurch gekennzeichnet, daß man in vitro die Konzentrationen von S-TAG bzw. F-TAG nach den Ansprüchen 15 bzw. 17, hergestellt durch ein bekanntes Volumen des Blutserums des Säugetiers oder einer anderen Körperflüssigkeit, bestimmt.

19. Verfahren zur in vitro erfolgenden Diagnose des Vorhandenseins von Tumorzellen in einem histologischen Schnitt, dadurch gekennzeichnet, daß man ein Produkt, bestehend aus Fluorescein, konjugiert mit einem TAG-Produkt nach Ansprüchen 15 bzw. 17 oder mit Anti-Recognin L bzw. Anti-Recognin M nach Anspruch 10 oder hergestellt durch ein Verfahren nach Anspruch 9, auf den genannten histologischen Schnitt aufbringt und bestimmt, ob eine Fluoreszenz auftritt oder nicht, wobei die Fluoreszenz nur in den genannten Tumorzellen auftritt.

20. Die polypeptidprodukte Recognin L und Recognin M nach Anspruch 5 bzw. 6 oder wie nach einem der Ansprüche 1 bis 4 hergestellt, zur Verwendung bei der Krebsdiagnose.

21. Die Antikörperprodukte Anti-Recognin L bzw. Anti-Recognin M nach Anspruch 8 oder hergestellt durch ein Verfahren nach Anspruch 7, zur Verwendung bei der Krebsdiagnose.

22. Die Komplexe Bromacetylcellulose-Recognin L und Bromacetylcellulose-Recognin M nach Anspruch 10 oder hergestellt durch eine Verfahren nach Anspruch 9, zur Verwendung bei der Krebsdiagnose.

23. Bromacetylcellulose-Recognin-L-Anti-Recognin L bzw. Bromacetylcellulose-Recognin-M-Anti-Recognin M nach Anspruch 12 oder hergestellt durch ein Verfahren nach Anspruch 11 zur Verwendung bei der Krebsdiagnose.

24. S-TAG- und F-TAG-Produkte nach Anspruch 15 bzw. 17 oder hergestellt durch ein Verfahren nach Anspruch 14 bzw. 16 zur Verwendung bei der Krebsdiagnose.

25. Die Polypeptidprodukte Recognin L und Recognin M nach Anspruch 5 bzw. 6 oder hergestellt nach einem der Ansprüche 1 bis 4 zur Verwendung in der Krebstherapie.

26. Die Antikörperprodukte Anti-Recognin L bzw. Anti-Recognin M nach Anspruch 8 oder hergestellt durch ein Verfahren nach Anspruch 7 zur Verwendung bei der Krebstherapie.

27. Die Komplexe Bromacetylcellulose-Recognin L und Bromacetylcellulose-Recognin M nach Anspruch 10 oder hergestellt durch ein Verfahren nach Anspruch 9 zur Verwendung bei der Krebstherapie.

28. Bromacetylcellulose-Recognin-L-Anti-Recognin L bzw. Bromacetylcellulose-Recognin-M-Anti-Recognin M nach Anspruch 12 oder hergestellt nach einem Verfahren nach Anspruch 11, zur Verwendung bei der Krebstherapie.

29. S-TAG- und F-TAG-Produkte nach Anspruch 15 bzw. 17 oder hergestellt durch ein Verfahren nach Anspruch 14 bzw. 16 zur Verwendung bei der Krebstherapie.

**Patentansprüche für den Vertragsstaat: AT**

1. Verfahren zur Herstellung der Polypeptidprodukte Recognin L bzw. Recognin M, dadurch gekennzeichnet, daß man

(i) künstliche Lymphom-Tumorzellen im Falle von Recognin L und Mamma-Tumorzellen im Falle von Recognin M mit einem neutralen Puffer in Kultur züchtet und durch wiederholtes Aufbrechen des Gewebes zur Solubilisierung der Proteinfraktionen extrahiert,

(ii) die Fraktion mit einem pK-Bereich von 1 bis 4 von dem resultierenden Extrakt der solubilisierten Proteine abtrennt und

(iii) das gewünschte Recognin-L- und Recognin-M-Produkt aus der genannten Fraktion (ii) isoliert, wobei die Recognin-L- und Recognin-M-Produkte jeweils durch folgendes charakterisiert sind:

(a) Bildung eines Niederschlags mit seinem spezifischen Antikörper bei quantitativen Präzipitintests und Ouchterlony-Geldiffusionstests,

(b) Löslichkeit in Wasser und wäßrigen Lösungen mit einem sauren oder neutralen pH-Wert und Unlöslichkeit bei einem alkalischen pH-Wert,

(c) eine spektrophotometrische Adsorptionswellenlänge von 280 mμ und

(d) ein Molekulargewicht von 8000 (bestimmt durch Dünnschicht-Gelchromatographie), wobei das Recognin-L-Produkt weiterhin durch eine Aminosäurezusammensetzung, die im wesentlichen wie folgt ist:

# 0 015 755

|  | Anzahl der Amino-säurereste |
|---|---|
| Asparaginsäure | 8 |
| Threonin | 5 |
| Serin | 5 |
| Glutaminsäure | 10 |
| Prolin | 5 |
| Glycin | 13 |
| Alanin | 10 |
| Valin | 6 |
| 1/2-Cystein | 1 |
| Methionin | 1 |
| Isoleucin | 4 |
| Leucin | 7 |
| Tyrosin | 1 |
| Phenylalanin | 3 |
| Lysin | 6 |
| Histidin | 2 |
| Arginin | 5 |
| ungefähre Gesamtsumme | 92 |

und durch die Tatsache, daß die Aminosäuren Cysteinsäure, Hydroxyprolin, Norleucin, Ammoniak, Isodesmosin, Desmosin, Hydroxylysin, Lysinonorleucin und gamma-Aminobuttersäure in erfaßbaren Mengen nicht vorhanden sind, charakterisiert ist und wobei eas Recognin-M-Produkt weiterhin durch eine Aminosäurenzusammensetzung, die im wesentlichen wie folgt ist:

|  | Anzahl der Amino-säurereste |
|---|---|
| Asparaginsäure | 9 |
| Threonin | 5 |
| Serin | 5 |
| Glutaminsäure | 11 |
| Prolin | 4 |
| Glycin | 9 |
| Alanin | 9 |
| Valin | 6 |
| 1/2-Cystein | 1 |
| Methionin | 1 |
| Isoleucin | 4 |
| Leucin | 8 |
| Tyrosin | 2 |
| Phenylalanin | 3 |
| Lysin | 6 |
| Histidin | 2 |
| Arginin | 5 |
| ungefähre Gesamtsumme | 90 |

un durch die Tatsache, daß die Aminosäuren Cysteinsäure, Hydroxyprolin, Norleucin, Ammoniak, Isodesmosin, Desmosin, Hydroxylysin, Lysinonorleucin und gamma-Aminobuttersäure in erfaßbaren Mengen nicht vorhanden sind, charakterisiert ist.

2. Verfahren nach Anspruch 1, dadurch gekennzeichnet, daß das genannte Recognin-L- oder Recognin-M-Produkt dazu imstande ist, mit Bromacetylcellulose unter Bildung von Bromacetylcellulose-Recognin L bzw. Bromacetylcellulose-Recognin M einen Komplex zu bilden, und daß das genannte Recognin L und Recognin M die spezifischen Antikörper Anti-Recognin L bzw. Anti-Recognin M nach Injektion in Säugetiere erzeugen, wobei das genannte Anti-Recognin L bzw. Anti-Recognin M gegenüber Tumorzellen in vitro toxisch ist und bei der Kupplung mit Fluorescein eine Fluoreszenz von Glyomzellen erzeugen.

3. Verfahren nach Anspruch 1 oder 2, dadurch gekennzeichnet, daß man die Abtrennungsstufe (ii) in der Weise bewirkt, daß man den genannten Extrakt von solubilisierten Proteinen auf eine chromatographische Säule aufgibt und mit steigend sauren Lösungsmitteln eluiert.

4. Verfahren nach Anspruch 3, dadurch gekennzeichnet, daß man die genannte Isolierungsstufe (iii) nach Anspruch 1 in der Weise durchführt, daß man das Eluat der Stufe (ii) filtriert, um eine Recognin L bzw. Recognin M enthaltende Fraktion zu erhalten, und daß man das Recognin L oder recognin M daraus durch Dünnschicht-Gelchromatographie abtrennt.

5. Als Recognin L bezeichnetes Polypeptidprodukt, dadurch gekennzeichnet, daß es ein Einzellinien-Präzipitat mit seinem spezifischen Antikörper bei quantitativen Präzipitintests und Ouchterlony-Geldiffusionstests bildet, daß es in Wasser und wäßrigen Lösungen mit einem sauren oder neutralen pH-Wert löslich und bei einem alkalischen pH-Wert unlöslich ist, daß es einen spektrophotometrischen Absorptionspeak bei einer Wellenlänge von 280 m$\mu$ hat, daß es ein Molekulargewicht von 8000 (bestimmt durch Dünnschicht-Gelchromatographie) hat und daß es eine Aminosäurenzusammensetzung, die im wesentlichen wie folgt ist:

|  | Anzahl der Amino-säurereste. |
|---|---|
| Asparaginsäure | 8 |
| Threonin | 5 |
| Serin | 5 |
| Glutaminsäure | 10 |
| Prolin | 5 |
| Glycin | 13 |
| Alanin | 10 |
| Valin | 6 |
| 1/2-Cystein | 1 |
| Methionin | 1 |
| Isoleucin | 4 |
| Leucin | 7 |
| Tyrosin | 1 |
| Phenylalanin | 3 |
| Lysin | 6 |
| Histidin | 2 |
| Arginin | 5 |
| ungefähre Gesamtsumme | 92 |

aufweist und gekennzeichnet durch die Tatsache, daß die Aminosäuren Cysteinsäure, Hydroxyprolin, Norleucin, Ammoniak, Isodesmosin, Desmosin, Hydroxylysin, Lysinonorleucin und gamma-Aminobuttersäure in erfaßbaren Mengen nicht vorhanden sind, zur Verwendung bei der Krebsdiagnose.

6. Als Recognin M bezeichnetes Polypeptidprodukt, dadurch gekennzeichnet, daß es ein Einzellinien-Präzipitat mit seinem spezifischen Antikörper bei quantitativen Präzipitintests und Ouchterlony-Geldiffusionstests bildet, daß es in Wasser und wäßrigen Lösungen mit einem sauren oder neutralen pH-Wert löslich und bei einem alkalischen pH-Wert unlöslich ist, daß es einen spektrophotometrischen Absorptionspeak bei einer Wellenlänge von 280 m$\mu$ hat, daß es ein Molekulargewicht von 8000 (bestimmt durch Dünnschicht-Gelchromatographie) hat und daß es eine Aminosäurenzusammensetzung, die im wesentlichen wie folgt ist:

|  | Anzahl der Amino-säurereste |
|---|---|
| Asparaginsäure | 9 |
| Threonin | 5 |
| Serin | 5 |
| Glutaminsäure | 11 |
| Prolin | 4 |
| Glycin | 9 |
| Alanin | 9 |
| Valin | 6 |
| 1/2-Cystein | 1 |
| Methionin | 1 |
| Isoleucin | 4 |
| Leucin | 8 |
| Tyrosin | 2 |
| Phenylalanin | 3 |
| Lysin | 6 |
| Histidin | 2 |
| Arginin | 5 |
| ungefähre Gesamtsumme | 90 |

53

**0 015 755**

aufweist und gekennzeichnet durch die Tatsache, daß die Aminosäuren Cysteinsäure, Hydroxyprolin, Norleucin, Ammoniak, Isodesmosin, Desmosin, Hydroxylysin, Lysinonorleucin und gamma-Aminobuttersäure in erfaßbaren Mengen nicht vorhanden sind, zur Verwendung bei der Krebsdiagnose.

7. Verfahren zur Herstellung des Produkts Anti-Recognin L oder Anti-Recognin M, dadurch gekennzeichnet, daß man in ein Säugetier das genannte Recognin L oder Recognin M nach Anspruch 5 bzw. 6 oder hergestellt nach einem Verfahren nach einem der Ansprüche 1 bis 4 injiziert, wodurch man den spezifischen Antikörper Anti-Recognin L oder Anti-Recognin M erzeugt, wobei das Anti-Recognin L oder Anti-Recognin M gegenüber Tumorzellen in vitro toxisch ist, und bei der Kupplung mit Fluorescein eine Fluoreszenz von Glyomzellen erzeugt, und daß man das Anti-Recognin L bzw. Anti-Recognin M gewinnt.

8. Die Produkte Anti-Recognin L und Anti-Recognin M, bestehend aud dem Antikörperprodukt, hergestellt durch Injektion von Recognin L oder Recognin M nach Anspruch 5 bzw. 6 oder hergestellt nach einem der Ansprüche 1 bis 4 in ein Säugetier, wobei die spezifischen Antikörper Anti-Recognin L und Anti-Recognin M jeweils gegenüber Tumorzellen in vitro toxisch sind und bei der Kupplung mit Fluorescein eine Fluoreszenz von Glyomzellen erzeugen, zur Verwendung bei der Krebsdiagnose.

9. Verfahren zur Herstellung des Komplexes Bromacetylcellulose-Recognin L oder Bromacetylcellulose-Recognin M, dadurch gekennzeichnet, daß man Recognin L oder Recognin M nach Anspruch 5 bzw. 6 oder hergestellt nach einem der Ansprüche 1 bis 4 mit Bromacetylcellulose in einer Weise vermischt, die dazu geeignet ist, den genannten Komplex Bromacetylcellulose-Recognin L bzw. Bromacetylcellulose-Recognin M zu bilden.

10. Die Komplexe Bromacetylcellulose-Recognin L und Bromacetylcellulose-Recognin M, gekennzeichnet als Komplexe von Bromacetylcellulose mit Recognin L oder Recognin M nach Anspruch 5 bzw. 6 oder hergestellt durch ein Verfahren nach einem der Ansprüche 1 bis 4, zur Verwendung bei der Krebsdiagnose.

11. Verfahren zur Herstellung von Bromacetylcellulose-Recognin-L-Anti-Recognin L oder Bromacetylcellulose-Recognin-M-Anti-Recognin M, gekennzeichnet durch Reaktion von Anti-Recognin L bzw. Anti-Recognin M nach Anspruch 6 oder hergestellt nach einem Verfahren nach Anspruch 7, mit Bromacetylcellulose-Recognin L bzw. Bromacetylcellulose-Recognin M nach Anspruch 10 oder hergestellt nach einem Verfahren nach Anspruch 9.

12. Die Komplexe Bromacetylcellulose-Recognin-L-Anti-Recognin L und Bromacetylcellulose-Recognin-M-Anti-Recognin M, gekennzeichnet als Komplexe von Anti-Recognin L bzw. Anti-Recognin M nach Anspruch 8 oder hergestellt nach einem Verfahren nach Anspruch 7, mit Bromacetylcellulose-Recognin L bzw. Bromacetylcellulose-Recognin M nach Anspruch 10 oder hergestellt durch ein Verfahren nach Anspruch 9, zur Verwendung bei der Krebsdiagnose.

13. Verfahren zur Herstellung von Anti-Recognin L bzw. Anti-Recognin M nach Anspruch 8, dadurch gekennzeichnet, daß man Bromacetylcellulose-Recognin-L-Anti-Recognin L bzw. Bromacetylcellulose-Recognin-M-Anti-Recognin M nach Anspruch 12 oder hergestellt durch ein Verfahren nach Anspruch 11 durch hydrolytische Behandlung mit Säure oder mit einem Proteinaseenzym entkomplexiert.

14. Verfahren zur Herstellung eines sich langsam an das Ziel anheftenden Globulins (S-TAG), dadurch gekennzeichnet, daß man Blutserum oder eine andere Körperflüssigkeit mit Bromacetylcellulose-Recognin L bzw. Bromacetylcellulose-Recognin M nach Anspruch 10 oder hergestellt durch ein Verfahren nach Anspruch 9 mindestens 2 h bei niedriger Temperatur, daß Abbaureaktionen verhindert werden, umsetzt und daß man das resultierende Material mit verdünnter Säure behandelt, um davon das genannte sich langsam an das Ziel anheftende Globulin (S-TAG) abzuspalten.

15. Ein produkt S-TAG, hergestellt nach dem Verfahren nach Anspruch 14, wobei das Produkt dadurch charakterisiert ist, daß es in wäßrigen gepufferten Lösungen löslich ist, ein Einzellinien-Präzipitat mit Recognin L oder Recognin M bei Ouchterlony-Geldiffusionstests bildet, in Cellophanmembranen nicht dialysierbar ist, von Milliporefiltern, die Moleküle mit einem Molekulargewicht von über 25000 zurückhalten, zurückgehalten wird, Molekulargewichte in verschiedenen Aggregationszuständen, bestimmt durch Dünnschicht-Gel chromatographie, von ungefähr 50000 und vielfachem davon in den Makroglobulinbereich hinein aufweist, und daß es eine spektrophotometrische Adsorptionspeak-Wellenlänge von 280 m$\mu$ aufweist, zur Verwendung bei der Krebsdiagnose.

16. Verfahren zur Herstellung von sich Schnell an das Ziel anheftendem Globulin (F-TAG), dadurch gekennzeichnet, daß man Blutserum oder eine andere Körperflüssigkeit mit Bromacetylcellulose-Recognin L bzw. Bromacetylcellulose-Recognin M nach Anspruch 10 oder hergestellt nach einem Verfahren nach Anspruch 9 ungefähr 10 min bei einer niedrigen Temperatur, daß Abbaureaktionen verhindert werden, umsetzt und daß man das resultierende Material mit verdünnter Säure behandelt, um davon das sich schnell an das Ziel anheftende Globulin (F-TAG) abzuspalten.

17. Ein Produkt F-TAG, hergestellt gemäß dem Verfahren nach Anspruch 16, wobei das Produkt dadurch charakterisiert ist, daß es in wäßrigen gepufferten Lösungen löslich ist, ein Einzellinien-Präzipitat mit Recognin L oder Recognin M bei Ouchterlony-Geldiffusionstests bildet, in Cellophanmembranen nicht dialysierbar ist, von Milliporefiltern, die Moleküle mit einem Molekulargewicht von mehr als 25000 zurückhalten, zurückgehalten wird, daß es Molekulargewichte in verschiedenen Aggregationszuständen, bestimmt durch Dünnschicht-Gelchromatographie, von ungefähr 50000 und

54

Vielfache davon un den Makroglobulinbereich hinein aufweist und daß es eine spektrophotometrische Adsorptionspeak-Wellenlänge von 280 mµ hat, zur Verwendung bei der Krebsdiagnose.

18. Verfahren zur Erfassung von Krebstumoren in lebenden Tieren, dadurch gekennzeichnet, daß man in vitro die Konzentrationen von S-TAG bzw. F-TAG nach den Ansprüchen 15 bzw. 17, hergestellt durch ein bekanntes Volumen des Blutserums des Säugetiers oder einer anderen Körperflüssigkeit, bestimmt.

19. Verfahren zur in vitro erfolgenden Diagnose des Vorhandenseins von Tumorzellen in einem histologischen Schnitt, dadurch gekennzeichnet, daß man ein Produkt, bestehend aus Fluorescein, konjugiert mit einem TAG-Produkt nach Ansprüchen 15 bzw. 17 oder mit Anti-Recognin L bzw. Anti-Recognin M nach Anspruch 10 oder hergestellt durch ein Verfahren nach Anspruch 9, auf den genannten histologischen Schnitt aufbringt und bestimmt, ob eine Fluoreszenz auftritt oder nicht, wobei die Fluoreszenz nur in den genannten Tumorzellen auftritt.

20. Als Recognin L bezeichnetes Polypeptidprodukt, dadurch gekennzeichnet, daß es mit seinem spezifischen Antikörper bei quantitativen Präzipitintests und Ouchterlony-Geldiffusionstests ein Einzellinien-Präzipitat bildet, in Wasser und wäßrigen Lösungen mit einem sauren oder neutralen pH-Wert löslich ist und bei einem alkalischen pH-Wert unlöslich ist, eine spektrophotometrische Adsorptionspeak-Wellenlänge von 280 mµ hat, ein Molekulargewicht von 8000 (bestimmt durch Dünnschicht-Gelchromatographie) hat und eine Aminosäurezusammensetzung, die im wesentlichen wie folgt ist:

|  | Anzahl der Amino-säurereste |
|---|---|
| Asparaginsäure | 8 |
| Threonin | 5 |
| Serin | 5 |
| Glutaminsäure | 10 |
| Prolin | 5 |
| Glycin | 13 |
| Alanin | 10 |
| Valin | 6 |
| 1/2-Cystein | 1 |
| Methionin | 1 |
| Isoleucin | 4 |
| Leucin | 7 |
| Tyrosin | 1 |
| Phenylalanin | 3 |
| Lysin | 6 |
| Histidin | 2 |
| Arginin | 5 |
| ungefähre Gesamtsumme | 92 |

aufweist und gekennzeichnet durch die Tatsache, daß die Aminosäuren Cysteinsäure, Hydroxyprolin, Norleucin, Ammoniak, Isodesmosin, Desmosin, Hydroxylysin, Lysinonorleucin und gamma-Aminobuttersäure in erfaßbaren Mengen abwesend sind, zur Verwendung bei der Krebstherapie.

21. Als Recognin M bezeichnetes Polypeptidprodukt, dadurch gekennzeichnet, daß es mit seinem spezifischen Antikörper bei quantitativen Präzipitintests und Ouchterlony-Geldiffusionstests ein Einzellinien-Präzipitat bildet, in Wasser und wäßrigen Lösungen mit einem sauren oder neutralen pH-Wert löslich ist und bei einem alkalischen pH-Wert unlöslich ist, eine spektrophotometrische Adsorptionspeak-Wellenlänge von 280 mµ hat, ein Molekulargewicht von 8000 (bestimmt durch Dünnschicht-Gelchromatographie) hat und eine Aminosäurezusammensetzung, die im wesentlichen wie folgt ist:

| | Anzahl der Amino-<br>säurereste |
|---|---|
| Asparaginsäure | 9 |
| Threonin | 5 |
| Serin | 5 |
| Glutaminsäure | 11 |
| Prolin | 4 |
| Glycin | 9 |
| Alanin | 9 |
| Valin | 6 |
| 1/2-Cystein | 1 |
| Methionin | 1 |
| Isoleucin | 4 |
| Leucin | 8 |
| Tyrosin | 2 |
| Phenylalanin | 3 |
| Lysin | 6 |
| Histidin | 2 |
| Arginin | 5 |
| ungefähre Gesamtsumme | 90 |

aufweist und gekennzeichnet durch die Tatsache, daß die Aminosäuren Cysteinsäure, Hydroxyprolin, Norleucin, Ammoniak, Isodesmosin, Desmosin, Hydroxylysin, Lysinonorleucin und gamma-Aminobuttersäure in erfaßbaren Mengen abwesend sind, zur Verwendung bei der Krebstherapie.

22. Die Produkte Anti-Recognin L und Anti-Recognin M, bestehend aus dem Antikörperprodukt, hergestellt durch Injektion von Recognin L oder Recognin M nach Anspruch 5 bzw. 6 oder hergestellt nach einem der Ansprüche 1 bis 4 in ein Säugetier, wobei die genannten spezifischen Anti-körper Anti-Recognin L und Anti-Recognin M jeweils gegenüber Tumorzellen in vitro toxisch sind und bei der Kupplung mit Fluorescein eine Fluoreszenz von Glyomzellen ergeben, zur Verwendung bei der Krebstherapie.

23. Die Komplexe Bromacetylcellulose-Recognin L und Bromacetylcellulose-Recognin M, gekennzeichnet als Komplexe von Bromacetylcellulose mit Recognin L oder Recognin M nach Anspruch 5 bzw. 6 oder hergestellt durch ein Verfahren nach einem der Ansprüche 1 bis 4, zur Verwendung bei der Krebstherapie.

24. Die Komplexe Bromacetylcellulose-Recognin-L-Anti-Recognin L und Bromacetylcellulose-Recognin-M-Anti-Recognin M, gekennzeichnet als Komplexe von Anti-Recognin L bzw. Anti-Recognin M nach Anspruch 8 oder hergestellt durch ein Verfahren nach Anspruch 7, mit Bromacetylcellulose-Recognin L oder Bromacetylcellulose-Recognin M nach Anspruch 10 oder hergestellt durch ein Verfahren nach Anspruch 9, zur Verwendung bei der Krebstherapie.

25. Ein Produkt S-TAG, hergestellt gemäß dem Verfahren nach Anspruch 14, wobei das genannte Produkt dadurch charakterisiert ist, daß es in wäßrigen gepufferten Lösungen löslich ist, ein Einzellinien-Präzipitat mit Recognin L oder Recognin M bei Ouchterlony-Geldiffusionstests bildet, in Cellophanmembranen nicht dialysierbar ist, von Milliporefiltern, die Moleküle mit einem Molekulargewicht von mehr als 25000 zurückhalten, zurückgehalten wird, Molekulargewichte in verschiedenen Aggregationszuständen, bestimmt durch Dünnschicht-Gelchromatographie, von ungefähr 50000 und Vielfachem davon in den Makroglobulinbereich hinein aufweist und daß es eine spektrophotometrische Adsorptionspeak-Wellenlänge von 280 m$\mu$ aufweist, zur Verwendung bei der Krebstherapie.

26. Ein Produkt F-TAG, hergestellt gemäß dem Verfahren nach Anspruch 16, wobei das Produkt dadurch charakterisiert ist, daß es in wäßrigen gepufferten Lösungen löslich ist, ein Einzellinien-Präzipitat mit Recognin L oder Recognin M bei Ouchterlony-Geldiffusionstests bildet, in Cellophanmembranen nicht dialysierbar ist, von Milliporefiltern, die Moleküle mit einem Molekulargewicht von mehr als 25000 zurückhalten, zurückgehalten wird, Molekulargewichte in verschiedenen Aggregationszuständen, bestimmt durch Dünnschicht-Gelchromatographie, von ungefähr 50000 und Vielfachem davon in den Makroglobulinbereich hinein hat und daß es eine spektrophotometrische Adsorptionspeak-Wellenlänge von 280 m$\mu$ aufweist, zur Verwendung bei der Krebstherapie.

Amount of Malignin Produced as a Function of Degree of Malignancy.

Fig.1.

Fig.1A.